# EUROPEAN PATENT APPLICATION

(11) **EP 4 729 546 A1**
(43) Date of publication of application: **22.04.2026**
(21) Application number: 24822743.1
(22) Date of filing: 13.06.2024
(51) Int. Cl.: C07K 16/36, A61K 39/395, C12N 15/13, C12N 15/63, C12N 5/10, C12N 1/19, A61P 9/10

(54) **ANTIBODY SPECIFICALLY RECOGNIZING FACTOR XIIA AND USE THEREOF**

(30) Priority: 16.06.2023 WO PCT/CN2023/100653
(71) Applicant: Jiangsu BioJeTay Biotechnology Co., Ltd., Wuxi, Jiangsu 214183 (CN)
(72) Inventor: ZHANG, Chao, Beijing 100176 (CN); FU, Wenyan, Beijing 100176 (CN); CUI, Jinglan, Beijing 100176 (CN); LI, Zhong, Beijing 100176 (CN)
(74) Representative: HGF
(86) International application number: PCT/CN2024/098977
(87) International publication number: WO 2024/255794

(57) **Abstract**

An antibody or an antigen-binding fragment thereof specifically recognizing factor XIIa, and a preparation method thereof and the use thereof.

## Description

### CROSS REFERENCE TO RELATED APPLICATIONS

This application claims priority to PCT Application No.PCT/CN2023/100653, filed on June 16, 2023 and entitled "ANTIBODIES SPECIFICALLY RECOGNIZING FACTOR XIIa AND USES THEREOF", the contents of which are incorporated herein by reference in their entirety.

### REFERENCE TO AN ELECTRONIC SEQUENCE LISTING

The contents of the electronic sequence listing (FXIIa-WIPO seq.xml, size: 149 KB, date recorded: 2025.10.16) is herein incorporated by reference in its entirety.

### FIELD

This application pertains to antibodies that specifically recognize FXIIa, and methods of manufacture and uses thereof, including methods of treating thrombosis, FXII/FXIIa induced kinin formation related disease, FXII/FXIIa induced complement activation related disease, contact activation system related disease, and inflammation related diseases.

### BACKGROUND

Factor XII (FXII) is an 80kDa glycoprotein with a plasma concentration of approximately 40 µg/mL (about 500 nmol/L). FXII in plasma is mainly synthesized in the liver. FXII plays a role in activating the endogenous pathways of coagulation and the Kallikrein kinin system. FXII undergoes auto-activation by exposure to negatively charged surfaces (such as polyanionic surfaces, glass, polyphosphates, ellagic acid, *etc*.), producing the active form FXIIa. Activated FXIIa can cleave plasma prekallikrein (PK) and produce active plasma kallikrein (pKal); Subsequently, the activated pKal can cleave FXII into FXIIa; The formed FXIIa activates more PKs, generating more pKal, thus forming positive feedback and amplification effects.

The activated FXIIa is composed of heavy and light chains, which are connected by disulfide bonds. The heavy chain contains the following domains: fibronectin type II domain, epidermal growth factor (EGF) - like domain I, fibronectin type I domain, epidermal growth factor like domain II, kringle domain, and proline rich domain, which are unique to FXII. The heavy chain contains regions that bind to negatively charged surfaces, while the light chain contains the catalytic domain typical of serine proteases. Therefore, FXII has a domain composition similar to other members of the serine protease family. The active sites on the FXII light chain are composed of typical catalytic triple residues His40, Asp89, and Ser191. This site is also a target of the main intrinsic inhibitor of the coagulation pathway, C1 esterase inhibitor (C1-INH). Cleavage or self activation by kallikrein leads to the splitting of Arg353 Va1354 bound to FXII zymogen, forming α - FXIIa (α - FXIIa), which contains heavy and light chains connected by disulfide bonds. In the form of α - FXIIa, two additional peptide bonds produce 30kD β - FXIIa under the action of trypsin, which contains truncated fragments of the intact light and heavy chains. α - FXIIa can bind to negatively charged surfaces and activate FXI and PK. β - FXIIa does not have surface binding ability, but can activate PK.

FXII deficiency, known as Hageman trait, was first identified by Oscar Ratnoff as a defect that leads to surface activated blood clotting tests such as prolonged partial thromboplastin time (PTT) (Ratnoff et al., 1955, J Clin Invest, 34:602-13.). Earl Davie was the first person to purify the Hageman factor and named it Factor XII according to convention. He and Ratnoff first demonstrated that FXII activates the plasma thrombin precursor FXI, and later proved that FXIa activates FIX (Ratnoff et al., 1961, J Clin Invest, 40:803-19; Ratnoff et al., 1962, Biochemistry, 1:677-85.). These results form the basis for their co-proposed coagulation cascade hypothesis with R.G. MacFarland, as well as the contact activation hypothesis that initiates blood clotting. It is known that the deficiency of coagulation factor XII is not associated with increased spontaneous or injury related bleeding complications, but rather the deficiency of FXII is associated with an increased risk of venous thrombosis (Kuhli C et al., 2004, Am J Ophthalmol, 137:459-464; Halbmayer WM et al., 1993, Wien Med Wochenschr. 143:43-50). Subsequent studies have also shown that FXII deficient mice do not experience spontaneous or excessive injury related bleeding, but have a protective effect against collagen and adrenaline induced thromboembolism. Transfusion of human FXII to FXII deficient mice can restore traumatic thrombosis (Renne T et al., 2005, J Exp Med; 202 (2): 271-281). These results indicate that FXII plays an important role in the pathological process of thrombosis, therefore interfering or blocking FXII activity may be suitable for blocking pathogenic arterial thrombosis and its clinical consequences.

FXII not only plays a role in the endogenous coagulation pathway, but the pKa formed through FXIIa activation or other means can activate the kallikrein system. PKa cleaves high molecular weight kininogen (HK) to release bradykinin and kininogen fragments (i.e. cHK). Bradykinin affects vascular permeability by binding to G protein coupled receptors bradykinin B2 and B1 receptors. As the main inhibitor of FXIIa and pKa, C1 INH regulates the contact activation system. Insufficient or dysfunctional plasma levels of C1-INH lead to sustained activation of FXIIa and pKa, resulting in increased formation of bradykinin (Caccia et al., 2014, Pediatr Allergy Immunol Pulmonol.; 27(4):159-163). Hereditary angioedema (HAE) syndrome is a group of diseases caused by a lack or deficiency of C1-INH (most commonly type I and II), or by increased function of FXIIa (type III), fibronectin, or angiopoietin 1 (Bafuno V et al., 2018, J Allergy Clin Immunol; 141:1009-17.). In type I HAE with C1 INH deficiency, both FXIIa and pKa were not sufficiently inhibited, resulting in increased production of bradykinin and increased vascular permeability. So far, all of these different mechanisms, except for the angiopoietin I mechanism of functional acquisition, have led to increased delivery of local bradykinin to tissues. Bradykinin causes vasodilation, increases vascular permeability, and leads to angioedema (Caccia et al., 2014, Pediatr Allergy Immunol Pulmonol.; 27(4):159-163). Blocking the bradykinin B2 receptor can alleviate symptoms in many HAE patients. Importantly, inhibiting the active enzymes of the contact activation system through injection of C1 INH substitute products, or inhibiting pKa through monoclonal antibodies or non peptide mimetics, can also improve and prevent type I and type II HAE in most patients (Banerji et al., 2018, JAMA, 320:2108-21; Aygören-Pürsün E et al., 2018, N Engl J Med; 379:352-62.).

Patent application WO2013014092A1 discloses a monoclonal antibody or its antigen-binding fragment against FXII/FXIIa, which has a binding affinity for human FXIIa - β that is more than twice that of human FXII and can inhibit the amide cleavage activity of human FXIIa. Patent application WO2017015431A1 discloses anti-FXIIa antibodies and methods for treating FXII related diseases using anti-FXII antibodies.

There is still a need in this field for therapeutic antibodies that can effective inhibit or otherwise antagonize FXII, as well as related methods for treating diseases or conditions mediated by FXII, such as thrombosis, FXII/FXIIa induced kinin formation related diseases, FXII/FXIIa induced complement activation related diseases, and contact activation system related diseases.

The disclosures of all publications, patents, patent applications and published patent applications referred to herein are hereby incorporated herein by reference in their entirety.

### BRIEF SUMMARY OF THE APPLICATION

In some embodiments, there is provided an isolated anti-FXIIa antibody comprising: a heavy chain variable domain (V_{H}) comprising a heavy chain complementarity determining region (HC-CDR) 1 comprising SQTMS (SEQ ID NO: 1); an HC-CDR2 comprising SX₁X₂X₃ISX₄RX₅YYADSVKG (SEQ ID NO: 89), wherein X₁ is I or S, X₂ is H or S, X₃ is H, V, or Y, X₄ is G, K, or R, and X₅ is A, D, K, M, N, P, R, S, T, or Y; and an HC-CDR3 comprising X₁HX₂FDX₃ (SEQ ID NO: 90), wherein X₁ is A, G, H, Q, S, or W, X₂ is A, D, or R, and X₃ is L or V; and a light chain variable domain (V_{L}) comprising a light chain complementarity determining region (LC-CDR) 1 comprising RASQSX₁X₂X₃X₄LA (SEQ ID NO: 91), wherein X₁ is L or V, X₂ is F, W, or Y, X₃ is S or Y, and X₄ is A, H, or N; an LC-CDR2 comprising GASTRAT (SEQ ID NO: 65); and an LC-CDR3 comprising QQX₁X₂X₃WPLS (SEQ ID NO: 92), wherein X₁ is N or Y, X₂ is H, K, N, or Q, and X₃ is K, R, or W.

In some embodiments, there is provided an isolated anti-FXIIa antibody comprising: (i) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74; (ii) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (iii) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76; (iv) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (v) a V_{H} comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34; and a V_{L} comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75; (vi) a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (vii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (viii) a V_{H} comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (ix) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (x) a V_{H} comprising an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xi) a V_{H} comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (xiii) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37; and a V_{L} comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77; (xiv) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75; (xv) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76; (xvi) a V_{H} comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xvii) a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xviii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (xix) a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (xx) a V_{H} comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxi) a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (xxii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxiii) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxiv) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxv) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78; (xxvi) a V_{H} comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (xxvii) a V_{H} comprising an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxviii) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76; (xxix) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75; (xxx) a V_{H} comprising an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxxi) a V_{H} comprising an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxxii) a V_{H} comprising an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75; (xxxiii) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79; (xxxiv) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80; (xxxv) a V_{H} comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the isolated anti-FXIIa antibody comprises: (i) a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 139, or a variant thereof having at least about 80% sequence identity to the amino acid sequence of SEQ ID NO: 139; (ii) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 140, or a variant thereof having at least about 80% sequence identity to the amino acid sequence of SEQ ID NO: 140; (iii) a V_{H} comprising the amino acid sequence of SEQ ID NO: 95, or a variant thereof having at least about 80% sequence identity to the amino acid sequence of SEQ ID NO: 95; and a V_{L} comprising the amino acid sequence of SEQ ID NO: 141, or a variant thereof having at least about 80% sequence identity to the amino acid sequence of SEQ ID NO: 141; (iv) a V_{H} comprising the amino acid sequence of SEQ ID NO: 96, or a variant thereof having at least about 80% sequence identity to the amino acid sequence of SEQ ID NO: 96; and a V_{L} comprising the amino acid sequence of SEQ ID NO: 142, or a variant thereof having at least about 80% sequence identity to the amino acid sequence of SEQ ID NO: 142; (v) a V_{H} comprising the amino acid sequence of SEQ ID NO: 97, or a variant thereof having at least about 80% sequence identity to the amino acid sequence of SEQ ID NO: 97; and a V_{L} comprising the amino acid sequence of SEQ ID NO: 143, or a variant thereof having at least about 80% sequence identity to the amino acid sequence of SEQ ID NO: 143; (vi) a V_{H} comprising the amino acid sequence of SEQ ID NO: 98, or a variant thereof having at least about 80% sequence identity to the amino acid sequence of SEQ ID NO: 98; and a V_{L} comprising the amino acid sequence of SEQ ID NO: 140, or a variant thereof having at least about 80% sequence identity to the amino acid sequence of SEQ ID NO: 140; (vii) a V_{H} comprising the amino acid sequence of SEQ ID NO: 96, or a variant thereof having at least about 80% sequence identity to the amino acid sequence of SEQ ID NO: 96; and a V_{L} comprising the amino acid sequence of SEQ ID NO: 140, or a variant thereof having at least about 80% sequence identity to the amino acid sequence of SEQ ID NO: 140; (viii) a V_{H} comprising the amino acid sequence of SEQ ID NO: 99, or a variant thereof having at least about 80% sequence identity to the amino acid sequence of SEQ ID NO: 99; and a V_{L} comprising the amino acid sequence of SEQ ID NO: 140, or a variant thereof having at least about 80% sequence identity to the amino acid sequence of SEQ ID NO: 140; (ix) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 142, or a variant thereof having at least about 80% sequence identity to the amino acid sequence of SEQ ID NO: 142; (x) a V_{H} comprising the amino acid sequence of SEQ ID NO: 100, or a variant thereof having at least about 80% sequence identity to the amino acid sequence of SEQ ID NO: 100; and a V_{L} comprising the amino acid sequence of SEQ ID NO: 140, or a variant thereof having at least about 80% sequence identity to the amino acid sequence of SEQ ID NO: 140; (xi) a V_{H} comprising the amino acid sequence of SEQ ID NO: 101, or a variant thereof having at least about 80% sequence identity to the amino acid sequence of SEQ ID NO: 101; and a V_{L} comprising the amino acid sequence of SEQ ID NO: 140, or a variant thereof having at least about 80% sequence identity to the amino acid sequence of SEQ ID NO: 140; (xii) a V_{H} comprising the amino acid sequence of SEQ ID NO: 102, or a variant thereof having at least about 80% sequence identity to the amino acid sequence of SEQ ID NO: 102; and a V_{L} comprising the amino acid sequence of SEQ ID NO: 142, or a variant thereof having at least about 80% sequence identity to the amino acid sequence of SEQ ID NO: 142; (xiii) a V_{H} comprising the amino acid sequence of SEQ ID NO: 103, or a variant thereof having at least about 80% sequence identity to the amino acid sequence of SEQ ID NO: 103; and a V_{L} comprising the amino acid sequence of SEQ ID NO: 144, or a variant thereof having at least about 80% sequence identity to the amino acid sequence of SEQ ID NO: 144; (xiv) a V_{H} comprising the amino acid sequence of SEQ ID NO: 104, or a variant thereof having at least about 80% sequence identity to the amino acid sequence of SEQ ID NO: 104; and a V_{L} comprising the amino acid sequence of SEQ ID NO: 145, or a variant thereof having at least about 80% sequence identity to the amino acid sequence of SEQ ID NO: 145; (xv) a V_{H} comprising the amino acid sequence of SEQ ID NO: 105, or a variant thereof having at least about 80% sequence identity to the amino acid sequence of SEQ ID NO: 105; and a V_{L} comprising the amino acid sequence of SEQ ID NO: 141, or a variant thereof having at least about 80% sequence identity to the amino acid sequence of SEQ ID NO: 141; (xvi) a V_{H} comprising the amino acid sequence of SEQ ID NO: 106, or a variant thereof having at least about 80% sequence identity to the amino acid sequence of SEQ ID NO: 106; and a V_{L} comprising the amino acid sequence of SEQ ID NO: 140, or a variant thereof having at least about 80% sequence identity to the amino acid sequence of SEQ ID NO: 140; (xvii) a V_{H} comprising the amino acid sequence of SEQ ID NO: 107, or a variant thereof having at least about 80% sequence identity to the amino acid sequence of SEQ ID NO: 107; and a V_{L} comprising the amino acid sequence of SEQ ID NO: 140, or a variant thereof having at least about 80% sequence identity to the amino acid sequence of SEQ ID NO: 140; (xviii) a V_{H} comprising the amino acid sequence of SEQ ID NO: 108, or a variant thereof having at least about 80% sequence identity to the amino acid sequence of SEQ ID NO: 108; and a V_{L} comprising the amino acid sequence of SEQ ID NO: 142, or a variant thereof having at least about 80% sequence identity to the amino acid sequence of SEQ ID NO: 142; (xix) a V_{H} comprising the amino acid sequence of SEQ ID NO: 109, or a variant thereof having at least about 80% sequence identity to the amino acid sequence of SEQ ID NO: 109; and a V_{L} comprising the amino acid sequence of SEQ ID NO: 142, or a variant thereof having at least about 80% sequence identity to the amino acid sequence of SEQ ID NO: 142; (xx) a V_{H} comprising the amino acid sequence of SEQ ID NO: 110, or a variant thereof having at least about 80% sequence identity to the amino acid sequence of SEQ ID NO: 110; and a V_{L} comprising the amino acid sequence of SEQ ID NO: 140, or a variant thereof having at least about 80% sequence identity to the amino acid sequence of SEQ ID NO: 140; (xxi) a V_{H} comprising the amino acid sequence of SEQ ID NO: 111, or a variant thereof having at least about 80% sequence identity to the amino acid sequence of SEQ ID NO: 111; and a V_{L} comprising the amino acid sequence of SEQ ID NO: 140, or a variant thereof having at least about 80% sequence identity to the amino acid sequence of SEQ ID NO: 140; (xxii) a V_{H} comprising the amino acid sequence of SEQ ID NO: 112, or a variant thereof having at least about 80% sequence identity to the amino acid sequence of SEQ ID NO: 112; and a V_{L} comprising the amino acid sequence of SEQ ID NO: 141, or a variant thereof having at least about 80% sequence identity to the amino acid sequence of SEQ ID NO: 141; (xxiii) a V_{H} comprising the amino acid sequence of SEQ ID NO: 113, or a variant thereof having at least about 80% sequence identity to the amino acid sequence of SEQ ID NO: 113; and a V_{L} comprising the amino acid sequence of SEQ ID NO: 142, or a variant thereof having at least about 80% sequence identity to the amino acid sequence of SEQ ID NO: 142; (xxiv) a V_{H} comprising the amino acid sequence of SEQ ID NO: 114, or a variant thereof having at least about 80% sequence identity to the amino acid sequence of SEQ ID NO: 114; and a V_{L} comprising the amino acid sequence of SEQ ID NO: 142, or a variant thereof having at least about 80% sequence identity to the amino acid sequence of SEQ ID NO: 142; (xxv) a V_{H} comprising the amino acid sequence of SEQ ID NO: 108, or a variant thereof having at least about 80% sequence identity to the amino acid sequence of SEQ ID NO: 108; and a V_{L} comprising the amino acid sequence of SEQ ID NO: 140, or a variant thereof having at least about 80% sequence identity to the amino acid sequence of SEQ ID NO: 140; (xxvi) a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 140, or a variant thereof having at least about 80% sequence identity to the amino acid sequence of SEQ ID NO: 140; (xxvii) a V_{H} comprising the amino acid sequence of SEQ ID NO: 115, or a variant thereof having at least about 80% sequence identity to the amino acid sequence of SEQ ID NO: 115; and a V_{L} comprising the amino acid sequence of SEQ ID NO: 140, or a variant thereof having at least about 80% sequence identity to the amino acid sequence of SEQ ID NO: 140; (xxviii) a V_{H} comprising the amino acid sequence of SEQ ID NO: 116, or a variant thereof having at least about 80% sequence identity to the amino acid sequence of SEQ ID NO: 116; and a V_{L} comprising the amino acid sequence of SEQ ID NO: 146, or a variant thereof having at least about 80% sequence identity to the amino acid sequence of SEQ ID NO: 146; (xxix) a V_{H} comprising the amino acid sequence of SEQ ID NO: 117, or a variant thereof having at least about 80% sequence identity to the amino acid sequence of SEQ ID NO: 117; and a V_{L} comprising the amino acid sequence of SEQ ID NO: 142, or a variant thereof having at least about 80% sequence identity to the amino acid sequence of SEQ ID NO: 142; (xxx) a V_{H} comprising the amino acid sequence of SEQ ID NO: 118, or a variant thereof having at least about 80% sequence identity to the amino acid sequence of SEQ ID NO: 118; and a V_{L} comprising the amino acid sequence of SEQ ID NO: 140, or a variant thereof having at least about 80% sequence identity to the amino acid sequence of SEQ ID NO: 140; (xxxi) a V_{H} comprising the amino acid sequence of SEQ ID NO: 104, or a variant thereof having at least about 80% sequence identity to the amino acid sequence of SEQ ID NO: 104; and a V_{L} comprising the amino acid sequence of SEQ ID NO: 142, or a variant thereof having at least about 80% sequence identity to the amino acid sequence of SEQ ID NO: 142; (xxxii) a V_{H} comprising the amino acid sequence of SEQ ID NO: 119, or a variant thereof having at least about 80% sequence identity to the amino acid sequence of SEQ ID NO: 119; and a V_{L} comprising the amino acid sequence of SEQ ID NO: 140, or a variant thereof having at least about 80% sequence identity to the amino acid sequence of SEQ ID NO: 140; (xxxiii) a V_{H} comprising the amino acid sequence of SEQ ID NO: 120, or a variant thereof having at least about 80% sequence identity to the amino acid sequence of SEQ ID NO: 120; and a V_{L} comprising the amino acid sequence of SEQ ID NO: 140, or a variant thereof having at least about 80% sequence identity to the amino acid sequence of SEQ ID NO: 140; (xxxiv) a V_{H} comprising the amino acid sequence of SEQ ID NO: 111, or a variant thereof having at least about 80% sequence identity to the amino acid sequence of SEQ ID NO: 111; and a V_{L} comprising the amino acid sequence of SEQ ID NO: 142, or a variant thereof having at least about 80% sequence identity to the amino acid sequence of SEQ ID NO: 142; (xxxv) a V_{H} comprising the amino acid sequence of SEQ ID NO: 121, or a variant thereof having at least about 80% sequence identity to the amino acid sequence of SEQ ID NO: 121; and a V_{L} comprising the amino acid sequence of SEQ ID NO: 140, or a variant thereof having at least about 80% sequence identity to the amino acid sequence of SEQ ID NO: 140; (xxxvi) a V_{H} comprising the amino acid sequence of SEQ ID NO: 122, or a variant thereof having at least about 80% sequence identity to the amino acid sequence of SEQ ID NO: 122; and a V_{L} comprising the amino acid sequence of SEQ ID NO: 141, or a variant thereof having at least about 80% sequence identity to the amino acid sequence of SEQ ID NO: 141; (xxxvii) a V_{H} comprising the amino acid sequence of SEQ ID NO: 123, or a variant thereof having at least about 80% sequence identity to the amino acid sequence of SEQ ID NO: 123; and a V_{L} comprising the amino acid sequence of SEQ ID NO: 142, or a variant thereof having at least about 80% sequence identity to the amino acid sequence of SEQ ID NO: 142; (xxxviii) a V_{H} comprising the amino acid sequence of SEQ ID NO: 124, or a variant thereof having at least about 80% sequence identity to the amino acid sequence of SEQ ID NO: 124; and a V_{L} comprising the amino acid sequence of SEQ ID NO: 140, or a variant thereof having at least about 80% sequence identity to the amino acid sequence of SEQ ID NO: 140; (xxxix) a V_{H} comprising the amino acid sequence of SEQ ID NO: 125, or a variant thereof having at least about 80% sequence identity to the amino acid sequence of SEQ ID NO: 125; and a V_{L} comprising the amino acid sequence of SEQ ID NO: 140, or a variant thereof having at least about 80% sequence identity to the amino acid sequence of SEQ ID NO: 140; (xl) a V_{H} comprising the amino acid sequence of SEQ ID NO: 126, or a variant thereof having at least about 80% sequence identity to the amino acid sequence of SEQ ID NO: 126; and a V_{L} comprising the amino acid sequence of SEQ ID NO: 140, or a variant thereof having at least about 80% sequence identity to the amino acid sequence of SEQ ID NO: 140; (xli) a V_{H} comprising the amino acid sequence of SEQ ID NO: 112, or a variant thereof having at least about 80% sequence identity to the amino acid sequence of SEQ ID NO: 112; and a V_{L} comprising the amino acid sequence of SEQ ID NO: 147, or a variant thereof having at least about 80% sequence identity to the amino acid sequence of SEQ ID NO: 147; (xlii) a V_{H} comprising the amino acid sequence of SEQ ID NO: 108, or a variant thereof having at least about 80% sequence identity to the amino acid sequence of SEQ ID NO: 108; and a V_{L} comprising the amino acid sequence of SEQ ID NO: 148, or a variant thereof having at least about 80% sequence identity to the amino acid sequence of SEQ ID NO: 148; (xliii) a V_{H} comprising the amino acid sequence of SEQ ID NO: 127, or a variant thereof having at least about 80% sequence identity to the amino acid sequence of SEQ ID NO: 127; and a V_{L} comprising the amino acid sequence of SEQ ID NO: 140, or a variant thereof having at least about 80% sequence identity to the amino acid sequence of SEQ ID NO: 140; (xliv) a V_{H} comprising the amino acid sequence of SEQ ID NO: 128, or a variant thereof having at least about 80% sequence identity to the amino acid sequence of SEQ ID NO: 128; and a V_{L} comprising the amino acid sequence of SEQ ID NO: 140, or a variant thereof having at least about 80% sequence identity to the amino acid sequence of SEQ ID NO: 140; (xlv) a V_{H} comprising the amino acid sequence of SEQ ID NO: 129, or a variant thereof having at least about 80% sequence identity to the amino acid sequence of SEQ ID NO: 129; and a V_{L} comprising the amino acid sequence of SEQ ID NO: 140, or a variant thereof having at least about 80% sequence identity to the amino acid sequence of SEQ ID NO: 140; (xlvi) a V_{H} comprising the amino acid sequence of SEQ ID NO: 130, or a variant thereof having at least about 80% sequence identity to the amino acid sequence of SEQ ID NO: 130; and a V_{L} comprising the amino acid sequence of SEQ ID NO: 140, or a variant thereof having at least about 80% sequence identity to the amino acid sequence of SEQ ID NO: 140.

In some embodiments, there is provided an isolated anti-FXIIa antibody that specifically binds to the human FXIIa with a Kd from 0.1 pM to 1 nM.

In some embodiments according to any of the isolated anti-FXIIa antibodies described above, the isolated anti-FXIIa antibody comprises an Fc fragment. In some embodiments, the isolated anti-FXIIa antibody is a full-length IgG antibody. In some embodiments, the isolated anti-FXIIa antibody is a full-length IgG1, IgG2, IgG3, or IgG4 antibody. In some embodiments, the anti-FXIIa antibody is a chimeric, human, or humanized antibody. In some embodiments, the anti-FXIIa antibody is an antigen binding fragment selected from the group consisting of a Fab, a Fab', a F(ab)'₂, a Fab'-SH, a single-chain Fv (scFv), an Fv fragment, a dAb, a Fd, a nanobody, a diabody, and a linear antibody.

In some embodiments, there is provided isolated nucleic acid molecule(s) that encodes any one of the anti-FXIIa antibodies described above. In some embodiments, there is provided a vector comprising any one of the nucleic acid molecules described above. In some embodiments, there is provided a host cell comprising any one of the anti-FXIIa antibodies described above, any one of the nucleic acid molecules described above, or any one of the vectors described above. In some embodiments, there is provided a method of producing an anti-FXIIa antibody, comprising: a) culturing any one of the host cells described above under conditions effective to express the anti-FXIIa antibody; and b) obtaining the expressed anti-FXIIa antibody from the host cell.

In some embodiments, there is provided a method of treating a disease or condition in an individual in need thereof, comprising administering to the individual an effective amount of any one of the anti-FXIIa antibodies described above. In some embodiments, there is provided the use of any one of the anti-FXIIa antibodies described herein in the manufacture of a pharmaceutical compositions for treating a disease or condition in an individual in need. In some embodiments, provided is the use of any one of the anti-FXIIa antibodies described above, or a pharmaceutical composition comprising any one of anti-FXIIa antibodies described above in the manufacture of a medicament for treating a disease or condition. In some embodiments, the disease or condition is associated with FXIIa, comprising thrombosis, FXII/FXIIa induced kinin ogenesis related disease, FXII/FXIIa induced complement activation related disease, contact activation system related disease, inflammation related diseases or condition. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, and multiple sclerosis.

Also provided are pharmaceutical compositions, kits and articles of manufacture comprising any one of the anti-FXIIa antibodies described above.

### BRIEF DESCRIPTION OF THE DRAWINGS

FIG. 1 shows the inhibitory effect of the exemplary anti-FXIIa antibodies FX-Y17-Y4, FX-Y17-Y8, FX-Y17-Y11, FX-Y17-Y23, FX-Y17-Y30, FX-Y17-Y34, and FX-Y17-Y45 on the coagulation caused by FXIIa detected using APTT.
FIG. 2 shows the inhibitory effects of the exemplary anti-FXIIa antibodies FX-Y17-Y4, FX-Y17-Y34, and FX-Y17-Y45 on the FXIIa cleavage of the prekallikrein (PK) detected using the pKa chemical substrate CS-3102.
FIG. 3 shows the pharmacokinetic study results of exemplary anti-FXIIa antibodies FX-Y17-Y4 and FX-Y17-Y4 (long-acting) in rats.
FIG. 4A shows the effect of the exemplary anti-FXIIa antibody FX-Y17-Y4 on dye leakage in mouse intestinal tissue. FIG. 4B shows the effect of the exemplary anti-FXIIa antibody FX-Y17-Y4 on dye residue in mouse blood.

### DETAILED DESCRIPTION OF THE APPLICATION

The present application in one aspect provides an anti-FXIIa antibody molecule. By using a combination of selections on naive scFv yeast libraries, affinity maturation and appropriately designed biochemical and biological assays, we have identified highly potent antibody molecules that bind to human FXIIa and inhibit the action of human FXIIa to its receptor. The results presented herein indicate that the present application antibodies bind to different region or epitope of FXIIa compared with the known anti-FXIIa antibody CSL312, and surprisingly are even more potent than CSL312 as demonstrated in a variety of biological assays.

The anti-FXIIa antibodies provided by the present application include, for example, full-length anti-FXIIa antibodies, anti-FXIIa scFvs, anti-FXIIa Fc fusion proteins, multi-specific (such as bispecific) anti-FXIIa antibodies, anti-FXIIa immunoconjugates, and the like.

In some embodiments, the isolated anti-FXIIa antibody comprises: a heavy chain variable domain (V_{H}) comprising a heavy chain complementarity determining region (HC-CDR) 1 comprising SQTMS (SEQ ID NO: 1); an HC-CDR2 comprising SX₁X₂X₃ISX₄RX₅YYADSVKG (SEQ ID NO: 89), wherein X₁ is I or S, X₂ is H or S, X₃ is H, V, or Y, X₄ is G, K, or R, and X₅ is A, D, K, M, N, P, R, S, T, or Y; and an HC-CDR3 comprising X₁HX₂FDX₃ (SEQ ID NO: 90), wherein X₁ is A, G, H, Q, S, or W, X₂ is A, D, or R, and X₃ is L or V; and a light chain variable domain (V_{L}) comprising a light chain complementarity determining region (LC-CDR) 1 comprising RASQSX₁X₂X₃X₄LA (SEQ ID NO: 91), wherein X₁ is L or V, X₂ is F, W, or Y, X₃ is S or Y, and X₄ is A, H, or N; an LC-CDR2 comprising GASTRAT (SEQ ID NO: 65); and an LC-CDR3 comprising QQX₁X₂X₃WPLS (SEQ ID NO: 92), wherein X₁ is N or Y, X₂ is H, K, N, or Q, and X₃ is K, R, or W.

In some embodiments, the isolated anti-FXIIa antibody comprises: a heavy chain variable domain (V_{H}) comprising a heavy chain complementarity determining region (HC-CDR) 1 comprising GYHIH (SEQ ID NO: 2); an HC-CDR2 comprising WIRVNGGNTMYARKFQG (SEQ ID NO: 26); and an HC-CDR3 comprising RPYCSGGTCSDS (SEQ ID NO: 43); and a light chain variable domain (V_{L}) comprising a light chain complementarity determining region (LC-CDR) 1 comprising RASESVFSNLA (SEQ ID NO: 57); an LC-CDR2 comprising DGNKRAT (SEQ ID NO: 66); and an LC-CDR3 comprising HQYNNWPPET (SEQ ID NO: 81).

In some embodiments, the isolated anti-FXIIa antibody comprises: a heavy chain variable domain (V_{H}) comprising a heavy chain complementarity determining region (HC-CDR) 1 comprising DYSMN (SEQ ID NO: 3); an HC-CDR2 comprising YISNSRSRSSKYYADSVKG (SEQ ID NO: 27); and an HC-CDR3 comprising IGYCKNGVCGGAYDY (SEQ ID NO: 44); and a light chain variable domain (V_{L}) comprising a light chain complementarity determining region (LC-CDR) 1 comprising RASQAIRNNLA (SEQ ID NO: 58); an LC-CDR2 comprising TASSLES (SEQ ID NO: 67); and an LC-CDR3 comprising LQYHDSPWT (SEQ ID NO: 82).

In some embodiments, the isolated anti-FXIIa antibody comprises: a heavy chain variable domain (V_{H}) comprising a heavy chain complementarity determining region (HC-CDR) 1 comprising SSSYYWV (SEQ ID NO: 4); an HC-CDR2 comprising SIYHSGSTYYNPSLKS (SEQ ID NO: 28); and an HC-CDR3 comprising AATSGWYLRYWYFDL (SEQ ID NO: 45); and a light chain variable domain (V_{L}) comprising a light chain complementarity determining region (LC-CDR) 1 comprising RANQNINSYLN (SEQ ID NO: 59); an LC-CDR2 comprising AATRLQS (SEQ ID NO: 68); and an LC-CDR3 comprising QQSYSSLRT (SEQ ID NO: 83).

In some embodiments, the isolated anti-FXIIa antibody comprises: a heavy chain variable domain (V_{H}) comprising a heavy chain complementarity determining region (HC-CDR) 1 comprising HYWMH (SEQ ID NO: 5); an HC-CDR2 comprising GITSSGGSKYYADSVKG (SEQ ID NO: 29); and an HC-CDR3 comprising RGPPTHDS (SEQ ID NO: 46); and a light chain variable domain (V_{L}) comprising a light chain complementarity determining region (LC-CDR) 1 comprising QASQDITKYLN (SEQ ID NO: 60); an LC-CDR2 comprising DASILET (SEQ ID NO: 69); and an LC-CDR3 comprising QHYNSYSWT (SEQ ID NO: 84).

In some embodiments, the isolated anti-FXIIa antibody comprises: a heavy chain variable domain (V_{H}) comprising a heavy chain complementarity determining region (HC-CDR) 1 comprising SNSADWN (SEQ ID NO: 6); an HC-CDR2 comprising RTYYRSKWYNDYAVSVKS (SEQ ID NO: 30); and an HC-CDR3 comprising GGPRPLDI (SEQ ID NO: 47); and a light chain variable domain (V_{L}) comprising a light chain complementarity determining region (LC-CDR) 1 comprising KSSQSVLYRSKRKPNLA (SEQ ID NO: 61); an LC-CDR2 comprising WASTRES (SEQ ID NO: 70); and an LC-CDR3 comprising QQYYSTPLT (SEQ ID NO: 85).

In some embodiments, the isolated anti-FXIIa antibody comprises: a heavy chain variable domain (V_{H}) comprising a heavy chain complementarity determining region (HC-CDR) 1 comprising SNWIG (SEQ ID NO: 7); an HC-CDR2 comprising IIYPDDSDTRYSPSFQG (SEQ ID NO: 31); and an HC-CDR3 comprising QGSSTYYRVGWFDP (SEQ ID NO: 48); and a light chain variable domain (V_{L}) comprising a light chain complementarity determining region (LC-CDR) 1 comprising RASQSISSYLN (SEQ ID NO: 62); an LC-CDR2 comprising AASSLQS (SEQ ID NO: 71); and an LC-CDR3 comprising QQSYSTPQT (SEQ ID NO: 86).

In some embodiments, the isolated anti-FXIIa antibody comprises: a heavy chain variable domain (V_{H}) comprising a heavy chain complementarity determining region (HC-CDR) 1 comprising TNYMT (SEQ ID NO: 8); an HC-CDR2 comprising VIYSGGSTYYADSVKG (SEQ ID NO: 32); and an HC-CDR3 comprising GRYYDGVDYGPKYGMDV (SEQ ID NO: 49); and a light chain variable domain (V_{L}) comprising a light chain complementarity determining region (LC-CDR) 1 comprising RASQSVRYNLA (SEQ ID NO: 63); an LC-CDR2 comprising GASIRAT (SEQ ID NO: 72); and an LC-CDR3 comprising QFYGSSPLT (SEQ ID NO: 87).

In some embodiments, the isolated anti-FXIIa antibody comprises: a heavy chain variable domain (V_{H}) comprising a heavy chain complementarity determining region (HC-CDR) 1 comprising SYWMH (SEQ ID NO: 9); an HC-CDR2 comprising RINLDGSSTGY ADSVRG (SEQ ID NO: 33); and an HC-CDR3 comprising SSRGFDP (SEQ ID NO: 50); and a light chain variable domain (V_{L}) comprising a light chain complementarity determining region (LC-CDR) 1 comprising RASQSISSSYLA (SEQ ID NO: 64); an LC-CDR2 comprising GASRRAT (SEQ ID NO: 73); and an LC-CDR3 comprising QLYGHSAYT (SEQ ID NO: 88).

Also provided are nucleic acids encoding the anti-FXIIa antibodies, compositions comprising the anti-FXIIa antibodies, and methods of making and using the anti-FXIIa antibodies.

### Definitions

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (*e.g*., preventing or delaying the worsening of the disease), preventing or delaying the spread (*e.g*., metastasis) of the disease, preventing or delaying the recurrence of the disease, delaying or slowing the progression of the disease, ameliorating the disease state, providing a remission (partial or total) of the disease, decreasing the dose of one or more of other medications required to treat the disease, delaying the progression of the disease, increasing or improving the quality of life, inhibiting the coagulation and/or inhibiting the thrombus formation. Also encompassed by "treatment" is a reduction of pathological consequence of the disease (such as, for example, vascular permeability for hereditary angioedema). The methods of the application contemplate any one or more of these aspects of treatment.

The term "antibody" includes full-length antibodies and antigen-binding fragments thereof. A full-length antibody comprises two heavy chains and two light chains. The variable regions of the light and heavy chains are responsible for antigen binding. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain).

The term "antigen-binding fragment" as used herein includes an antibody fragment including, for example, a diabody, a Fab, a Fab', a F(ab')₂, an Fv fragment, a disulfide stabilized Fv fragment (dsFv), a (dsFv)₂, a bispecific dsFv (dsFv-dsFv'), a disulfide stabilized diabody (ds diabody), a single-chain Fv (scFv), an scFv dimer (bivalent diabody), a multispecific antibody formed from a portion of an antibody comprising one or more CDRs, a single domain antibody, a nanobody, a domain antibody, a bivalent domain antibody, or any other antibody fragments that bind to an antigen but do not comprise a complete antibody structure. An antigen-binding fragment also includes a fusion protein comprising the antibody fragment described above. An antigen-binding fragment is capable of binding to the same antigen to which the parent antibody or a parent antibody fragment (*e.g*., a parent scFv) binds. In some embodiments, an antigen-binding fragment may comprise one or more CDRs from a particular human antibody grafted to a framework region from one or more different human antibodies.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody "competes" for binding to a target FXIIa with a second antibody when the first antibody inhibits target FXIIa binding of the second antibody by at least about 50% (such as at least about any of 55%, 60%, 65%, 70%, 75%, 80%, 85%, 90%, 95%, 98% or 99%) in the presence of an equimolar concentration of the first antibody, or *vice versa.* A high throughput process for "binning" antibodies based upon their cross-competition is described in PCT Publication No. WO 03/48731.

As used herein, the term "specifically binds", "specifically recognizing", or "is specific for" refers to measurable and reproducible interactions, such as binding between a target and an antibody that is determinative of the presence of the target in the presence of a heterogeneous population of molecules, including biological molecules. For example, an antibody that specifically recognizes a target (which can be an epitope) is an antibody that binds to this target with greater affinity, avidity, more readily, and/or with greater duration than its binding to other targets. In some embodiments, an antibody that specifically recognizes an antigen reacts with one or more antigenic determinants of the antigen with a binding affinity that is at least about 10 times its binding affinity for other targets.

An "isolated" anti-FXIIa antibody as used herein refers to an anti-FXIIa antibody that (1) is not associated with proteins found in nature, (2) is free of other proteins from the same source, (3) is expressed by a cell from a different species, or, (4) does not occur in nature.

The term "isolated nucleic acid" as used herein is intended to mean a nucleic acid of genomic, cDNA, or synthetic origin or some combination thereof, which by virtue of its origin the "isolated nucleic acid" (1) is not associated with all or a portion of a polynucleotide in which the "isolated nucleic acid" is found in nature, (2) is operably linked to a polynucleotide which it is not linked to in nature, or (3) does not occur in nature as part of a larger sequence.

As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**TABLE 1: CDR DEFINITIONS**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat *et al., supra* ²Residue numbering follows the nomenclature of Chothia *et al., supra* ³Residue numbering follows the nomenclature of MacCallum *et al., supra* ⁴Residue numbering follows the nomenclature of Lefranc *et al., supra* ⁵Residue numbering follows the nomenclature of Honegger and Plückthun, *supra* | | | | | |

The term "chimeric antibody" refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this application (*see* U.S. Patent No. 4,816,567; and Morrison et al., Proc. Natl. Acad. Sci. USA, 81:6851-6855 (1984)).

"Fv" is the minimum antibody fragment which contains a complete antigen-recognition and binding site. This fragment consists of a dimer of one heavy- and one light-chain variable region domain in tight, noncovalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv", also abbreviated as "sFv" or "scFv", are antibody fragments that comprise the V_{H} and V_{L} antibody domains connected into a single polypeptide chain. In some embodiments, the scFv polypeptide further comprises a polypeptide linker between the V_{H} and V_{L} domains which enables the scFv to form the desired structure for antigen binding. For a review of scFv, *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments prepared by constructing scFv fragments (see preceding paragraph) typically with short linkers (such as about 5 to about 10 residues) between the V_{H} and V_{L} domains such that inter-chain but not intra-chain pairing of the V domains is achieved, resulting in a bivalent fragment, *i.e*., fragment having two antigen-binding sites. Bispecific diabodies are heterodimers of two "crossover" scFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (*e.g*., rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skilled in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

The terms "Fc receptor" or "FcR" are used to describe a receptor that binds to the Fc region of an antibody. In some embodiments, an FcR of this application is one that binds to an IgG antibody (a γ receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review M. in Daëron, Annu. Rev. Immunol. 15:203-234 (1997)). The term includes allotypes, such as FcγRIIIA allotypes: FcγRIIIA-Phe158, FcγRIIIA-Val158, FcγRIIA-R131 and/or FcγRIIA-H131. FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "FcRn" refers to the neonatal Fc receptor (FcRn). FcRn is structurally similar to major histocompatibility complex (MHC) and consists of an α-chain noncovalently bound to β2-microglobulin. The multiple functions of the neonatal Fc receptor FcRn are reviewed in Ghetie and Ward (2000) Annu. Rev. Immunol. 18, 739-766. FcRn plays a role in the passive delivery of immunoglobulin IgGs from mother to young and the regulation of serum IgG levels. FcRn can act as a salvage receptor, binding and transporting pinocytosed IgGs in intact form both within and across cells, and rescuing them from a default degradative pathway.

The "CH1 domain" of a human IgG Fc region usually extends from about amino acid 118 to about amino acid 215 (EU numbering system).

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" comprises the stretch of residues of C-terminal to a CH2 domain in an Fc region (*i.e.* from about amino acid residue 341 to the C-terminal end of an antibody sequence, typically at amino acid residue 446 or 447 of an IgG).

A "functional Fc fragment" possesses an "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g*., B cell receptor; BCR), etc. Such effector functions generally require the Fc region to be combined with a binding domain (*e.g*., an antibody variable domain) and can be assessed using various assays known in the art.

An antibody with a variant IgG Fc with "altered" FcR binding affinity or ADCC activity is one which has either enhanced or diminished FcR binding activity (*e.g*., FcyR or FcRn) and/or ADCC activity compared to a parent polypeptide or to a polypeptide comprising a native sequence Fc region. The variant Fc which "exhibits increased binding" to an FcR binds at least one FcR with higher affinity (*e.g*., lower apparent Kd or IC₅₀ value) than the parent polypeptide or a native sequence IgG Fc. According to some embodiments, the improvement in binding compared to a parent polypeptide is about 3-fold, such as about any of 5, 10, 25, 50, 60, 100, 150, 200, or up to 500-fold, or about 25% to 1000% improvement in binding. The polypeptide variant which "exhibits decreased binding" to an FcR, binds at least one FcR with lower affinity (*e.g.,* higher apparent Kd or IC₅₀ value) than a parent polypeptide. The decrease in binding compared to a parent polypeptide may be about 40% or more decrease in binding.

"Antibody-dependent cell-mediated cytotoxicity" or "ADCC" refers to a form of cytotoxicity in which secreted Ig bound to Fc receptors (FcRs) present on certain cytotoxic cells (*e.g.,* Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are required for such killing. The primary cells for mediating ADCC, NK cells, express FcγRIII only, whereas monocytes express FcyRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo*, *e.g*., in an animal model such as that disclosed in Clynes et al. PNAS (USA) 95:652-656 (1998).

The polypeptide comprising a variant Fc region which "exhibits increased ADCC" or mediates ADCC in the presence of human effector cells more effectively than a polypeptide having wild type IgG Fc or a parent polypeptide is one which *in vitro* or *in vivo* is substantially more effective at mediating ADCC, when the amounts of polypeptide with variant Fc region and the polypeptide with wild type Fc region (or the parent polypeptide) in the assay are essentially the same. Generally, such variants will be identified using any *in vitro* ADCC assay known in the art, such as assays or methods for determining ADCC activity, *e.g*., in an animal model etc. In some embodiments, the variant is from about 5-fold to about 100-fold, *e.g.* from about 25 to about 50-fold, more effective at mediating ADCC than the wild type Fc (or parent polypeptide).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed. Polypeptide variants with altered Fc region amino acid sequences and increased or decreased C1q binding capability are described in US patent No. 6,194,551B1 and WO99/51642. The contents of those patent publications are specifically incorporated herein by reference. *See* also, Idusogie et al. J. Immunol. 164: 4178-4184 (2000).

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Homologous" refers to the sequence similarity or sequence identity between two polypeptides or between two nucleic acid molecules. When a position in both of the two compared sequences is occupied by the same base or amino acid monomer subunit, *e.g*., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

An "effective amount" of an anti-FXIIa antibody or composition as disclosed herein, is an amount sufficient to carry out a specifically stated purpose. An "effective amount" can be determined empirically and by known methods relating to the stated purpose.

The term "therapeutically effective amount" refers to an amount of an anti-FXIIa antibody or composition as disclosed herein, effective to "treat" a disease or disorder in an individual. In the case of thrombosis, the therapeutically effective amount of the anti-FXIIa antibody or composition as disclosed herein can inhibit (i.e. slow down to some extent) FXII/FXIIa enzyme activity; extend (i.e. to some extent) activated partial thromboplastin time (aPTT); reduce (i.e. eliminate to some extent) the size and/or volume of thrombus formation; reduce (i.e. eliminate to some extent) the deposition of fibrin; reduce (i.e. eliminate to some extent) platelet deposition; Reduce the rate of vascular occlusion; increase vascular permeability; inhibit (i.e. slowing down or selectively stopping) the excessive activation of kininase to a certain extent; alleviate one or more symptoms related to thrombosis to some extent. To the extent the anti-FXIIa antibody or composition as disclosed herein can prevent growth and/or kill existing cancer cells, it can be cytostatic and/or cytotoxic. In some embodiments, the therapeutically effective amount is a growth inhibitory amount. In some embodiments, the therapeutically effective amount is an amount that extends the survival of a patient. In some embodiments, the therapeutically effective amount is an amount that improves progression free survival of a patient.

As used herein, by "pharmaceutically acceptable" or "pharmacologically compatible" is meant a material that is not biological or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug Administration.

It is understood that embodiments of the application described herein include "consisting of' and/or "consisting essentially of' embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat cancer of type X means the method is used to treat cancer of types other than X.

As used herein and in the appended claims, the singular forms "a", "an", and "the" include plural referents unless the context clearly dictates otherwise.

### Anti-FXIIa antibodies

In one aspect, the present application provides anti-FXIIa antibodies that specifically bind to human and/or rhesus monkey FXIIa. Anti-FXIIa antibodies include, but are not limited to, humanized antibodies, chimeric antibodies, mouse antibodies, human antibodies, and antibodies comprising the heavy chain and/or light chain CDRs discussed herein. In one aspect, the present application provides isolated antibodies that bind to FXIIa. Contemplated anti-FXIIa antibodies include, for example, full-length anti-FXIIa antibodies (*e.g*., full-length IgG1 or IgG4), anti-FXIIa scFvs, anti-FXIIa Fc fusion proteins, multi-specific (such as bispecific) anti-FXIIa antibodies, anti-FXIIa immunoconjugates, and the like. In some embodiments, the anti-FXIIa antibody is a full-length antibody (*e.g*., full-length IgG1 or IgG4) or antigen-binding fragment thereof, which specifically binds to FXIIa. In some embodiments, the anti-FXIIa antibody is a Fab, a Fab', a F(ab)'₂, a Fab'-SH, a single-chain Fv (scFv), an Fv fragment, a dAb, a Fd,a nanobody, a diabody, or a linear antibody. In some embodiments, reference to an antibody that specifically binds to FXIIa means that the antibody binds to FXIIa with an affinity that is at least about 10 times (including for example at least about any one of 10, 10², 10³, 10⁴, 10⁵, 10⁶, or 10⁷ times) more tightly than its binding affinity for a non-target. In some embodiments, the non-target is an antigen that is not FXIIa. Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although anti-FXIIa antibodies containing human sequences (*e.g*., human heavy and light chain variable domain sequences comprising human CDR sequences) are extensively discussed herein, non-human anti-FXIIa antibodies are also contemplated. In some embodiments, non-human anti-FXIIa antibodies comprise human CDR sequences from an anti-FXIIa antibody as described herein and non-human framework sequences. Non-human framework sequences include, in some embodiments, any sequence that can be used for generating synthetic heavy and/or light chain variable domains using one or more human CDR sequences as described herein, including, *e.g*., mammals, *e.g*., mouse, rat, rabbit, pig, bovine (*e.g.,* cow, bull, buffalo), deer, sheep, goat, chicken, cat, dog, ferret, primate (*e.g.,* marmoset, rhesus monkey), *etc.* In some embodiments, a non-human anti-FXIIa antibody includes an anti-FXIIa antibody generated by grafting one or more human CDR sequences as described herein onto a non-human framework sequence (e.g., a mouse or chicken framework sequence).

The complete amino acid sequence of an exemplary native human FXIIa comprises or consists of the amino acid sequence of SEQ ID NO: 161.

In some embodiments, the anti-FXIIa antibody described herein specifically recognizes an epitope within human FXIIa. In some embodiments, the anti-FXIIa antibody cross-reacts with FXIIa from species other than human. In some embodiments, the anti-FXIIa antibody is completely specific for human FXIIa and does not exhibit cross-reactivity with FXIIas from other non-human species.

In some embodiments, the anti-FXIIa antibody cross-reacts with at least one allelic variant of the FXIIa protein (or fragments thereof). In some embodiments, the allelic variant has up to about 30 (such as about any of 1, 2, 3, 4, 5, 6, 7, 8, 9, 10, 15, 20, 25, or 30) amino acid substitutions (such as a conservative substitution) when compared to the naturally occurring FXIIa (or fragments thereof). In some embodiments, the anti-FXIIa antibody does not cross-react with any allelic variants of the FXIIa protein (or fragments thereof).

In some embodiments, the anti-FXIIa antibody cross-reacts with at least one interspecies variant of the FXIIa protein. In some embodiments, for example, the FXIIa protein (or fragments thereof) is human FXIIa and the interspecies variant of the FXIIa protein (or fragments thereof) is a cynomolgus monkey variant thereof. In some embodiments, the anti-FXIIa antibody does not cross-react with any interspecies variants of the FXIIa protein.

In some embodiments, according to any of the anti-FXIIa antibodies described herein, the anti-FXIIa antibody comprises an antibody heavy chain constant region and an antibody light chain constant region. In some embodiments, the anti-FXIIa antibody comprises an IgG1 heavy chain constant region. In some embodiments, the anti-FXIIa antibody comprises an IgG2 heavy chain constant region. In some embodiments, the anti-FXIIa antibody comprises an IgG3 heavy chain constant region. In some embodiments, the anti-FXIIa antibody comprises an IgG4 heavy chain constant region. In some embodiments, the heavy chain constant region comprises (including consisting of or consisting essentially of) the amino acid sequence of SEQ ID NO: 157. In some embodiments, the heavy chain constant region comprises (including consisting of or consisting essentially of) the amino acid sequence of SEQ ID NO: 158. In some embodiments, the anti-FXIIa antibody comprises a kappa light chain constant region. In some embodiments, the light chain constant region comprises (including consisting of or consisting essentially of) the amino acid sequence of SEQ ID NO: 159. In some embodiments, the anti-FXIIa antibody comprises a lambda light chain constant region. In some embodiments, the light chain constant region comprises (including consisting of or consisting essentially of) the amino acid sequence of SEQ ID NO: 160. In some embodiments, the anti-FXIIa antibody comprises an antibody heavy chain variable domain and an antibody light chain variable domain.

In some embodiments, the isolated anti-FXIIa antibody comprises: a heavy chain variable domain (V_{H}) comprising a heavy chain complementarity determining region (HC-CDR) 1 comprising SQTMS (SEQ ID NO: 1); an HC-CDR2 comprising SX₁X₂X₃ISX₄RX₅YYADSVKG (SEQ ID NO: 89), wherein X₁ is I or S, X₂ is H or S, X₃ is H, V, or Y, X₄ is G, K, or R, and X₅ is A, D, K, M, N, P, R, S, T, or Y; and an HC-CDR3 comprising X₁HX₂FDX₃ (SEQ ID NO: 90), wherein X₁ is A, G, H, Q, S, or W, X₂ is A, D, or R, and X₃ is L or V; and a light chain variable domain (V_{L}) comprising a light chain complementarity determining region (LC-CDR) 1 comprising RASQSX₁X₂X₃X₄LA (SEQ ID NO: 91), wherein X₁ is L or V, X₂ is F, W, or Y, X₃ is S or Y, and X₄ is A, H, or N; an LC-CDR2 comprising GASTRAT (SEQ ID NO: 65); and an LC-CDR3 comprising QQX₁X₂X₃WPLS (SEQ ID NO: 92), wherein X₁ is N or Y, X₂ is H, K, N, or Q, and X₃ is K, R, or W

In some embodiments, the anti-FXIIa antibody comprises a V_{H} comprising: an HC-CDR1 comprising the amino acid sequence of SEQ ID NO: 1, or a variant thereof comprising up to about 3 (such as about any of 1, 2, or 3) amino acid substitutions, an HC-CDR2 comprising the amino acid sequence of any one of SEQ ID NOs: 10-25, or a variant thereof comprising up to about 3 (such as about any of 1, 2, or 3) amino acid substitutions, and an HC-CDR3 comprising the amino acid sequence of any one of SEQ ID NOs: 34-42, or a variant thereof comprising up to about 3 (such as about any of 1, 2, or 3) amino acid substitutions.

In some embodiments, the anti-FXIIa antibody comprises a V_{H} comprising: an HC-CDR1 comprising the amino acid sequence of SEQ ID NO: 1, an HC-CDR2 comprising the amino acid sequence of any one of SEQ ID NOs: 10-25, and an HC-CDR3 comprising the amino acid sequence of any one of SEQ ID NOs: 34-42.

In some embodiments, the anti-FXIIa antibody comprises a V_{L} comprising: an LC-CDR1 comprising the amino acid sequence of any one of SEQ ID NOs: 51-56, or a variant thereof comprising up to about 3 (such as about any of 1, 2, or 3) amino acid substitutions, an LC-CDR2 comprising the amino acid sequence of SEQ ID NO: 65, or a variant thereof comprising up to about 3 (such as about any of 1, 2, or 3) amino acid substitutions, and an LC-CDR3 comprising the amino acid sequence of any one of SEQ ID NOs: 74-80, or a variant thereof comprising up to about 3 (such as about any of 1, 2, or 3) amino acid substitutions.

In some embodiments, the anti-FXIIa antibody comprises a V_{L} comprising: an LC-CDR1 comprising the amino acid sequence of any one of SEQ ID NOs: 51-56, an LC-CDR2 comprising the amino acid sequence of SEQ ID NO: 65, and an LC-CDR3 comprising the amino acid sequence of any one of SEQ ID NOs: 74-80.

In some embodiments, the anti-FXIIa antibody comprises a V_{H} comprising: an HC-CDR1 comprising the amino acid sequence of SEQ ID NO: 1, or a variant thereof comprising up to about 3 (such as about any of 1, 2, or 3) amino acid substitutions, an HC-CDR2 comprising the amino acid sequence of any one of SEQ ID NOs: 10-25, or a variant thereof comprising up to about 3 (such as about any of 1, 2, or 3) amino acid substitutions, and an HC-CDR3 comprising the amino acid sequence of any one of SEQ ID NOs: 34-42, or a variant thereof comprising up to about 3 (such as about any of 1, 2, or 3) amino acid substitutions; and a V_{L} comprising: an LC-CDR1 comprising the amino acid sequence of any one of SEQ ID NOs: 51-56, or a variant thereof comprising up to about 3 (such as about any of 1, 2, or 3) amino acid substitutions, an LC-CDR2 comprising the amino acid sequence of SEQ ID NO: 65, or a variant thereof comprising up to about 3 (such as about any of 1, 2, or 3) amino acid substitutions, and an LC-CDR3 comprising the amino acid sequence of any one of SEQ ID NOs: 74-80, or a variant thereof comprising up to about 3 (such as about any of 1, 2, or 3) amino acid substitutions.

In some embodiments, the anti-FXIIa antibody comprises a V_{H} comprising: an HC-CDR1 comprising the amino acid sequence of SEQ ID NO: 1, an HC-CDR2 comprising the amino acid sequence of any one of SEQ ID NOs: 10-25, and an HC-CDR3 comprising the amino acid sequence of any one of SEQ ID NOs: 34-42; and a V_{L} comprising: an LC-CDR1 comprising the amino acid sequence of any one of SEQ ID NOs: 51-56, an LC-CDR2 comprising the amino acid sequence of SEQ ID NO: 65, and an LC-CDR3 comprising the amino acid sequence of any one of SEQ ID NOs: 74-80.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34; and a V_{L} comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37, or a variant thereof comprising up to about 5 amino acid substitutions in the HC-CDRs; and a V_{L} comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37; and a V_{L} comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39, or a variant thereof comprising up to about 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

In some embodiments, the anti-FXIIa antibody comprises a V_{H} comprising an HC-CDR1, an HC-CDR2 and an HC-CDR3 of the V_{H} comprising the amino acid sequence of any one of SEQ ID NOs: 93-130; and a V_{L} comprising an LC-CDR1, an LC-CDR2, and an LC-CDR3 of the V_{L} comprising the amino acid sequence of any one of SEQ ID NOs: 139-148.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 93, and a V_{L} comprising an LC-CDR1, an LC-CDR2 and an LC-CDR3 of the V_{L} comprising the amino acid sequence of SEQ ID NO: 139.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 94, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 95, and a V_{L} comprising an LC-CDR1, an LC-CDR2 and an LC-CDR3 of the V_{L} comprising the amino acid sequence of SEQ ID NO: 141.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 96, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 97, and a V_{L} comprising an LC-CDR1, an LC-CDR2 and an LC-CDR3 of the V_{L} comprising the amino acid sequence of SEQ ID NO: 143.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 98, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 96, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 99, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 94, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 100, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 101, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 102, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 103, and a V_{L} comprising an LC-CDR1, an LC-CDR2 and an LC-CDR3 of the V_{L} comprising the amino acid sequence of SEQ ID NO: 144.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 104, and a V_{L} comprising an LC-CDR1, an LC-CDR2 and an LC-CDR3 of the V_{L} comprising the amino acid sequence of SEQ ID NO: 145.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 105, and a V_{L} comprising an LC-CDR1, an LC-CDR2 and an LC-CDR3 of the V_{L} comprising the amino acid sequence of SEQ ID NO: 141.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 106, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 107, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 108, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 109, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 110, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 111, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 112, and a V_{L} comprising an LC-CDR1, an LC-CDR2 and an LC-CDR3 of the V_{L} comprising the amino acid sequence of SEQ ID NO: 141.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 113, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 114, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 108, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 93, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 115, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 116, and a V_{L} comprising an LC-CDR1, an LC-CDR2 and an LC-CDR3 of the V_{L} comprising the amino acid sequence of SEQ ID NO: 146.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 117, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 118, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 104, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 119, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 120, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 111, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 121, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 122, and a V_{L} comprising an LC-CDR1, an LC-CDR2 and an LC-CDR3 of the V_{L} comprising the amino acid sequence of SEQ ID NO: 141.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 123, and a V_{L} comprising an LC-CDR1, an LC-CDR2 and an LC-CDR3 of the V_{L} comprising the amino acid sequence of SEQ ID NO: 142.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 124, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 125, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 126, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 112, and a V_{L} comprising an LC-CDR1, an LC-CDR2 and an LC-CDR3 of the V_{L} comprising the amino acid sequence of SEQ ID NO: 147.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 108, and a V_{L} comprising an LC-CDR1, an LC-CDR2 and an LC-CDR3 of the V_{L} comprising the amino acid sequence of SEQ ID NO: 148.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 127, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 128, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 129, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 130, and a V_{L} comprising an LC-CDR1, an LC-CDR2 and an LC-CDR3 of the V_{L} comprising the amino acid sequence of SEQ ID NO: 140.

In some embodiments, the anti-FXIIa antibody comprises a V_{H} comprising the amino acid sequence of any one of SEQ ID NOs: 93-130, or a variant thereof having at least about 80% (such as at least about any of 80%, 85%, 90%, 95%, 96%, 97%, 98%, or 99%) sequence identity to the amino acid sequence of any one of SEQ ID NOs: 93-130, and a V_{L} comprising the amino acid sequence of any one of SEQ ID NOs: 139-148, or a variant thereof having at least about 80% (such as at least about any of 80%, 85%, 90%, 95%, 96%, 97%, 98%, or 99%) sequence identity to the amino acid sequence of any one of SEQ ID NOs: 139-148. In some embodiments, the anti-FXIIa antibody comprises a V_{H} comprising the amino acid sequence of any one of SEQ ID NOs: 93-130, and a V_{L} comprising the amino acid sequence of any one of SEQ ID NOs: 139-148.

In some embodiments, the anti-FXIIa antibody comprises a V_{H} comprising the amino acid sequence of SEQ ID NO: 93, or a variant thereof having at least about 80% (such as at least about any of 80%, 85%, 90%, 95%, 96%, 97%, 98%, or 99%) sequence identity to the amino acid sequence of SEQ ID NO: 93, and a V_{L} comprising the amino acid sequence of SEQ ID NO: 139, or a variant thereof having at least about 80% (such as at least about any of 80%, 85%, 90%, 95%, 96%, 97%, 98%, or 99%) sequence identity to the amino acid sequence of SEQ ID NO: 139. In some embodiments, the anti-FXIIa antibody comprises a V_{H} comprising the amino acid sequence of SEQ ID NO: 93 and a V_{L} comprising the amino acid sequence of SEQ ID NO: 139.

In some embodiments, the anti-FXIIa antibody comprises a V_{H} comprising the amino acid sequence of SEQ ID NO: 94, or a variant thereof having at least about 80% (such as at least about any of 80%, 85%, 90%, 95%, 96%, 97%, 98%, or 99%) sequence identity to the amino acid sequence of SEQ ID NO: 94, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 94 and a V_{L} comprising the amino acid sequence of SEQ ID NO: 140.

In some embodiments, the anti-FXIIa antibody comprises a V_{H} comprising the amino acid sequence of SEQ ID NO: 95, or a variant thereof having at least about 80% (such as at least about any of 80%, 85%, 90%, 95%, 96%, 97%, 98%, or 99%) sequence identity to the amino acid sequence of SEQ ID NO: 95, and a V_{L} comprising the amino acid sequence of SEQ ID NO: 141, or a variant thereof having at least about 80% (such as at least about any of 80%, 85%, 90%, 95%, 96%, 97%, 98%, or 99%) sequence identity to the amino acid sequence of SEQ ID NO: 141. In some embodiments, the anti-FXIIa antibody comprises a V_{H} comprising the amino acid sequence of SEQ ID NO: 95 and a V_{L} comprising the amino acid sequence of SEQ ID NO: 141.

In some embodiments, the anti-FXIIa antibody comprises a V_{H} comprising the amino acid sequence of SEQ ID NO: 96, or a variant thereof having at least about 80% (such as at least about any of 80%, 85%, 90%, 95%, 96%, 97%, 98%, or 99%) sequence identity to the amino acid sequence of SEQ ID NO: 96, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 96 and a V_{L} comprising the amino acid sequence of SEQ ID NO: 142.

In some embodiments, the anti-FXIIa antibody comprises a V_{H} comprising the amino acid sequence of SEQ ID NO: 97, or a variant thereof having at least about 80% (such as at least about any of 80%, 85%, 90%, 95%, 96%, 97%, 98%, or 99%) sequence identity to the amino acid sequence of SEQ ID NO: 97, and a V_{L} comprising the amino acid sequence of SEQ ID NO: 143, or a variant thereof having at least about 80% (such as at least about any of 80%, 85%, 90%, 95%, 96%, 97%, 98%, or 99%) sequence identity to the amino acid sequence of SEQ ID NO: 143. In some embodiments, the anti-FXIIa antibody comprises a V_{H} comprising the amino acid sequence of SEQ ID NO: 97 and a V_{L} comprising the amino acid sequence of SEQ ID NO: 143.

In some embodiments, the anti-FXIIa antibody comprises a V_{H} comprising the amino acid sequence of SEQ ID NO: 98, or a variant thereof having at least about 80% (such as at least about any of 80%, 85%, 90%, 95%, 96%, 97%, 98%, or 99%) sequence identity to the amino acid sequence of SEQ ID NO: 98, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 98 and a V_{L} comprising the amino acid sequence of SEQ ID NO: 140.

In some embodiments, the anti-FXIIa antibody comprises a V_{H} comprising the amino acid sequence of SEQ ID NO: 96, or a variant thereof having at least about 80% (such as at least about any of 80%, 85%, 90%, 95%, 96%, 97%, 98%, or 99%) sequence identity to the amino acid sequence of SEQ ID NO: 96, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 96 and a V_{L} comprising the amino acid sequence of SEQ ID NO: 140.

In some embodiments, the anti-FXIIa antibody comprises a V_{H} comprising the amino acid sequence of SEQ ID NO: 99, or a variant thereof having at least about 80% (such as at least about any of 80%, 85%, 90%, 95%, 96%, 97%, 98%, or 99%) sequence identity to the amino acid sequence of SEQ ID NO: 99, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 99 and a V_{L} comprising the amino acid sequence of SEQ ID NO: 140.

In some embodiments, the anti-FXIIa antibody comprises a V_{H} comprising the amino acid sequence of SEQ ID NO: 94, or a variant thereof having at least about 80% (such as at least about any of 80%, 85%, 90%, 95%, 96%, 97%, 98%, or 99%) sequence identity to the amino acid sequence of SEQ ID NO: 94, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 94 and a V_{L} comprising the amino acid sequence of SEQ ID NO: 142.

In some embodiments, the anti-FXIIa antibody comprises a V_{H} comprising the amino acid sequence of SEQ ID NO: 100, or a variant thereof having at least about 80% (such as at least about any of 80%, 85%, 90%, 95%, 96%, 97%, 98%, or 99%) sequence identity to the amino acid sequence of SEQ ID NO: 100, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 100 and a V_{L} comprising the amino acid sequence of SEQ ID NO: 140.

In some embodiments, the anti-FXIIa antibody comprises a V_{H} comprising the amino acid sequence of SEQ ID NO: 101, or a variant thereof having at least about 80% (such as at least about any of 80%, 85%, 90%, 95%, 96%, 97%, 98%, or 99%) sequence identity to the amino acid sequence of SEQ ID NO: 101, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 101 and a V_{L} comprising the amino acid sequence of SEQ ID NO: 140.

In some embodiments, the anti-FXIIa antibody comprises a V_{H} comprising the amino acid sequence of SEQ ID NO: 102, or a variant thereof having at least about 80% (such as at least about any of 80%, 85%, 90%, 95%, 96%, 97%, 98%, or 99%) sequence identity to the amino acid sequence of SEQ ID NO: 102, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 102 and a V_{L} comprising the amino acid sequence of SEQ ID NO: 142.

In some embodiments, the anti-FXIIa antibody comprises a V_{H} comprising the amino acid sequence of SEQ ID NO: 103, or a variant thereof having at least about 80% (such as at least about any of 80%, 85%, 90%, 95%, 96%, 97%, 98%, or 99%) sequence identity to the amino acid sequence of SEQ ID NO: 103, and a V_{L} comprising the amino acid sequence of SEQ ID NO: 144, or a variant thereof having at least about 80% (such as at least about any of 80%, 85%, 90%, 95%, 96%, 97%, 98%, or 99%) sequence identity to the amino acid sequence of SEQ ID NO: 144. In some embodiments, the anti-FXIIa antibody comprises a V_{H} comprising the amino acid sequence of SEQ ID NO: 103 and a V_{L} comprising the amino acid sequence of SEQ ID NO: 144.

In some embodiments, the anti-FXIIa antibody comprises a V_{H} comprising the amino acid sequence of SEQ ID NO: 104, or a variant thereof having at least about 80% (such as at least about any of 80%, 85%, 90%, 95%, 96%, 97%, 98%, or 99%) sequence identity to the amino acid sequence of SEQ ID NO: 104, and a V_{L} comprising the amino acid sequence of SEQ ID NO: 145, or a variant thereof having at least about 80% (such as at least about any of 80%, 85%, 90%, 95%, 96%, 97%, 98%, or 99%) sequence identity to the amino acid sequence of SEQ ID NO: 145. In some embodiments, the anti-FXIIa antibody comprises a V_{H} comprising the amino acid sequence of SEQ ID NO: 104 and a V_{L} comprising the amino acid sequence of SEQ ID NO: 145.

In some embodiments, the anti-FXIIa antibody comprises a V_{H} comprising the amino acid sequence of SEQ ID NO: 105, or a variant thereof having at least about 80% (such as at least about any of 80%, 85%, 90%, 95%, 96%, 97%, 98%, or 99%) sequence identity to the amino acid sequence of SEQ ID NO: 105, and a V_{L} comprising the amino acid sequence of SEQ ID NO: 141, or a variant thereof having at least about 80% (such as at least about any of 80%, 85%, 90%, 95%, 96%, 97%, 98%, or 99%) sequence identity to the amino acid sequence of SEQ ID NO: 141. In some embodiments, the anti-FXIIa antibody comprises a V_{H} comprising the amino acid sequence of SEQ ID NO: 105 and a V_{L} comprising the amino acid sequence of SEQ ID NO: 141.

In some embodiments, the anti-FXIIa antibody comprises a V_{H} comprising the amino acid sequence of SEQ ID NO: 106, or a variant thereof having at least about 80% (such as at least about any of 80%, 85%, 90%, 95%, 96%, 97%, 98%, or 99%) sequence identity to the amino acid sequence of SEQ ID NO: 106, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 106 and a V_{L} comprising the amino acid sequence of SEQ ID NO: 140.

In some embodiments, the anti-FXIIa antibody comprises a V_{H} comprising the amino acid sequence of SEQ ID NO: 107, or a variant thereof having at least about 80% (such as at least about any of 80%, 85%, 90%, 95%, 96%, 97%, 98%, or 99%) sequence identity to the amino acid sequence of SEQ ID NO: 107, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 107 and a V_{L} comprising the amino acid sequence of SEQ ID NO: 140.

In some embodiments, the anti-FXIIa antibody comprises a V_{H} comprising the amino acid sequence of SEQ ID NO: 108, or a variant thereof having at least about 80% (such as at least about any of 80%, 85%, 90%, 95%, 96%, 97%, 98%, or 99%) sequence identity to the amino acid sequence of SEQ ID NO: 108, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 108 and a V_{L} comprising the amino acid sequence of SEQ ID NO: 142.

In some embodiments, the anti-FXIIa antibody comprises a V_{H} comprising the amino acid sequence of SEQ ID NO: 109, or a variant thereof having at least about 80% (such as at least about any of 80%, 85%, 90%, 95%, 96%, 97%, 98%, or 99%) sequence identity to the amino acid sequence of SEQ ID NO: 109, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 109 and a V_{L} comprising the amino acid sequence of SEQ ID NO: 142.

In some embodiments, the anti-FXIIa antibody comprises a V_{H} comprising the amino acid sequence of SEQ ID NO: 110, or a variant thereof having at least about 80% (such as at least about any of 80%, 85%, 90%, 95%, 96%, 97%, 98%, or 99%) sequence identity to the amino acid sequence of SEQ ID NO: 110, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 110 and a V_{L} comprising the amino acid sequence of SEQ ID NO: 140.

In some embodiments, the anti-FXIIa antibody comprises a V_{H} comprising the amino acid sequence of SEQ ID NO: 111, or a variant thereof having at least about 80% (such as at least about any of 80%, 85%, 90%, 95%, 96%, 97%, 98%, or 99%) sequence identity to the amino acid sequence of SEQ ID NO: 111, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 111 and a V_{L} comprising the amino acid sequence of SEQ ID NO: 140.

In some embodiments, the anti-FXIIa antibody comprises a V_{H} comprising the amino acid sequence of SEQ ID NO: 112, or a variant thereof having at least about 80% (such as at least about any of 80%, 85%, 90%, 95%, 96%, 97%, 98%, or 99%) sequence identity to the amino acid sequence of SEQ ID NO: 112, and a V_{L} comprising the amino acid sequence of SEQ ID NO: 141, or a variant thereof having at least about 80% (such as at least about any of 80%, 85%, 90%, 95%, 96%, 97%, 98%, or 99%) sequence identity to the amino acid sequence of SEQ ID NO: 141. In some embodiments, the anti-FXIIa antibody comprises a V_{H} comprising the amino acid sequence of SEQ ID NO: 112 and a V_{L} comprising the amino acid sequence of SEQ ID NO: 141.

In some embodiments, the anti-FXIIa antibody comprises a V_{H} comprising the amino acid sequence of SEQ ID NO: 113, or a variant thereof having at least about 80% (such as at least about any of 80%, 85%, 90%, 95%, 96%, 97%, 98%, or 99%) sequence identity to the amino acid sequence of SEQ ID NO: 113, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 113 and a V_{L} comprising the amino acid sequence of SEQ ID NO: 142.

In some embodiments, the anti-FXIIa antibody comprises a V_{H} comprising the amino acid sequence of SEQ ID NO: 114, or a variant thereof having at least about 80% (such as at least about any of 80%, 85%, 90%, 95%, 96%, 97%, 98%, or 99%) sequence identity to the amino acid sequence of SEQ ID NO: 114, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 114 and a V_{L} comprising the amino acid sequence of SEQ ID NO: 142.

In some embodiments, the anti-FXIIa antibody comprises a V_{H} comprising the amino acid sequence of SEQ ID NO: 108, or a variant thereof having at least about 80% (such as at least about any of 80%, 85%, 90%, 95%, 96%, 97%, 98%, or 99%) sequence identity to the amino acid sequence of SEQ ID NO: 108, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 108 and a V_{L} comprising the amino acid sequence of SEQ ID NO: 140.

In some embodiments, the anti-FXIIa antibody comprises a V_{H} comprising the amino acid sequence of SEQ ID NO: 93, or a variant thereof having at least about 80% (such as at least about any of 80%, 85%, 90%, 95%, 96%, 97%, 98%, or 99%) sequence identity to the amino acid sequence of SEQ ID NO: 93, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 93 and a V_{L} comprising the amino acid sequence of SEQ ID NO: 140.

In some embodiments, the anti-FXIIa antibody comprises a V_{H} comprising the amino acid sequence of SEQ ID NO: 115, or a variant thereof having at least about 80% (such as at least about any of 80%, 85%, 90%, 95%, 96%, 97%, 98%, or 99%) sequence identity to the amino acid sequence of SEQ ID NO: 115, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 115 and a V_{L} comprising the amino acid sequence of SEQ ID NO: 140.

In some embodiments, the anti-FXIIa antibody comprises a V_{H} comprising the amino acid sequence of SEQ ID NO: 116, or a variant thereof having at least about 80% (such as at least about any of 80%, 85%, 90%, 95%, 96%, 97%, 98%, or 99%) sequence identity to the amino acid sequence of SEQ ID NO: 116, and a V_{L} comprising the amino acid sequence of SEQ ID NO: 146, or a variant thereof having at least about 80% (such as at least about any of 80%, 85%, 90%, 95%, 96%, 97%, 98%, or 99%) sequence identity to the amino acid sequence of SEQ ID NO: 146. In some embodiments, the anti-FXIIa antibody comprises a V_{H} comprising the amino acid sequence of SEQ ID NO: 116 and a V_{L} comprising the amino acid sequence of SEQ ID NO: 146.

In some embodiments, the anti-FXIIa antibody comprises a V_{H} comprising the amino acid sequence of SEQ ID NO: 117, or a variant thereof having at least about 80% (such as at least about any of 80%, 85%, 90%, 95%, 96%, 97%, 98%, or 99%) sequence identity to the amino acid sequence of SEQ ID NO: 117, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 117 and a V_{L} comprising the amino acid sequence of SEQ ID NO: 142.

In some embodiments, the anti-FXIIa antibody comprises a V_{H} comprising the amino acid sequence of SEQ ID NO: 118, or a variant thereof having at least about 80% (such as at least about any of 80%, 85%, 90%, 95%, 96%, 97%, 98%, or 99%) sequence identity to the amino acid sequence of SEQ ID NO: 118, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 118 and a V_{L} comprising the amino acid sequence of SEQ ID NO: 140.

In some embodiments, the anti-FXIIa antibody comprises a V_{H} comprising the amino acid sequence of SEQ ID NO: 104, or a variant thereof having at least about 80% (such as at least about any of 80%, 85%, 90%, 95%, 96%, 97%, 98%, or 99%) sequence identity to the amino acid sequence of SEQ ID NO: 104, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 104 and a V_{L} comprising the amino acid sequence of SEQ ID NO: 142.

In some embodiments, the anti-FXIIa antibody comprises a V_{H} comprising the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 119 and a V_{L} comprising the amino acid sequence of SEQ ID NO: 140.

In some embodiments, the anti-FXIIa antibody comprises a V_{H} comprising the amino acid sequence of SEQ ID NO: 120, or a variant thereof having at least about 80% (such as at least about any of 80%, 85%, 90%, 95%, 96%, 97%, 98%, or 99%) sequence identity to the amino acid sequence of SEQ ID NO: 120, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 120 and a V_{L} comprising the amino acid sequence of SEQ ID NO: 140.

In some embodiments, the anti-FXIIa antibody comprises a V_{H} comprising the amino acid sequence of SEQ ID NO: 111, or a variant thereof having at least about 80% (such as at least about any of 80%, 85%, 90%, 95%, 96%, 97%, 98%, or 99%) sequence identity to the amino acid sequence of SEQ ID NO: 111, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 111 and a V_{L} comprising the amino acid sequence of SEQ ID NO: 142.

In some embodiments, the anti-FXIIa antibody comprises a V_{H} comprising the amino acid sequence of SEQ ID NO: 121, or a variant thereof having at least about 80% (such as at least about any of 80%, 85%, 90%, 95%, 96%, 97%, 98%, or 99%) sequence identity to the amino acid sequence of SEQ ID NO: 121, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 121 and a V_{L} comprising the amino acid sequence of SEQ ID NO: 140.

In some embodiments, the anti-FXIIa antibody comprises a V_{H} comprising the amino acid sequence of SEQ ID NO: 122, or a variant thereof having at least about 80% (such as at least about any of 80%, 85%, 90%, 95%, 96%, 97%, 98%, or 99%) sequence identity to the amino acid sequence of SEQ ID NO: 122, and a V_{L} comprising the amino acid sequence of SEQ ID NO: 141, or a variant thereof having at least about 80% (such as at least about any of 80%, 85%, 90%, 95%, 96%, 97%, 98%, or 99%) sequence identity to the amino acid sequence of SEQ ID NO: 141. In some embodiments, the anti-FXIIa antibody comprises a V_{H} comprising the amino acid sequence of SEQ ID NO: 122 and a V_{L} comprising the amino acid sequence of SEQ ID NO: 141.

In some embodiments, the anti-FXIIa antibody comprises a V_{H} comprising the amino acid sequence of SEQ ID NO: 123, or a variant thereof having at least about 80% (such as at least about any of 80%, 85%, 90%, 95%, 96%, 97%, 98%, or 99%) sequence identity to the amino acid sequence of SEQ ID NO: 123, and a V_{L} comprising the amino acid sequence of SEQ ID NO: 142, or a variant thereof having at least about 80% (such as at least about any of 80%, 85%, 90%, 95%, 96%, 97%, 98%, or 99%) sequence identity to the amino acid sequence of SEQ ID NO: 142. In some embodiments, the anti-FXIIa antibody comprises a V_{H} comprising the amino acid sequence of SEQ ID NO: 123 and a V_{L} comprising the amino acid sequence of SEQ ID NO: 142.

In some embodiments, the anti-FXIIa antibody comprises a V_{H} comprising the amino acid sequence of SEQ ID NO: 124, or a variant thereof having at least about 80% (such as at least about any of 80%, 85%, 90%, 95%, 96%, 97%, 98%, or 99%) sequence identity to the amino acid sequence of SEQ ID NO: 124, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 124 and a V_{L} comprising the amino acid sequence of SEQ ID NO: 140.

In some embodiments, the anti-FXIIa antibody comprises a V_{H} comprising the amino acid sequence of SEQ ID NO: 125, or a variant thereof having at least about 80% (such as at least about any of 80%, 85%, 90%, 95%, 96%, 97%, 98%, or 99%) sequence identity to the amino acid sequence of SEQ ID NO: 125, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 125 and a V_{L} comprising the amino acid sequence of SEQ ID NO: 140.

In some embodiments, the anti-FXIIa antibody comprises a V_{H} comprising the amino acid sequence of SEQ ID NO: 126, or a variant thereof having at least about 80% (such as at least about any of 80%, 85%, 90%, 95%, 96%, 97%, 98%, or 99%) sequence identity to the amino acid sequence of SEQ ID NO: 126, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 126 and a V_{L} comprising the amino acid sequence of SEQ ID NO: 140.

In some embodiments, the anti-FXIIa antibody comprises a V_{H} comprising the amino acid sequence of SEQ ID NO: 112, or a variant thereof having at least about 80% (such as at least about any of 80%, 85%, 90%, 95%, 96%, 97%, 98%, or 99%) sequence identity to the amino acid sequence of SEQ ID NO: 112, and a V_{L} comprising the amino acid sequence of SEQ ID NO: 147, or a variant thereof having at least about 80% (such as at least about any of 80%, 85%, 90%, 95%, 96%, 97%, 98%, or 99%) sequence identity to the amino acid sequence of SEQ ID NO: 147. In some embodiments, the anti-FXIIa antibody comprises a V_{H} comprising the amino acid sequence of SEQ ID NO: 112 and a V_{L} comprising the amino acid sequence of SEQ ID NO: 147.

In some embodiments, the anti-FXIIa antibody comprises a V_{H} comprising the amino acid sequence of SEQ ID NO: 108, or a variant thereof having at least about 80% (such as at least about any of 80%, 85%, 90%, 95%, 96%, 97%, 98%, or 99%) sequence identity to the amino acid sequence of SEQ ID NO: 108, and a V_{L} comprising the amino acid sequence of SEQ ID NO: 148, or a variant thereof having at least about 80% (such as at least about any of 80%, 85%, 90%, 95%, 96%, 97%, 98%, or 99%) sequence identity to the amino acid sequence of SEQ ID NO: 148. In some embodiments, the anti-FXIIa antibody comprises a V_{H} comprising the amino acid sequence of SEQ ID NO: 108 and a V_{L} comprising the amino acid sequence of SEQ ID NO: 148.

In some embodiments, the anti-FXIIa antibody comprises a V_{H} comprising the amino acid sequence of SEQ ID NO: 127, or a variant thereof having at least about 80% (such as at least about any of 80%, 85%, 90%, 95%, 96%, 97%, 98%, or 99%) sequence identity to the amino acid sequence of SEQ ID NO: 127, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 127 and a V_{L} comprising the amino acid sequence of SEQ ID NO: 140.

In some embodiments, the anti-FXIIa antibody comprises a V_{H} comprising the amino acid sequence of SEQ ID NO: 128, or a variant thereof having at least about 80% (such as at least about any of 80%, 85%, 90%, 95%, 96%, 97%, 98%, or 99%) sequence identity to the amino acid sequence of SEQ ID NO: 128, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 128 and a V_{L} comprising the amino acid sequence of SEQ ID NO: 140.

In some embodiments, the anti-FXIIa antibody comprises a V_{H} comprising the amino acid sequence of SEQ ID NO: 129, or a variant thereof having at least about 80% (such as at least about any of 80%, 85%, 90%, 95%, 96%, 97%, 98%, or 99%) sequence identity to the amino acid sequence of SEQ ID NO: 129, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 129 and a V_{L} comprising the amino acid sequence of SEQ ID NO: 140.

In some embodiments, the anti-FXIIa antibody comprises a V_{H} comprising the amino acid sequence of SEQ ID NO: 130, or a variant thereof having at least about 80% (such as at least about any of 80%, 85%, 90%, 95%, 96%, 97%, 98%, or 99%) sequence identity to the amino acid sequence of SEQ ID NO: 130, and a V_{L} comprising the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140. In some embodiments, the anti-FXIIa antibody comprises a V_{H} comprising the amino acid sequence of SEQ ID NO: 130 and a V_{L} comprising the amino acid sequence of SEQ ID NO: 140.

In some embodiments, the anti-FXIIa antibody comprises a V_{H} comprising: an HC-CDR1 comprising the amino acid sequence of SEQ ID NO: 2, an HC-CDR2 comprising the amino acid sequence of SEQ ID NO: 26, and an HC-CDR3 comprising the amino acid sequence of SEQ ID NO: 43, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 66, and an LC-CDR3 comprising the amino acid sequence of SEQ ID NO: 81, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 2, an HC-CDR2 comprising the amino acid sequence of SEQ ID NO: 26, and an HC-CDR3 comprising the amino acid sequence of SEQ ID NO: 43; and a V_{L} comprising an LC-CDR1 comprising the amino acid sequence of SEQ ID NO: 57, an LC-CDR2 comprising the amino acid sequence of SEQ ID NO: 66, and an LC-CDR3 comprising the amino acid sequence of SEQ ID NO: 81.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 131, and a V_{L} comprising an LC-CDR1, an LC-CDR2 and an LC-CDR3 of the V_{L} comprising the amino acid sequence of SEQ ID NO: 149.

In some embodiments, the anti-FXIIa antibody comprises a V_{H} comprising the amino acid sequence of SEQ ID NO: 131, or a variant thereof having at least about 80% (such as at least about any of 80%, 85%, 90%, 95%, 96%, 97%, 98%, or 99%) sequence identity to the amino acid sequence of SEQ ID NO: 131, and a V_{L} comprising the amino acid sequence of SEQ ID NO: 149, or a variant thereof having at least about 80% (such as at least about any of 80%, 85%, 90%, 95%, 96%, 97%, 98%, or 99%) sequence identity to the amino acid sequence of SEQ ID NO: 149. In some embodiments, the anti-FXIIa antibody comprises a V_{H} comprising the amino acid sequence of SEQ ID NO: 131 and a V_{L} comprising the amino acid sequence of SEQ ID NO: 149.

In some embodiments, the anti-FXIIa antibody comprises a V_{H} comprising: an HC-CDR1 comprising the amino acid sequence of SEQ ID NO: 3, an HC-CDR2 comprising the amino acid sequence of SEQ ID NO: 27, and an HC-CDR3 comprising the amino acid sequence of SEQ ID NO: 44, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 67, and an LC-CDR3 comprising the amino acid sequence of SEQ ID NO: 82, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 3, an HC-CDR2 comprising the amino acid sequence of SEQ ID NO: 27, and an HC-CDR3 comprising the amino acid sequence of SEQ ID NO: 44; and a V_{L} comprising an LC-CDR1 comprising the amino acid sequence of SEQ ID NO: 58, an LC-CDR2 comprising the amino acid sequence of SEQ ID NO: 67, and an LC-CDR3 comprising the amino acid sequence of SEQ ID NO: 82.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 132, and a V_{L} comprising an LC-CDR1, an LC-CDR2 and an LC-CDR3 of the V_{L} comprising the amino acid sequence of SEQ ID NO: 150.

In some embodiments, the anti-FXIIa antibody comprises a V_{H} comprising the amino acid sequence of SEQ ID NO: 132, or a variant thereof having at least about 80% (such as at least about any of 80%, 85%, 90%, 95%, 96%, 97%, 98%, or 99%) sequence identity to the amino acid sequence of SEQ ID NO: 132, and a V_{L} comprising the amino acid sequence of SEQ ID NO: 150, or a variant thereof having at least about 80% (such as at least about any of 80%, 85%, 90%, 95%, 96%, 97%, 98%, or 99%) sequence identity to the amino acid sequence of SEQ ID NO: 150. In some embodiments, the anti-FXIIa antibody comprises a V_{H} comprising the amino acid sequence of SEQ ID NO: 132 and a V_{L} comprising the amino acid sequence of SEQ ID NO: 150.

In some embodiments, the anti-FXIIa antibody comprises a V_{H} comprising: an HC-CDR1 comprising the amino acid sequence of SEQ ID NO: 4, an HC-CDR2 comprising the amino acid sequence of SEQ ID NO: 28, and an HC-CDR3 comprising the amino acid sequence of SEQ ID NO: 45, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 68, and an LC-CDR3 comprising the amino acid sequence of SEQ ID NO: 83, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 4, an HC-CDR2 comprising the amino acid sequence of SEQ ID NO: 28, and an HC-CDR3 comprising the amino acid sequence of SEQ ID NO: 45; and a V_{L} comprising an LC-CDR1 comprising the amino acid sequence of SEQ ID NO: 59, an LC-CDR2 comprising the amino acid sequence of SEQ ID NO: 68, and an LC-CDR3 comprising the amino acid sequence of SEQ ID NO: 83.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 133, and a V_{L} comprising an LC-CDR1, an LC-CDR2 and an LC-CDR3 of the V_{L} comprising the amino acid sequence of SEQ ID NO: 151.

In some embodiments, the anti-FXIIa antibody comprises a V_{H} comprising the amino acid sequence of SEQ ID NO: 133, or a variant thereof having at least about 80% (such as at least about any of 80%, 85%, 90%, 95%, 96%, 97%, 98%, or 99%) sequence identity to the amino acid sequence of SEQ ID NO: 133, and a V_{L} comprising the amino acid sequence of SEQ ID NO: 151, or a variant thereof having at least about 80% (such as at least about any of 80%, 85%, 90%, 95%, 96%, 97%, 98%, or 99%) sequence identity to the amino acid sequence of SEQ ID NO: 151. In some embodiments, the anti-FXIIa antibody comprises a V_{H} comprising the amino acid sequence of SEQ ID NO: 133 and a V_{L} comprising the amino acid sequence of SEQ ID NO: 151.

In some embodiments, the anti-FXIIa antibody comprises a V_{H} comprising: an HC-CDR1 comprising the amino acid sequence of SEQ ID NO: 5, an HC-CDR2 comprising the amino acid sequence of SEQ ID NO: 29, and an HC-CDR3 comprising the amino acid sequence of SEQ ID NO: 46, or a variant thereof comprising up to about 5 amino acid substitutions in the HC-CDRs; and a V_{L} comprising an LC-CDR1 comprising the amino acid sequence of SEQ ID NO: 60, an LC-CDR2 comprising the amino acid sequence of SEQ ID NO: 69, and an LC-CDR3 comprising the amino acid sequence of SEQ ID NO: 84, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 5, an HC-CDR2 comprising the amino acid sequence of SEQ ID NO: 29, and an HC-CDR3 comprising the amino acid sequence of SEQ ID NO: 46; and a V_{L} comprising an LC-CDR1 comprising the amino acid sequence of SEQ ID NO: 60, an LC-CDR2 comprising the amino acid sequence of SEQ ID NO: 69, and an LC-CDR3 comprising the amino acid sequence of SEQ ID NO: 84.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 134, and a V_{L} comprising an LC-CDR1, an LC-CDR2 and an LC-CDR3 of the V_{L} comprising the amino acid sequence of SEQ ID NO: 152.

In some embodiments, the anti-FXIIa antibody comprises a V_{H} comprising the amino acid sequence of SEQ ID NO: 134, or a variant thereof having at least about 80% (such as at least about any of 80%, 85%, 90%, 95%, 96%, 97%, 98%, or 99%) sequence identity to the amino acid sequence of SEQ ID NO: 134, and a V_{L} comprising the amino acid sequence of SEQ ID NO: 152, or a variant thereof having at least about 80% (such as at least about any of 80%, 85%, 90%, 95%, 96%, 97%, 98%, or 99%) sequence identity to the amino acid sequence of SEQ ID NO: 152. In some embodiments, the anti-FXIIa antibody comprises a V_{H} comprising the amino acid sequence of SEQ ID NO: 134 and a V_{L} comprising the amino acid sequence of SEQ ID NO: 152.

In some embodiments, the anti-FXIIa antibody comprises a V_{H} comprising: an HC-CDR1 comprising the amino acid sequence of SEQ ID NO: 6, an HC-CDR2 comprising the amino acid sequence of SEQ ID NO: 30, and an HC-CDR3 comprising the amino acid sequence of SEQ ID NO: 47, or a variant thereof comprising up to about 5 amino acid substitutions in the HC-CDRs; and a V_{L} comprising an LC-CDR1 comprising the amino acid sequence of SEQ ID NO: 61, an LC-CDR2 comprising the amino acid sequence of SEQ ID NO: 70, and an LC-CDR3 comprising the amino acid sequence of SEQ ID NO: 85, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 6, an HC-CDR2 comprising the amino acid sequence of SEQ ID NO: 30, and an HC-CDR3 comprising the amino acid sequence of SEQ ID NO: 47; and a V_{L} comprising an LC-CDR1 comprising the amino acid sequence of SEQ ID NO: 61, an LC-CDR2 comprising the amino acid sequence of SEQ ID NO: 70, and an LC-CDR3 comprising the amino acid sequence of SEQ ID NO: 85.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 135, and a V_{L} comprising an LC-CDR1, an LC-CDR2 and an LC-CDR3 of the V_{L} comprising the amino acid sequence of SEQ ID NO: 153.

In some embodiments, the anti-FXIIa antibody comprises a V_{H} comprising the amino acid sequence of SEQ ID NO: 135, or a variant thereof having at least about 80% (such as at least about any of 80%, 85%, 90%, 95%, 96%, 97%, 98%, or 99%) sequence identity to the amino acid sequence of SEQ ID NO: 135, and a V_{L} comprising the amino acid sequence of SEQ ID NO: 153, or a variant thereof having at least about 80% (such as at least about any of 80%, 85%, 90%, 95%, 96%, 97%, 98%, or 99%) sequence identity to the amino acid sequence of SEQ ID NO: 153. In some embodiments, the anti-FXIIa antibody comprises a V_{H} comprising the amino acid sequence of SEQ ID NO: 135 and a V_{L} comprising the amino acid sequence of SEQ ID NO: 153.

In some embodiments, the anti-FXIIa antibody comprises a V_{H} comprising: an HC-CDR1 comprising the amino acid sequence of SEQ ID NO: 7, an HC-CDR2 comprising the amino acid sequence of SEQ ID NO: 31, and an HC-CDR3 comprising the amino acid sequence of SEQ ID NO: 48, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 71, and an LC-CDR3 comprising the amino acid sequence of SEQ ID NO: 86, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 7, an HC-CDR2 comprising the amino acid sequence of SEQ ID NO: 31, and an HC-CDR3 comprising the amino acid sequence of SEQ ID NO: 48; and a V_{L} comprising an LC-CDR1 comprising the amino acid sequence of SEQ ID NO: 62, an LC-CDR2 comprising the amino acid sequence of SEQ ID NO: 71, and an LC-CDR3 comprising the amino acid sequence of SEQ ID NO: 86.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 136, and a V_{L} comprising an LC-CDR1, an LC-CDR2 and an LC-CDR3 of the V_{L} comprising the amino acid sequence of SEQ ID NO: 154.

In some embodiments, the anti-FXIIa antibody comprises a V_{H} comprising the amino acid sequence of SEQ ID NO: 136, or a variant thereof having at least about 80% (such as at least about any of 80%, 85%, 90%, 95%, 96%, 97%, 98%, or 99%) sequence identity to the amino acid sequence of SEQ ID NO: 136, and a V_{L} comprising the amino acid sequence of SEQ ID NO: 154, or a variant thereof having at least about 80% (such as at least about any of 80%, 85%, 90%, 95%, 96%, 97%, 98%, or 99%) sequence identity to the amino acid sequence of SEQ ID NO: 154. In some embodiments, the anti-FXIIa antibody comprises a V_{H} comprising the amino acid sequence of SEQ ID NO: 136 and a V_{L} comprising the amino acid sequence of SEQ ID NO: 154.

In some embodiments, the anti-FXIIa antibody comprises a V_{H} comprising: an HC-CDR1 comprising the amino acid sequence of SEQ ID NO: 8, an HC-CDR2 comprising the amino acid sequence of SEQ ID NO: 32, and an HC-CDR3 comprising the amino acid sequence of SEQ ID NO: 49, or a variant thereof comprising up to about 5 amino acid substitutions in the HC-CDRs; and a V_{L} comprising an LC-CDR1 comprising the amino acid sequence of SEQ ID NO: 63, an LC-CDR2 comprising the amino acid sequence of SEQ ID NO: 72, and an LC-CDR3 comprising the amino acid sequence of SEQ ID NO: 87, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 8, an HC-CDR2 comprising the amino acid sequence of SEQ ID NO: 32, and an HC-CDR3 comprising the amino acid sequence of SEQ ID NO: 49; and a V_{L} comprising an LC-CDR1 comprising the amino acid sequence of SEQ ID NO: 63, an LC-CDR2 comprising the amino acid sequence of SEQ ID NO: 72, and an LC-CDR3 comprising the amino acid sequence of SEQ ID NO: 87.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 137, and a V_{L} comprising an LC-CDR1, an LC-CDR2 and an LC-CDR3 of the V_{L} comprising the amino acid sequence of SEQ ID NO: 155.

In some embodiments, the anti-FXIIa antibody comprises a V_{H} comprising the amino acid sequence of SEQ ID NO: 137, or a variant thereof having at least about 80% (such as at least about any of 80%, 85%, 90%, 95%, 96%, 97%, 98%, or 99%) sequence identity to the amino acid sequence of SEQ ID NO: 137, and a V_{L} comprising the amino acid sequence of SEQ ID NO: 155, or a variant thereof having at least about 80% (such as at least about any of 80%, 85%, 90%, 95%, 96%, 97%, 98%, or 99%) sequence identity to the amino acid sequence of SEQ ID NO: 155. In some embodiments, the anti-FXIIa antibody comprises a V_{H} comprising the amino acid sequence of SEQ ID NO: 137 and a V_{L} comprising the amino acid sequence of SEQ ID NO: 155.

In some embodiments, the anti-FXIIa antibody comprises a V_{H} comprising: an HC-CDR1 comprising the amino acid sequence of SEQ ID NO: 9, an HC-CDR2 comprising the amino acid sequence of SEQ ID NO: 33, and an HC-CDR3 comprising the amino acid sequence of SEQ ID NO: 50, or a variant thereof comprising up to about 5 amino acid substitutions in the HC-CDRs; and a V_{L} comprising an LC-CDR1 comprising the amino acid sequence of SEQ ID NO: 64, an LC-CDR2 comprising the amino acid sequence of SEQ ID NO: 73, and an LC-CDR3 comprising the amino acid sequence of SEQ ID NO: 88, or a variant thereof comprising up to about 5 amino acid substitutions in the LC-CDRs.

In some embodiments, the anti-FXIIa antibody comprises a V_{H} comprising: an HC-CDR1 comprising the amino acid sequence of SEQ ID NO: 9, an HC-CDR2 comprising the amino acid sequence of SEQ ID NO: 33, and an HC-CDR3 comprising the amino acid sequence of SEQ ID NO: 50; and a V_{L} comprising an LC-CDR1 comprising the amino acid sequence of SEQ ID NO: 64, an LC-CDR2 comprising the amino acid sequence of SEQ ID NO: 73, and an LC-CDR3 comprising the amino acid sequence of SEQ ID NO: 88.

In some embodiments, the anti-FXIIa antibody comprises a V_{H} comprising an HC-CDR1, an HC-CDR2 and an HC-CDR3 of the V_{H} comprising the amino acid sequence of SEQ ID NO: 138, and a V_{L} comprising an LC-CDR1, an LC-CDR2 and an LC-CDR3 of the V_{L} comprising the amino acid sequence of SEQ ID NO: 156.

In some embodiments, the anti-FXIIa antibody comprises a V_{H} comprising the amino acid sequence of SEQ ID NO: 138, or a variant thereof having at least about 80% (such as at least about any of 80%, 85%, 90%, 95%, 96%, 97%, 98%, or 99%) sequence identity to the amino acid sequence of SEQ ID NO: 138, and a V_{L} comprising the amino acid sequence of SEQ ID NO: 156, or a variant thereof having at least about 80% (such as at least about any of 80%, 85%, 90%, 95%, 96%, 97%, 98%, or 99%) sequence identity to the amino acid sequence of SEQ ID NO: 156. In some embodiments, the anti-FXIIa antibody comprises a V_{H} comprising the amino acid sequence of SEQ ID NO: 138 and a V_{L} comprising the amino acid sequence of SEQ ID NO: 156.

In some embodiments, the amino acid substitutions described above are limited to "exemplary substitutions" shown in Table 4 of this application.In some embodiments, the amino acid substitutions are limited to "preferred substitutions" shown in Table 4 of this application.

In some embodiments, functional epitopes can be mapped by combinatorial alanine scanning. In this process, a combinatorial alanine-scanning strategy can be used to identify amino acids in the FXIIa protein that are necessary for interaction with FXIIa antibodies. In some embodiments, the epitope is conformational and crystal structure of anti-FXIIa antibodies bound to FXIIa may be employed to identify the epitopes.

In some embodiments, the present application provides antibodies which compete with any one of the FXIIa antibodies described herein for binding to FXIIa. In some embodiments, the present application provides antibodies which compete with any one of the anti-FXIIa antibodies provided herein for binding to an epitope on the FXIIa. In some embodiments, an anti-FXIIa antibody is provided that binds to the same epitope as an anti-FXIIa antibody comprising a V_{H} comprising the amino acid sequence of any one of SEQ ID NOs: 93-138, and a V_{L} comprising the amino acid sequence of any one of SEQ ID NOs: 139-156. In some embodiments, an anti-FXIIa antibody is provided that specifically binds to FXIIa competitively with an anti-FXIIa antibody comprising a V_{H} comprising the amino acid sequence of any one of SEQ ID NOs: 93-138 and a V_{L} comprising the amino acid sequence of any one of SEQ ID NOs: 139-156.

In some embodiments, competition assays may be used to identify a monoclonal antibody that competes with an anti-FXIIa antibody described herein for binding to FXIIa. Competition assays can be used to determine whether two antibodies bind to the same epitope by recognizing identical or sterically overlapping epitopes or one antibody competitively inhibits binding of another antibody to the antigen. In certain embodiments, such a competing antibody binds to the same epitope that is bound by an antibody described herein. Exemplary competition assays include, but are not limited to, routine assays such as those provided in Harlow and Lane (1988) Antibodies: A Laboratory Manual ch.14 (Cold Spring Harbor Laboratory, Cold Spring Harbor, N.Y.). Detailed exemplary methods for mapping an epitope to which an antibody binds are provided in Morris (1996) "Epitope Mapping Protocols", in Methods in Molecular Biology vol. 66 (Humana Press, Totowa, N.J.). In some embodiments, two antibodies are said to bind to the same epitope if each blocks binding of the other by 50% or more. In some embodiments, the antibody that competes with an anti-FXIIa antibody described herein is a chimeric, humanized or human antibody.

Exemplary anti-FXIIa antibody sequences are shown in Tables 2 and 3, wherein the CDR numbering is according to the EU index of Kabat. Those skilled in the art will recognize that many algorithms are known for prediction of CDR positions and for delimitation of antibody heavy chain and light chain variable regions. Anti-FXIIa antibodies comprising CDRs, V_{H} and/or V_{L} sequences from antibodies described herein, but based on prediction algorithms other than those exemplified in the tables below, are within the scope of this invention.

**Table 2. Exemplary anti-FXIIa antibody CDR sequences.**

| **Antibody Name** | **HC-CDR1** | **HC-CDR2** | **HC-CDR3** |
|---|---|---|---|
| FX-Y17 | SQTMS (SEQ ID NO: 1) | SISVISGRTYYADSVKG (SEQ ID NO: 10) | SHAFDV (SEQ ID NO: 34) |
| FX-Y17-Y1 | SQTMS (SEQ ID NO: 1) | SISVISRRTYYADSVKG (SEQ ID NO: 11) | SHAFDV (SEQ ID NO: 34) |
| FX-Y17-Y2 | SQTMS (SEQ ID NO: 1) | SISHISGRTYYADSVKG (SEQ ID NO: 12) | SHAFDV (SEQ ID NO: 34) |
| FX-Y17-Y3 | SQTMS (SEQ ID NO: 1) | SISYISGRTYYADSVKG (SEQ ID NO: 13) | SHAFDV (SEQ ID NO: 34) |
| FX-Y17-Y4 | SQTMS (SEQ ID NO: 1) | SSHVISGRTYYADSVKG (SEQ ID NO: 14) | SHAFDV (SEQ ID NO: 34) |
| FX-Y17-Y5 | SQTMS (SEQ ID NO: 1) | SISVISGRYYYADSVKG (SEQ ID NO: 15) | SHAFDV (SEQ ID NO: 34) |
| FX-Y17-Y6 | SQTMS (SEQ ID NO: 1) | SISYISGRTYYADSVKG (SEQ ID NO: 13) | SHAFDV (SEQ ID NO: 34) |
| FX-Y17-Y7 | SQTMS (SEQ ID NO: 1) | SISVISGRAYYADSVKG (SEQ ID NO: 16) | SHAFDV (SEQ ID NO: 34) |
| FX-Y17-Y8 | SQTMS (SEQ ID NO: 1) | SISVISRRTYYADSVKG (SEQ ID NO: 11) | SHAFDV (SEQ ID NO: 34) |
| FX-Y17-Y9 | SQTMS (SEQ ID NO: 1) | SISVISGRKYYADSVKG (SEQ ID NO: 17) | SHAFDV (SEQ ID NO: 34) |
| FX-Y17-Y10 | SQTMS (SEQ ID NO: 1) | SISVISGRAYYADSVKG (SEQ ID NO: 16) | AHAFDV (SEQ ID NO: 35) |
| FX-Y17-Y11 | SQTMS (SEQ ID NO: 1) | SISYISGRTYYADSVKG (SEQ ID NO: 13) | SHAFDL (SEQ ID NO: 36) |
| FX-Y17-Y12 | SQTMS (SEQ ID NO: 1) | SISVISGRTYYADSVKG (SEQ ID NO: 10) | WHAFDV (SEQ ID NO: 37) |
| FX-Y17-Y13 | SQTMS (SEQ ID NO: 1) | SISVISRRTYYADSVKG (SEQ ID NO: 11) | SHAFDV (SEQ ID NO: 34) |
| FX-Y17-Y14 | SQTMS (SEQ ID NO: 1) | SISHISGRTYYADSVKG (SEQ ID NO: 12) | SHRFDV (SEQ ID NO: 38) |
| FX-Y17-Y15 | SQTMS (SEQ ID NO: 1) | SISVISGRPYYADSVKG (SEQ ID NO: 18) | SHAFDV (SEQ ID NO: 34) |
| FX-Y17-Y16 | SQTMS (SEQ ID NO: 1) | SISVISGRSYYADSVKG (SEQ ID NO: 19) | SHAFDV (SEQ ID NO: 34) |
| FX-Y17-Y17 | SQTMS (SEQ ID NO: 1) | SISYISGRTYYADSVKG (SEQ ID NO: 13) | GHAFDV (SEQ ID NO: 39) |
| FX-Y17-Y18 | SQTMS (SEQ ID NO: 1) | SISVISGRSYYADSVKG (SEQ ID NO: 19) | HHAFDV (SEQ ID NO: 40) |
| FX-Y17-Y19 | SQTMS (SEQ ID NO: 1) | SISVISKRTYYADSVKG (SEQ ID NO: 20) | SHAFDV (SEQ ID NO: 34) |
| FX-Y17-Y20 | SQTMS (SEQ ID NO: 1) | SISVISGRSYYADSVKG (SEQ ID NO: 19) | SHAFDV (SEQ ID NO: 34) |
| FX-Y17-Y21 | SQTMS (SEQ ID NO: 1) | SISHISGRTYYADSVKG (SEQ ID NO: 12) | SHAFDV (SEQ ID NO: 34) |
| FX-Y17-Y22 | SQTMS (SEQ ID NO: 1) | SISYISGRTYYADSVKG (SEQ ID NO: 13) | SHAFDV (SEQ ID NO: 34) |
| FX-Y17-Y23 | SQTMS (SEQ ID NO: 1) | SISVISGRSYYADSVKG (SEQ ID NO: 19) | SHAFDV (SEQ ID NO: 34) |
| FX-Y17-Y24 | SQTMS (SEQ ID NO: 1) | SISYISGRTYYADSVKG (SEQ ID NO: 13) | GHAFDV (SEQ ID NO: 39) |
| FX-Y17-Y26 | SQTMS (SEQ ID NO: 1) | SISVISGRTYYADSVKG (SEQ ID NO: 10) | SHAFDV (SEQ ID NO: 34) |
| FX-Y17-Y28 | SQTMS (SEQ ID NO: 1) | SISVISGRTYYADSVKG (SEQ ID NO: 10) | QHAFDV (SEQ ID NO: 41) |
| FX-Y17-Y30 | SQTMS (SEQ ID NO: 1) | SISVISGRTYYADSVKG (SEQ ID NO: 10) | SHAFDV (SEQ ID NO: 34) |
| FX-Y17-Y31 | SQTMS (SEQ ID NO: 1) | SISVISGRNYYADSVKG (SEQ ID NO: 21) | SHAFDV (SEQ ID NO: 34) |
| FX-Y17-Y33 | SQTMS (SEQ ID NO: 1) | SISVISGRKYYADSVKG (SEQ ID NO: 17) | SHAFDV (SEQ ID NO: 34) |
| FX-Y17-Y34 | SQTMS (SEQ ID NO: 1) | SISVISRRTYYADSVKG (SEQ ID NO: 11) | SHAFDV (SEQ ID NO: 34) |
| FX-Y17-Y35 | SQTMS (SEQ ID NO: 1) | SISVISGRDYYADSVKG (SEQ ID NO: 22) | SHAFDV (SEQ ID NO: 34) |
| FX-Y17-Y36 | SQTMS (SEQ ID NO: 1) | SISVISKRTYYADSVKG (SEQ ID NO: 20) | SHAFDV (SEQ ID NO: 34) |
| FX-Y17-Y37 | SQTMS (SEQ ID NO: 1) | SISVISGRSYYADSVKG (SEQ ID NO: 19) | SHAFDV (SEQ ID NO: 34) |
| FX-Y17-Y38 | SQTMS (SEQ ID NO: 1) | SISVISRRTYYADSVKG (SEQ ID NO: 11) | SHAFDV (SEQ ID NO: 34) |
| FX-Y17-Y39 | SQTMS (SEQ ID NO: 1) | SISHISGRTYYADSVKG (SEQ ID NO: 12) | SHDFDV (SEQ ID NO: 42) |
| FX-Y17-Y40 | SQTMS (SEQ ID NO: 1) | SISVISGRTYYADSVKG (SEQ ID NO: 10) | AHAFDV (SEQ ID NO: 35) |
| FX-Y17-Y41 | SQTMS (SEQ ID NO: 1) | SISVISGRRYYADSVKG (SEQ ID NO: 23) | SHAFDV (SEQ ID NO: 34) |
| FX-Y17-Y42 | SQTMS (SEQ ID NO: 1) | SISVISGRMYYADSVKG (SEQ ID NO: 24) | SHAFDV (SEQ ID NO: 34) |
| FX-Y17-Y43 | SQTMS (SEQ ID NO: 1) | SSSVISGRTYYADSVKG (SEQ ID NO: 25) | SHAFDV (SEQ ID NO: 34) |
| FX-Y17-Y45 | SQTMS (SEQ ID NO: 1) | SISHISGRTYYADSVKG (SEQ ID NO: 12) | SHAFDV (SEQ ID NO: 34) |
| FX-Y17-Y46 | SQTMS (SEQ ID NO: 1) | SISYISGRTYYADSVKG (SEQ ID NO: 13) | GHAFDV (SEQ ID NO: 39) |
| FX-Y17-Y47 | SQTMS (SEQ ID NO: 1) | SISVISGRDYYADSVKG (SEQ ID NO: 22) | SHAFDV (SEQ ID NO: 34) |
| FX-Y17-Y48 | SQTMS (SEQ ID NO: 1) | SISVISGRAYYADSVKG (SEQ ID NO: 16) | SHAFDV (SEQ ID NO: 34) |
| FX-Y17-Y50 | SQTMS (SEQ ID NO: 1) | SISVISGRRYYADSVKG (SEQ ID NO: 23) | SHAFDV (SEQ ID NO: 34) |
| FX-Y17-Y51 | SQTMS (SEQ ID NO: 1) | SISVISGRNYYADSVKG (SEQ ID NO: 21) | SHAFDV (SEQ ID NO: 34) |
| Composite 1 | SQTMS (SEQ ID NO: 1) | SX1X2X3ISX4RX5YYADSVK G (SEQ ID NO: 89) Wherein, X₁ is I or S, X₂ is H or S, X₃ is H, V, or Y, X₄ is G, K, or R, and X₅ is A, D, K, M, N, P, R, S, T, or Y | X₁HX₂FDX₃ (SEQ ID NO: 90) Wherein, X₁ is A, G, H, Q, S, or W, X₂ is A, D, or R, and X₃ is L or V |
| FX-Y34 | GYHIH (SEQ ID NO: 2) | WIRVNGGNTMYARKFQG (SEQ ID NO: 26) | RPYCSGGTCSDS (SEQ ID NO: 43) |
| Composite 2 | GYHIH (SEQ ID NO: 2) | WIRVNGGNTMYARKFQG (SEQ ID NO: 26) | RPYCSGGTCSDS (SEQ ID NO: 43) |
| FX-Y39 | DYSMN (SEQ ID NO: 3) | YISNSRSRSSKYYADSVKG (SEQ ID NO: 27) | IGYCKNGVCGGAYDY (SEQ ID NO: 44) |
| Composite 3 | DYSMN (SEQ ID NO: 3) | YISNSRSRSSKYYADSVKG (SEQ ID NO: 27) | IGYCKNGVCGGAYDY (SEQ ID NO: 44) |
| FX-Y42 | SSSYYWV (SEQ ID NO: 4) | SIYHSGSTYYNPSLKS (SEQ ID NO: 28) | AATSGWYLRYWYFDL (SEQ ID NO: 45) |
| Composite 4 | SSSYYWV (SEQ ID NO: 4) | SIYHSGSTYYNPSLKS (SEQ ID NO: 28) | AATSGWYLRYWYFDL (SEQ ID NO: 45) |
| FX-Y48 | HYWMH (SEQ ID NO: 5) | GITSSGGSKYYADSVKG (SEQ ID NO: 29) | RGPPTHDS (SEQ ID NO: 46) |
| Composite 5 | HYWMH (SEQ ID NO: 5) | GITSSGGSKYYADSVKG (SEQ ID NO: 29) | RGPPTHDS (SEQ ID NO: 46) |
| FX-Y51 | SNSADWN (SEQ ID NO: 6) | RTYYRSKWYNDYAVSVKS (SEQ ID NO: 30) | GGPRPLDI (SEQ ID NO: 47) |
| Composite 6 | SNSADWN (SEQ ID NO: 6) | RTYYRSKWYNDYAVSVKS (SEQ ID NO: 30) | GGPRPLDI (SEQ ID NO: 47) |
| FX-Y60 | SNWIG (SEQ ID NO: 7) | IIYPDDSDTRYSPSFQG (SEQ ID NO: 31) | QGSSTYYRVGWFDP (SEQ ID NO: 48) |
| Composite 7 | SNWIG (SEQ ID NO: 7) | IIYPDDSDTRYSPSFQG (SEQ ID NO: 31) | QGSSTYYRVGWFDP (SEQ ID NO: 48) |
| FX-Y64 | TNYMT (SEQ ID NO: 8) | VIYSGGSTYYADSVKG (SEQ ID NO: 32) | GRYYDGVDYGPKYGMD V (SEQ ID NO: 49) |
| Composite 8 | TNYMT (SEQ ID NO: 8) | VIYSGGSTYYADSVKG (SEQ ID NO: 32) | GRYYDGVDYGPKYGMD V (SEQ ID NO: 49) |
| FX-Y66 | SYWMH (SEQ ID NO: 9) | RINLDGSSTGY ADSVRG (SEQ ID NO: 33) | SSRGFDP (SEQ ID NO: 50) |
| Composite 9 | SYWMH (SEQ ID NO: 9) | RINLDGSSTGY ADSVRG (SEQ ID NO: 33) | SSRGFDP (SEQ ID NO: 50) |
| | | | |

| **Antibody Name** | **LC-CDR1** | **LC-CDR2** | **LC-CDR3** |
|---|---|---|---|
| FX-Y17 | RASQSVYSNLA (SEQ ID NO: 51) | GASTRAT (SEQ ID NO: 65) | QQYNKWPLS (SEQ ID NO: 74) |
| FX-Y17-Y1 | RASQSVYSALA (SEQ ID NO: 52) | GASTRAT (SEQ ID NO: 65) | QQYHKWPLS (SEQ ID NO: 75) |
| FX-Y17-Y2 | RASQSVYSALA (SEQ ID NO: 52) | GASTRAT (SEQ ID NO: 65) | QQNNKWPLS (SEQ ID NO: 76) |
| FX-Y17-Y3 | RASQSVWSNLA (SEQ ID NO: 53) | GASTRAT (SEQ ID NO: 65) | QQYHKWPLS (SEQ ID NO: 75) |
| FX-Y17-Y4 | RASQSVYYNLA (SEQ ID NO: 54) | GASTRAT (SEQ ID NO: 65) | QQYHKWPLS (SEQ ID NO: 75) |
| FX-Y17-Y5 | RASQSVYSALA (SEQ ID NO: 52) | GASTRAT (SEQ ID NO: 65) | QQYHKWPLS (SEQ ID NO: 75) |
| FX-Y17-Y6 | RASQSVYSALA (SEQ ID NO: 52) | GASTRAT (SEQ ID NO: 65) | QQYHKWPLS (SEQ ID NO: 75) |
| FX-Y17-Y7 | RASQSVYSALA (SEQ ID NO: 52) | GASTRAT (SEQ ID NO: 65) | QQYHKWPLS (SEQ ID NO: 75) |
| FX-Y17-Y8 | RASQSVWSNLA (SEQ ID NO: 53) | GASTRAT (SEQ ID NO: 65) | QQYHKWPLS (SEQ ID NO: 75) |
| FX-Y17-Y9 | RASQSVYSALA (SEQ ID NO: 52) | GASTRAT (SEQ ID NO: 65) | QQYHKWPLS (SEQ ID NO: 75) |
| FX-Y17-Y10 | RASQSVYSALA (SEQ ID NO: 52) | GASTRAT (SEQ ID NO: 65) | QQYHKWPLS (SEQ ID NO: 75) |
| FX-Y17-Y11 | RASQSVWSNLA (SEQ ID NO: 53) | GASTRAT (SEQ ID NO: 65) | QQYHKWPLS (SEQ ID NO: 75) |
| FX-Y17-Y12 | RASQSLYSHLA (SEQ ID NO: 55) | GASTRAT (SEQ ID NO: 65) | QQYKKWPLS (SEQ ID NO: 77) |
| FX-Y17-Y13 | RASQSVYSNLA (SEQ ID NO: 51) | GASTRAT (SEQ ID NO: 65) | QQYHKWPLS (SEQ ID NO: 75) |
| FX-Y17-Y14 | RASQSVYSALA (SEQ ID NO: 52) | GASTRAT (SEQ ID NO: 65) | QQNNKWPLS (SEQ ID NO: 76) |
| FX-Y17-Y15 | RASQSVYSALA (SEQ ID NO: 52) | GASTRAT (SEQ ID NO: 65) | QQYHKWPLS (SEQ ID NO: 75) |
| FX-Y17-Y16 | RASQSVYSALA (SEQ ID NO: 52) | GASTRAT (SEQ ID NO: 65) | QQYHKWPLS (SEQ ID NO: 75) |
| FX-Y17-Y17 | RASQSVWSNLA (SEQ ID NO: 53) | GASTRAT (SEQ ID NO: 65) | QQYHKWPLS (SEQ ID NO: 75) |
| FX-Y17-Y18 | RASQSVWSNLA (SEQ ID NO: 53) | GASTRAT (SEQ ID NO: 65) | QQYHKWPLS (SEQ ID NO: 75) |
| FX-Y17-Y19 | RASQSVYSALA (SEQ ID NO: 52) | GASTRAT (SEQ ID NO: 65) | QQYHKWPLS (SEQ ID NO: 75) |
| FX-Y17-Y20 | RASQSVYSALA (SEQ ID NO: 52) | GASTRAT (SEQ ID NO: 65) | QQYHKWPLS (SEQ ID NO: 75) |
| FX-Y17-Y21 | RASQSVYSALA (SEQ ID NO: 52) | GASTRAT (SEQ ID NO: 65) | QQNNKWPLS (SEQ ID NO: 76) |
| FX-Y17-Y22 | RASQSVWSNLA (SEQ ID NO: 53) | GASTRAT (SEQ ID NO: 65) | QQYHKWPLS (SEQ ID NO: 75) |
| FX-Y17-Y23 | RASQSVWSNLA (SEQ ID NO: 53) | GASTRAT (SEQ ID NO: 65) | QQYHKWPLS (SEQ ID NO: 75) |
| FX-Y17-Y24 | RASQSVYSALA (SEQ ID NO: 52) | GASTRAT (SEQ ID NO: 65) | QQYHKWPLS (SEQ ID NO: 75) |
| FX-Y17-Y26 | RASQSVYSALA (SEQ ID NO: 52) | GASTRAT (SEQ ID NO: 65) | QQYHKWPLS (SEQ ID NO: 75) |
| FX-Y17-Y28 | RASQSVYSALA (SEQ ID NO: 52) | GASTRAT (SEQ ID NO: 65) | QQYHKWPLS (SEQ ID NO: 75) |
| FX-Y17-Y30 | RASQSVFSNLA (SEQ ID NO: 56) | GASTRAT (SEQ ID NO: 65) | QQYHWWPLS (SEQ ID NO: 78) |
| FX-Y17-Y31 | RASQSVWSNLA (SEQ ID NO: 53) | GASTRAT (SEQ ID NO: 65) | QQYHKWPLS (SEQ ID NO: 75) |
| FX-Y17-Y33 | RASQSVYSALA (SEQ ID NO: 52) | GASTRAT (SEQ ID NO: 65) | QQYHKWPLS (SEQ ID NO: 75) |
| FX-Y17-Y34 | RASQSVWSNLA (SEQ ID NO: 53) | GASTRAT (SEQ ID NO: 65) | QQYHKWPLS (SEQ ID NO: 75) |
| FX-Y17-Y35 | RASQSVYSALA (SEQ ID NO: 52) | GASTRAT (SEQ ID NO: 65) | QQYHKWPLS (SEQ ID NO: 75) |
| FX-Y17-Y36 | RASQSVYSALA (SEQ ID NO: 52) | GASTRAT (SEQ ID NO: 65) | QQYHKWPLS (SEQ ID NO: 75) |
| FX-Y17-Y37 | RASQSVWSNLA (SEQ ID NO: 53) | GASTRAT (SEQ ID NO: 65) | QQYHKWPLS (SEQ ID NO: 75) |
| FX-Y17-Y38 | RASQSVYSALA (SEQ ID NO: 52) | GASTRAT (SEQ ID NO: 65) | QQYHKWPLS (SEQ ID NO: 75) |
| FX-Y17-Y39 | RASQSVYSALA (SEQ ID NO: 52) | GASTRAT (SEQ ID NO: 65) | QQNNKWPLS (SEQ ID NO: 76) |
| FX-Y17-Y40 | RASQSVWSNLA (SEQ ID NO: 53) | GASTRAT (SEQ ID NO: 65) | QQYHKWPLS (SEQ ID NO: 75) |
| FX-Y17-Y41 | RASQSVYSALA (SEQ ID NO: 52) | GASTRAT (SEQ ID NO: 65) | QQYHKWPLS (SEQ ID NO: 75) |
| FX-Y17-Y42 | RASQSVYSALA (SEQ ID NO: 52) | GASTRAT (SEQ ID NO: 65) | QQYHKWPLS (SEQ ID NO: 75) |
| FX-Y17-Y43 | RASQSVYSALA (SEQ ID NO: 52) | GASTRAT (SEQ ID NO: 65) | QQYHKWPLS (SEQ ID NO: 75) |
| FX-Y17-Y45 | RASQSVYSALA (SEQ ID NO: 52) | GASTRAT (SEQ ID NO: 65) | QQNNRWPLS (SEQ ID NO: 79) |
| FX-Y17-Y46 | RASQSVWSNLA (SEQ ID NO: 53) | GASTRAT (SEQ ID NO: 65) | QQYQKWPLS (SEQ ID NO: 80) |
| FX-Y17-Y47 | RASQSVYSALA (SEQ ID NO: 52) | GASTRAT (SEQ ID NO: 65) | QQYHKWPLS (SEQ ID NO: 75) |
| FX-Y17-Y48 | RASQSVYSALA (SEQ ID NO: 52) | GASTRAT (SEQ ID NO: 65) | QQYHKWPLS (SEQ ID NO: 75) |
| FX-Y17-Y50 | RASQSVYSALA (SEQ ID NO: 52) | GASTRAT (SEQ ID NO: 65) | QQYHKWPLS (SEQ ID NO: 75) |
| FX-Y17-Y51 | RASQSVYSALA (SEQ ID NO: 52) | GASTRAT (SEQ ID NO: 65) | QQYHKWPLS (SEQ ID NO: 75) |
| Composite 1 | RASQSX₁X₂X₃X₄LA (SEQ ID NO: 91) Wherein, X₁ is L or V, X₂ is F, W, or Y, X₃ is S or Y, and X₄ is A, H, or N | GASTRAT (SEQ ID NO: 65) | QQX₁X₂X₃WPLS (SEQ ID NO: 92) Wherein, X₁ is N or Y, X₂ is H, K, N, or Q, and X₃ is K, R, or W |
| FX-Y34 | RASESVFSNLA (SEQ ID NO: 57) | DGNKRAT (SEQ ID NO: 66) | HQYNNWPPET (SEQ ID NO: 81) |
| Composite 2 | RASESVFSNLA (SEQ ID NO: 57) | DGNKRAT (SEQ ID NO: 66) | HQYNNWPPET (SEQ ID NO: 81) |
| FX-Y39 | RASQAIRNNLA (SEQ ID NO: 58) | TASSLES (SEQ ID NO: 67) | LQYHDSPWT (SEQ ID NO: 82) |
| Composite 3 | RASQAIRNNLA (SEQ ID NO: 58) | TASSLES (SEQ ID NO: 67) | LQYHDSPWT (SEQ ID NO: 82) |
| FX-Y42 | RANQNINSYLN (SEQ ID NO: 59) | AATRLQS (SEQ ID NO: 68) | QQSYSSLRT (SEQ ID NO: 83) |
| Composite 4 | RANQNINSYLN (SEQ ID NO: 59) | AATRLQS (SEQ ID NO: 68) | QQSYSSLRT (SEQ ID NO: 83) |
| FX-Y48 | QASQDITKYLN (SEQ ID NO: 60) | DASILET (SEQ ID NO: 69) | QHYNSYSWT (SEQ ID NO: 84) |
| Composite 5 | QASQDITKYLN (SEQ ID NO: 60) | DASILET (SEQ ID NO: 69) | QHYNSYSWT (SEQ ID NO: 84) |
| FX-Y51 | KSSQSVLYRSKRKP NLA (SEQ ID NO: 61) | WASTRES (SEQ ID NO: 70) | QQYYSTPLT (SEQ ID NO: 85) |
| Composite 6 | KSSQSVLYRSKRKP NLA (SEQ ID NO: 61) | WASTRES (SEQ ID NO: 70) | QQYYSTPLT (SEQ ID NO: 85) |
| FX-Y60 | RASQSISSYLN (SEQ ID NO: 62) | AASSLQS (SEQ ID NO: 71) | QQSYSTPQT (SEQ ID NO: 86) |
| Composite 7 | RASQSISSYLN (SEQ ID NO: 62) | AASSLQS (SEQ ID NO: 71) | QQSYSTPQT (SEQ ID NO: 86) |
| FX-Y64 | RASQSVRYNLA (SEQ ID NO: 63) | GASIRAT (SEQ ID NO: 72) | QFYGSSPLT (SEQ ID NO: 87) |
| Composite 8 | RASQSVRYNLA (SEQ ID NO: 63) | GASIRAT (SEQ ID NO: 72) | QFYGSSPLT (SEQ ID NO: 87) |
| FX-Y66 | RASQSISSSYLA (SEQ ID NO: 64) | GASRRAT (SEQ ID NO: 73) | QLYGHSAYT (SEQ ID NO: 88) |
| Composite 9 | RASQSISSSYLA (SEQ ID NO: 64) | GASRRAT (SEQ ID NO: 73) | QLYGHSAYT (SEQ ID NO: 88) |

**Table 3. Exemplary sequences.**

| **SEQ ID NO** | **Description** | **Sequence** |
|---|---|---|
| 93 | FX-Y17 V_{H} | EVQLLESGGGLVQPGGSLRLSCAGSGFSFGSQTMSWVRQAPGKGL EWVSSISVISGRTYYADSVKGRFTISRDNSKNTLYLQMNSLRAEDT AVYYCTKSHAFDVWGQGTMVTVSS |
| | FX-Y17-Y26 V_{H} | |
| 94 | FX-Y17-Y1 V_{H} | EVQLLESGGGLVQPGGSLRLSCAGSGFSFGSQTMSWVRQAPGKGL EWVSSISVISRRTYYADSVKGRFTISRDNSKNTLYLQMNSLRAEDT AVYYCTKSHAFDVWGQGTMVTVSS |
| | FX-Y17-Y8 V_{H} | |
| 95 | FX-Y17-Y2 V_{H} | EVQLLESGGGLVQPGGSLRLSCAGSGFSFGSQTMSWVRQAPGKGL EWVSSISHISGRTYYADSVKGRFTISRDNSKNTLYLQMNSLRAEDT AVYYCTKSHAFDVWGQGTMVTVSS |
| 96 | FX-Y17-Y3 V_{H} | EVQLLESGGGLVQPGGSLRLSCAGSGFSFGSQTMSWVRQAPGKGL EWVSSISYISGRTYYADSVKGRFTISRDNSKNTLYLQMNSLRAEDT AVYYCTKSHAFDVWGQGTMVTVSS |
| | FX-Y17-Y6 V_{H} | |
| 97 | FX-Y17-Y4 V_{H} | EVQLLESGGGLVQPGGSLRLSCAGSGFSFGSQTMSWVRQAPGKGL EWVSSSHVISGRTYYADSVKGRFTISRDNSKNTLYLQMNSLRAED TAVYYCTKSHAFDVWGQGTMVTVSS |
| 98 | FX-Y17-Y5 V_{H} | EVQLLESGGGLVQPGGSLRLSCAGSGFSFGSQTMSWVRQAPGKGL EWVSSISVISGRYYYADSVKGRFTISRDNSKNTLYLQMNSLRAEDT AVYYCTKSHAFDVWGQGTMVTVSS |
| 99 | FX-Y17-Y7 V_{H} | EVQLLESGGGLVQPGGSLRLSCAGSGFSFGSQTMSWVRQAPGKGL EWVSSISVISGRAYYADSVKGRFTISRDNSKNTLYLQMNSLRAEDT AVYYCTKSHAFDVWGQGTMVTVSS |
| 100 | FX-Y17-Y9 V_{H} | EVQLLESGGGLVQPGGSLRLSCAGSGFSFGSQTMSWVRQAPGKGL EWVSSISVISGRKYYADSVKGRFTISRDNSKNTLYLQMNSLRAEDT AVYYCAKSHAFDVWGQGTMVTVSS |
| 101 | FX-Y17-Y10 V_{H} | EVQLLESGGGLVQPGGSLRLSCAGSGFSFGSQTMSWVRQAPGKGL EWVSSISVISGRAYYADSVKGRFTISRDNSKNTLYLQMNSLRAEDT AVYYCTKAHAFDVWGQGTMVTVSS |
| 102 | FX-Y17-Y11 V_{H} | EVQLLESGGGLVQPGGSLRLSCAGSGFSFGSQTMSWVRQAPGKGL EWVSSISYISGRTYYADSVKGRFTISRDNSNNTLYLQMNSLRAEEP AVYYCTKSHAFDLWGRGTMATWSP |
| 103 | FX-Y17-Y12 V_{H} | EVQLLESGGGLVQPGGSLRLSCAGSGFSFGSQTMSWVRQAPGKGL EWVSSISVISGRTYYADSVKGRFTISRDNSKNTLYLQMNSLRAEDT AVYYCTKWHAFDVWGQGTMVTVSS |
| 104 | FX-Y17-Y13 V_{H} | EVQLLESGGGLVQPGGSLRLSCAGSGFSFGSQTMSWVRQAPGKGL EWVSSISVISRRTYYADSVKGRFTISRDNSKNTLYLQMNSLRAEDT AVYYCTRSHAFDVWGQGTMVTVSS |
| | FX-Y17-Y34 V_{H} | |
| 105 | FX-Y17-Y14 V_{H} | EVQLLESGGGLVQPGGSLRLSCAGSGFSFGSQTMSWVRQAPGKGL EWVSSISHISGRTYYADSVKGRFTISRDNSKNTLYLQMNSLRAEDT AVYYCTKSHRFDVWGQGTMVTVSS |
| 106 | FX-Y17-Y15 V_{H} | EVQLLESGGGLVQPGGSLRLSCAGSGFSFGSQTMSWVRQAPGKGL EWVSSISVISGRPYYADSVKGRFTISRDNSKNTLYLQMNSLRAEDT AVYYCTKSHAFDVWGQGTMVTVSS |
| 107 | FX-Y17-Y16 V_{H} | EVQLLESGGGLVQPGGSLRLSCAGSGFSFGSQTMSWVRQAPGKGL EWVSSISVISGRSYYADSVKGRFTISRDNSKNTLYLQMNSLRAEDT AVYYCAKSHAFDVWGQGTMVTVSS |
| 108 | FX-Y17-Y17 V_{H} | EVQLLESGGGLVQPGGSLRLSCAGSGFSFGSQTMSWVRQAPGKGL EWVSSISYISGRTYYADSVKGRFTISRDNSKNTLYLQMNSLRAEDT AVYYCTKGHAFDVWGQGTMVTVSS |
| | FX-Y17-Y24 V_{H} | |
| | FX-Y17-Y46 V_{H} | |
| 109 | FX-Y17-Y18 V_{H} | EVQLLESGGGLVQPGGSLRLSCAGSGFSFGSQTMSWVRQAPGKGL EWVSSISVISGRSYYADSVKGRFTISRDNSKNTLYLQMNSLRAEDT AVYYCTKHHAFDVWGQGTMVTVSS |
| 110 | FX-Y17-Y19 V_{H} | EVQLLESGGGLVQPGGSLRLSCAGSGFSFGSQTMSWVRQAPGKGL EWVSSISVISKRTYYADSVKGRFTISRDNSKNTLYLQMNSLRAEDT AVYYCTRSHAFDVWGQGTMVTVSS |
| 111 | FX-Y17-Y20 V_{H} | EVQLLESGGGLVQPGGSLRLSCAGSGFSFGSQTMSWVRQAPGKGL EWVSSISVISGRSYYADSVKGRFTISRDNSKNTLYLQMNSLRAEDT AVYYCTKSHAFDVWGQGTMVTVSS |
| | FX-Y17-Y37 V_{H} | |
| 112 | FX-Y17-Y21 V_{H} | EVQLLESGGGLVQPGGSLRLSCAGSGFSFGSQTMSWVRQAPGKGL EWVSSISHISGRTYYADSVKGRFTISRDNSKNTLYLQMNSLRAEDT AVYYCAKSHAFDVWGQGTMVTVSS |
| | FX-Y17-Y45 V_{H} | |
| 113 | FX-Y17-Y22 V_{H} | EVQLLESGGGLVQPGGSLRLSCAGSGFSFGSQTMSWVRQAPGKGL EWVSSISYISGRTYYADSVKGRFTISRDNSKNTLYLQMNSLRAEDT AVYYCTRSHAFDVWGQGTMVTVSS |
| 114 | FX-Y17-Y23 V_{H} | EVQLLESGGGLVQPGGSLRLSCAGSGFSFGSQTMSWVRQAPGKGL EWVSSISVISGRSYYADSVKGRFTISRDNSKNTLYLQMNSLRAEDT AVYYCTRSHAFDVWGQGTMVTVSS |
| 115 | FX-Y17-Y28 V_{H} | EVQLLESGGGLVQPGGSLRLSCAGSGFSFGSQTMSWVRQAPGKGL EWVSSISVISGRTYYADSVKGRFTISRDNSKNTLYLQMNSLRAEDT AVYYCTKQHAFDVWGQGTMVTVSS |
| 116 | FX-Y17-Y30 V_{H} | EVQLLESGGGLVQPGGSLRLSCAGSGFSFGSQTMSWVRQAPGKGL EWVSSISVISGRTYYADSVKGRFTISRDNSKNTLYLQMNSLRAEDT AVYYCAKSHAFDVWGQGTMVTVSS |
| 117 | FX-Y17-Y31 V_{H} | EVQLLESGGGLVQPGGSLRLSCAGSGFSFGSQTMSWVRQAPGKGL EWVSSISVISGRNYYADSVKGRFTISRDNSKNTLYLQMNSLRAEDT AVYYCAKSHAFDVWGQGTMVTVSS |
| 118 | FX-Y17-Y33 V_{H} | EVQLLESGGGLVQPGGSLRLSCAGSGFSFGSQTMSWVRQAPGKGL EWVSSISVISGRKYYADSVKGRFTISRDNSKNTLYLQMNSLRAEDT AVYYCTKSHAFDVWGQGTMVTVSS |
| 119 | FX-Y17-Y35 V_{H} | EVQLLESGGGLVQPGGSLRLSCAGSGFSFGSQTMSWVRQAPGKGL EWVSSISVISGRDYYADSVKGRFTISRDNSKNTLYLQMNSLRAEDT AVYYCAKSHAFDVWGQGTMVTVSS |
| 120 | FX-Y17-Y36 V_{H} | EVQLLESGGGLVQPGGSLRLSCAGSGFSFGSQTMSWVRQAPGKGL EWVSSISVISKRTYYADSVKGRFTISRDNSKNTLYLQMNSLRAEDT AVYYCTKSHAFDVWGQGTMVTVSS |
| 121 | FX-Y17-Y38 V_{H} | EVQLLESGGGLVQPGGSLRLSCAGSGFSFGSQTMSWVRQAPGKGL EWVSSISVISRRTYYADSVKGRFTISRDNSKNTLYLQMNSLRAEDT AVYYCAKSHAFDVWGQGTMVTVSS |
| 122 | FX-Y17-Y39 V_{H} | EVQLLESGGGLVQPGGSLRLSCAGSGFSFGSQTMSWVRQAPGKGL EWVSSISHISGRTYYADSVKGRFTISRDNSKNTLYLQMNSLRAEDT AVYYCTKSHDFDVWGQGTMVTVSS |
| 123 | FX-Y17-Y40 V_{H} | EVQLLESGGGLVQPGGSLRLSCAGSGFSFGSQTMSWVRQAPGKGL EWVSSISVISGRTYYADSVKGRFTISRDNSKNTLYLQMNSLRAEDT AVYYCTKAHAFDVWGQGTMVTVSS |
| 124 | FX-Y17-Y41 V_{H} | EVQLLESGGGLVQPGGSLRLSCAGSGFSFGSQTMSWVRQAPGKGL EWVSSISVISGRRYYADSVKGRFTISRDNSKNTLYLQMNSLRAEDT AVYYCAKSHAFDVWGQGTMVTVSS |
| 125 | FX-Y17-Y42 V_{H} | EVQLLESGGGLVQPGGSLRLSCAGSGFSLGSQTMSWVRQAPGKGL EWVSSISVISGRMYYADSVKGRFTISRDNSKNTLYLQMNSLRAEDT AVYYCTKSHAFDVWGQGTMVTVSS |
| 126 | FX-Y17-Y43 V_{H} | EVQLLESGGGLVQPGGSLRLSCAGSGFSFGSQTMSWVRQAPGKGL EWVSSSSVISGRTYYADSVKGRFTISRDNSKNTLYLQMNSLRAEDT AVYYCTRSHAFDVWGQGTMVTVSS |
| 127 | FX-Y17-Y47 V_{H} | EVQLLESGGGLVQPGGSLRLSCAGSGFSFGSQTMSWVRQAPGKGL EWVSSISVISGRDYYADSVKGRFTISRDNSKNTLYLQMNSLRAEDT AVYYCTRSHAFDVWGQGTMVTVSS |
| 128 | FX-Y17-Y48 V_{H} | EVQLLESGGGLVQPGGSLRLSCAGSGFSFGSQTMSWVRQAPGKGL EWVSSISVISGRAYYADSVKGRFTISRDNSKNTLYLQMNSLRAEDT AVYYCTRSHAFDVWGQGTMVTVSS |
| 129 | FX-Y17-Y50 V_{H} | EVQLLESGGGLVQPGGSLRLSCAGSGFSFGSQTMSWVRQAPGKGL EWVSSISVISGRRYYADSVKGRFTISRDNSKNTLYLQMNSLRAEDT AVYYCTKSHAFDVWGQGTMVTVSS |
| 130 | FX-Y17-Y51 V_{H} | EVQLLESGGGLVQPGGSLRLSCAGSGFSFGSQTMSWVRQAPGKGL EWVSSISVISGRNYYADSVKGRFTISRDNSKNTLYLQMNSLRAEDT AVYYCTRSHAFDVWGQGTMVTVSS |
| 131 | FX-Y34 V_{H} | EVQLVESGAEVKKPGASVKVSCKASGYTFTGYHIHWVRQAPGQG LEWMGWIRVNGGNTMYARKFQGRVTMTTDTSASTAYMELRNLT SEDTAVYYCARRPYCSGGTCSDSWGQGTLVTVSS |
| 132 | FX-Y39 V_{H} | QVQLQQSGGGLVQTGGSLRLACAGTGFTFSDYSMNWVRQAPGKG LEWVSYISNSRSRSSKYYADSVKGRFTISRDNAKNSLYLQMNSLRA EDTAVYYCARIGYCKNGVCGGAYDYWGQGTLVTVSS |
| 133 | FX-Y42 V_{H} | QVQLQESGPGLVKPSETLSLTCTVSGGSISSSSYYWVWIRQPPGKG LEWIGSIYHSGSTYYNPSLKSRVTISVDTSKNQFSLKLSSVTAADTA VYYCARAATSGWYLRYWYFDLWGRGTLVTVSS |
| 134 | FX-Y48 V_{H} | QVQLVESGGGLVQPGGSLRLSCAASRFAFSHYWMHWVRQAPGK GLEWVSGITSSGGSKYYADSVKGRFTISRDNSENTLYLQMNSLRA DDTAVYYCVARGPPTHDSWGQGTLVTVSS |
| 135 | FX-Y51 V_{H} | QVQLQQSGPRLVKPSQTLSLTCAISGDSVSSNSADWNWIRQSPSRG LEWLGRTYYRSKWYNDYAVSVKSRITINPDTTKNQFSLQLNSVTP EDTAVYYCARGGPRPLDIWGQGTMVTVSS |
| 136 | FX-Y60 V_{H} | QVQLVQSRAEVKKPGESLRISCKASGYRFTSNWIGWVRQMPGKGL EWMGIIYPDDSDTRYSPSFQGQVTISADKSISTAYLEWSSLKASDTA MYYCVRQGSSTYYRVGWFDPWGQGTLVTVSS |
| 137 | FX-Y64 V_{H} | EVQLLESGGGLIQPGGSLRLSCAASAFTVSTNYMTWVRQAPGKGL EWVSVIYSGGSTYYADSVKGRFTISRDNSKNTLYLQMNSLRAEDT AVYYCARGRYYDGVDYGPKYGMDVWGQGTTVTVSS |
| 138 | FX-Y66 V_{H} | EVQLLESGGGLVQPGGSLRLSCAASGFTFSSYWMHWVRQAPGKG LVWVSRINLDGSSTGYADSVRGRFTISRDNAKSTLYLQMNSLRAE DTAVYYCARSSRGFDPWGQGTLVTVSS |
| | | |
| 139 | FX-Y17 V_{L} | EIVLTQSPATLSVSPGERATLSCRASQSVYSNLAWYQQKPGQAPRL LIYGASTRATGIPARFSGSGSGTEFTLTISSLQSEDFAVYYCQQYNK WPLSFGGGTKVEIK |
| 140 | FX-Y17-Y1 V_{L} | |
| | FX-Y17-Y5 V_{L} | |
| | FX-Y17-Y6 V_{L} | |
| | FX-Y17-Y7 V_{L} | |
| | FX-Y17-Y9 V_{L} | |
| | FX-Y17-Y10 V_{L} | |
| | FX-Y17-Y15 V_{L} | |
| | FX-Y17-Y16 V_{L} | |
| | FX-Y17-Y19 V_{L} | EIVLTQSPATLSVSPGERATLSCRASQSVYSALAWYQQKPGQAPRL LIYGASTRATGIPARFSGSGSGTEFTLTISSLQSEDFAVYYCQQYHK WPLSFGGGTKVEIK |
| | FX-Y17-Y20 V_{L} | |
| | FX-Y17-Y24 V_{L} | |
| | FX-Y17-Y26 V_{L} | |
| | FX-Y17-Y28 V_{L} | |
| | FX-Y17-Y33 V_{L} | |
| | FX-Y17-Y35 V_{L} | |
| | FX-Y17-Y36 V_{L} | |
| | FX-Y17-Y38 V_{L} | |
| | FX-Y17-Y41 V_{L} | |
| | FX-Y17-Y42 V_{L} | |
| | FX-Y17-Y43 V_{L} | |
| | FX-Y17-Y47 V_{L} | |
| | FX-Y17-Y48 V_{L} | |
| | FX-Y17-Y50 V_{L} | |
| | FX-Y17-Y51 V_{L} | |
| 141 | FX-Y17-Y2 V_{L} | EIVLTQSPATLSVSPGERATLSCRASQSVYSALAWYQQKPGQAPRL LIYGASTRATGIPARFSGSGSGTEFTLTISSLQSEDFAVYYCQQNNK WPLSFGGGTKVEIK |
| | FX-Y17-Y14 V_{L} | |
| | FX-Y17-Y21 V_{L} | |
| | FX-Y17-Y39 V_{L} | |
| | FX-Y17-Y3 V_{L} | |
| | FX-Y17-Y8 V_{L} | |
| | FX-Y17-Y11 V_{L} | |
| | FX-Y17-Y17 V_{L} | |
| 142 | FX-Y17-Y18 V_{L} | EIVLTQSPATLSVSPGERATLSCRASQSVWSNLAWYQQKPGQAPR LLIYGASTRATGIPARFSGSGSGTEFTLTISSLQSEDFAVYYCQQYH KWPLSFGGGTKVEIK |
| | FX-Y17-Y22 V_{L} | |
| | FX-Y17-Y23 V_{L} | |
| | FX-Y17-Y31 V_{L} | |
| | FX-Y17-Y34 V_{L} | |
| | FX-Y17-Y37 V_{L} | |
| | FX-Y17-Y40 V_{L} | |
| 143 | FX-Y17-Y4 V_{L} | EIVLTQSPATLSVSPGERATLSCRASQSVYYNLAWYQQKPGQAPRL LIYGASTRATGIPARFSGSGSGTEFTLTISSLQSEDFAVYYCQQYHK WPLSFGGGTKVEIK |
| 144 | FX-Y17-Y12 V_{L} | EIVLTQSPATLSVSPGERATLSCRASQSLYSHLAWYQQKPGQAPRL LIYGASTRATGIPARFSGSGSGTEFTLTISSLQSEDFAVYYCQQYKK WPLSFGGGTKVEIK |
| 145 | FX-Y17-Y13 V_{L} | EIVLTQSPATLSVSPGERATLSCRASQSVYSNLAWYQQKPGQAPRL LIYGASTRATGIPARFSGSGSGTEFTLTISSLQSEDFAVYYCQQYHK WPLSFGGGTKVEIK |
| 146 | FX-Y17-Y30 V_{L} | EIVLTQSPATLSVSPGERATLSCRASQSVFSNLAWYQQKPGQAPRL LIYGASTRATGIPARFSGSGSGTEFTLTISSLQSEDFAVYYCQQYHW WPLSFGGGTKVEIK |
| 147 | FX-Y17-Y45 V_{L} | EIVLTQSPATLSVSPGERATLSCRASQSVYSALAWYQQKPGQAPRL LIYGASTRATGIPARFSGSGSGTEFTLTISSLQSEDFAVYYCQQNNR WPLSFGGGTKVEIK |
| 148 | FX-Y17-Y46 V_{L} | EIVLTQSPATLSVSPGERATLSCRASQSVWSNLAWYQQKPGQAPR LLIYGASTRATGIPARFSGSGSGTEFTLTISSLQSEDFAVYYCQQYQ KWPLSFGGGTKVEIK |
| 149 | FX-Y34 V_{L} | ETTLTQSPATLSVSPGERATLSCRASESVFSNLAWYQLRPGQGPRL LIYDGNKRATGIPARFSGSGSGTEFTLTISSLQSEDFGIYYCHQYNN WPPETFGQGTKLEIK |
| 150 | FX-Y39 V_{L} | EIVLTQSPSAMSASVGDRVTITCRASQAIRNNLAWFQQKPGKVPKR LMNTASSLESGVPSRFSGSGSGTEFTLTITSLQPEDFASYYCLQYHD SPWTFGQGTKLEIK |
| 151 | FX-Y42 V_{L} | DIVMTQSPSSLSASVGDRVTITCRANQNINSYLNWYQQKPGKAPEL LIYAATRLQSGVPSRFSGSRSGTDFTLTISSLQPEDFATYYCQQSYSS LRTFGHGTKVDIK |
| 152 | FX-Y48 V_{L} | VIWMTQSPSSLSASVGDRVTITCQASQDITKYLNWYQQKPGKAPK LLIYDASILETGVPSRFSGRGSGTEFTLTINNLQPEDFATYYCQHYN SYSWTFGQGTKLEIK |
| 153 | FX-Y51 V_{L} | EIVLTQSPDSLAVSLGERVTINCKSSQSVLYRSKRKPNLAWYQQKP GHPPKLLVSWASTRESGVPERFTGSGSGTDFTLTISSLQAEDVAVY FCQQYYSTPLTFGGGTKVEIK |
| 154 | FX-Y60 V_{L} | DIQLTQSPSSLSASVGDRVTITCRASQSISSYLNWYQQKPGKAPKLL IYAASSLQSGVPSRFSGSGSGTDFTLTISSLQPEDFATYYCQQSYSTP QTFGQGTKVDIK |
| 155 | FX-Y64 V_{L} | DVVMTQSPGTLSVSPGERATLSCRASQSVRYNLAWYQQKPGQAP RLLMYGASIRATDIPDRFSGSGSGTDFTLTISRLEPEDFAVYYCQFY GSSPLTFGGGTKLEIK |
| 156 | FX-Y66 V_{L} | EIVLTQSPGSLSMSPGERATLSCRASQSISSSYLAWYQQKPGQAPRL LIYGASRRATGIPDRFSGSGSGTDFTLTISRLEPEDLAVYYCQLYGH SAYTFGQGTKVDIK |
| 157 | IgG1 heavy chain constant region | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTYR VVSVLTVLHQDWLNGKEYKCKVSNKALPAPIEKTISKAKGQPREP QVYTLPPSREEMTKNQVSLTCLVKGFYPSDIAVEWESNGQPENNY KTTPPVLDSDGSFFLYSKLTVDKSRWQQGNVFSCSVMHEALHNHY TQKSLSLSPGK |
| 158 | IgG4 heavy chain constant region | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCPSCPAPEFLGGPSVFLFPPKPKDTLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQV YTLPPSQEEMTKNQVSLTCLVKGFYPSDIAVEWESNGQPENNYKT TPPVLDSDGSFFLYSRLTVDKSRWQEGNVFSCSVMHEALHNHYTQ KSLSLSLGK |
| 159 | Light chain constant region(kappa) | |
| 160 | Light chain constant region(lambda) | GQPKAAPSVTLFPPSSEELQANKATLVCLISDFYPGAVTVAWKADS SPVKAGVETTTPSKQSNNKYAASSYLSLTPEQWKSHRSYSCQVTH EGSTVEKTVAPTECS |
| 161 | Native human FXIIa | |

### FXIIa

There are two coagulation convergence pathways triggered by exogenous (vascular wall) or endogenous (blood borne) components of the vascular system. The exogenous pathway is initiated by the complex of factor VII (FVII) and membrane intrinsic protein tissue factor (TF). TF expressed in circulating microvesicles may also promote thrombus propagation by maintaining thrombin production on the surface of activated platelets. When FXII comes into contact with a negatively charged surface in a reaction, endogenous or contact activation pathways are activated. Robert Colman first referred to FXII and contact activation as a surface activated defense mechanism, used to describe contact activation that occurs with foreign pathogens (such as bacteria, viruses, yeast, parasites) or artificial surfaces (such as glass, medical devices) (Colman RW et al., 1997, Blood; 90:3819-43.). FXII has the unique property of self activation from zymogen to active enzyme. When the zymogen is incubated alone with a negatively charged surface, the self activation process of FXII is slow (90-120 minutes). When FXII is incubated with HK and PK or in plasma, the self activation process is greatly accelerated. FXIIa is a serine protease with substrates including PK, FXI, plasminogen, complement component 1r (C1r), C1s, and HK. FXIIa activates PK to pKal, creating a mutual activation state between pKal and FXII. The resulting FXIIa activates more PK, thereby amplifying the activity of the system. HK stabilizes PK to achieve optimal activation of FXIIa. Self activation, reciprocal activation, and amplification are the essence of the Contact Activation System (CAS) (Tankersley DL et al., 1984, Biochemistry; 23:273-9.). The formation of pKal through FXIIa activation or other means signifies the activation of the so-called kallikrein-kinin system. PKal cleaves HK to release bradykinin and kininogen fragments (i.e. cHK). Bradykinin affects vascular permeability by binding to G protein coupled receptors bradykinin B2 and B1 receptors. FXIIa activates FXI to initiate contact activation of coagulation. FXIIa activates plasminogen, while C1r/C1s respectively initiate fibrinolysis and classical complement activation.

### Full-length anti-FXIIa antibody

The anti-FXIIa antibody in some embodiments is a full-length anti-FXIIa antibody. In some embodiments, the full-length anti-FXIIa antibody is an IgA, IgD, IgE, IgG, or IgM. In some embodiments, the full-length anti-FXIIa antibody comprises IgG constant domains, such as constant domains of any one of IgG1, IgG2, IgG3, and IgG4 including variants thereof. In some embodiments, the full-length anti-FXIIa antibody comprises a lambda light chain constant region. In some embodiments, the full-length anti-FXIIa antibody comprises a kappa light chain constant region. In some embodiments, the full-length anti-FXIIa antibody is a full-length human anti-FXIIa antibody. In some embodiments, the full-length anti-FXIIa antibody comprises an Fc sequence of a mouse immunoglobulin. In some embodiments, the full-length anti-FXIIa antibody comprises an Fc sequence that has been altered or otherwise changed so that it has enhanced antibody dependent cellular cytotoxicity (ADCC) or complement dependent cytotoxicity (CDC) effector function.

Thus, for example, in some embodiments, there is provided a full-length anti-FXIIa antibody comprising IgG1 constant domains, wherein the anti-FXIIa antibody specifically binds to FXIIa. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG2 constant domains, wherein the anti-FXIIa antibody specifically binds to FXIIa. In some embodiments, the IgG2 is human IgG2. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a full-length anti-FXIIa antibody comprising IgG3 constant domains, wherein the anti-FXIIa antibody specifically binds to FXIIa. In some embodiments, the IgG3 is human IgG3. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody specifically binds to FXIIa. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of any one of SEQ ID NOs: 1-9, or a variant thereof comprising up to about 3 (such as about any of 1, 2, or 3) amino acid substitutions, an HC-CDR2 comprising the amino acid sequence of any one of SEQ ID NOs: 10-33, or a variant thereof comprising up to about 3 (such as about any of 1, 2, or 3) amino acid substitutions, and an HC-CDR3 comprising the amino acid sequence of any one of SEQ ID NOs: 34-50, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 51-64, or a variant thereof comprising up to about 3 (such as about any of 1, 2, or 3) amino acid substitutions, an LC-CDR2 comprising the amino acid sequence of any one of SEQ ID NOs: 65-73, or a variant thereof comprising up to about 3 (such as about any of 1, 2, or 3) amino acid substitutions, and an LC-CDR3 comprising the amino acid sequence of any one of SEQ ID NOs: 74-88, or a variant thereof comprising up to about 3 (such as about any of 1, 2, or 3) amino acid substitutions. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG2 constant domains, wherein the anti-FXIIa antibody comprises a) a heavy chain variable domain comprising an HC-CDR1 comprising the amino acid sequence of any one of SEQ ID NOs: 1-9, or a variant thereof comprising up to about 3 (such as about any of 1, 2, or 3) amino acid substitutions, an HC-CDR2 comprising the amino acid sequence of any one of SEQ ID NOs: 10-33, or a variant thereof comprising up to about 3 (such as about any of 1, 2, or 3) amino acid substitutions, and an HC-CDR3 comprising the amino acid sequence of any one of SEQ ID NOs: 34-50, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 51-64, or a variant thereof comprising up to about 3 (such as about any of 1, 2, or 3) amino acid substitutions, an LC-CDR2 comprising the amino acid sequence of any one of SEQ ID NOs: 65-73, or a variant thereof comprising up to about 3 (such as about any of 1, 2, or 3) amino acid substitutions, and an LC-CDR3 comprising the amino acid sequence of any one of SEQ ID NOs: 74-88, or a variant thereof comprising up to about 3 (such as about any of 1, 2, or 3) amino acid substitutions. In some embodiments, the IgG2 is human IgG2. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a full-length anti-FXIIa antibody comprising IgG3 constant domains, wherein the anti-FXIIa antibody comprises a) a heavy chain variable domain comprising an HC-CDR1 comprising the amino acid sequence of any one of SEQ ID NOs: 1-9, or a variant thereof comprising up to about 3 (such as about any of 1, 2, or 3) amino acid substitutions, an HC-CDR2 comprising the amino acid sequence of any one of SEQ ID NOs: 10-33, or a variant thereof comprising up to about 3 (such as about any of 1, 2, or 3) amino acid substitutions, and an HC-CDR3 comprising the amino acid sequence of any one of SEQ ID NOs: 34-50, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 51-64, or a variant thereof comprising up to about 3 (such as about any of 1, 2, or 3) amino acid substitutions, an LC-CDR2 comprising the amino acid sequence of any one of SEQ ID NOs: 65-73, or a variant thereof comprising up to about 3 (such as about any of 1, 2, or 3) amino acid substitutions, and an LC-CDR3 comprising the amino acid sequence of any one of SEQ ID NOs: 74-88, or a variant thereof comprising up to about 3 (such as about any of 1, 2, or 3) amino acid substitutions. In some embodiments, the IgG3 is human IgG3. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of any one of SEQ ID NOs: 1-9, or a variant thereof comprising up to about 3 (such as about any of 1, 2, or 3) amino acid substitutions, an HC-CDR2 comprising the amino acid sequence of any one of SEQ ID NOs: 10-33, or a variant thereof comprising up to about 3 (such as about any of 1, 2, or 3) amino acid substitutions, and an HC-CDR3 comprising the amino acid sequence of any one of SEQ ID NOs: 34-50, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 51-64, or a variant thereof comprising up to about 3 (such as about any of 1, 2, or 3) amino acid substitutions, an LC-CDR2 comprising the amino acid sequence of any one of SEQ ID NOs: 65-73, or a variant thereof comprising up to about 3 (such as about any of 1, 2, or 3) amino acid substitutions, and an LC-CDR3 comprising the amino acid sequence of any one of SEQ ID NOs: 74-88, or a variant thereof comprising up to about 3 (such as about any of 1, 2, or 3) amino acid substitutions. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of any one of SEQ ID NOs: 1-9, an HC-CDR2 comprising the amino acid sequence of any one of SEQ ID NOs: 10-33, and an HC-CDR3 comprising the amino acid sequence of any one of SEQ ID NOs: 34-50, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 51-64, an LC-CDR2 comprising the amino acid sequence of any one of SEQ ID NOs: 65-73, and an LC-CDR3 comprising the amino acid sequence of any one of SEQ ID NOs: 74-88, or a variant thereof comprising up to about 5 (such as about any of 1, 2, 3, 4, or 5) amino acid substitutions in the LC-CDR sequences. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of any one of SEQ ID NOs: 1-9, an HC-CDR2 comprising the amino acid sequence of any one of SEQ ID NOs: 10-33, and an HC-CDR3 comprising the amino acid sequence of any one of SEQ ID NOs: 34-50, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 51-64, an LC-CDR2 comprising the amino acid sequence of any one of SEQ ID NOs: 65-73, and an LC-CDR3 comprising the amino acid sequence of any one of SEQ ID NOs: 74-88, or a variant thereof comprising up to about 5 (such as about any of 1, 2, 3, 4, or 5) amino acid substitutions in the LC-CDR sequences. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of any one of SEQ ID NOs: 1-9, an HC-CDR2 comprising the amino acid sequence of any one of SEQ ID NOs: 10-33, and an HC-CDR3 comprising the amino acid sequence of any one of SEQ ID NOs: 34-50; and b) a light chain variable domain comprising an LC-CDR1 comprising the amino acid sequence of any one of SEQ ID NOs: 51-64, an LC-CDR2 comprising the amino acid sequence of any one of SEQ ID NOs: 65-73, and an LC-CDR3 comprising the amino acid sequence of any one of SEQ ID NOs: 74-88. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of any one of SEQ ID NOs: 1-9, an HC-CDR2 comprising the amino acid sequence of any one of SEQ ID NOs: 10-33, and an HC-CDR3 comprising the amino acid sequence of any one of SEQ ID NOs: 34-50; and b) a light chain variable domain comprising an LC-CDR1 comprising the amino acid sequence of any one of SEQ ID NOs: 51-64, an LC-CDR2 comprising the amino acid sequence of any one of SEQ ID NOs: 65-73, and an LC-CDR3 comprising the amino acid sequence of any one of SEQ ID NOs: 74-88. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and b) a light chain variable domain comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34; and b) a light chain variable domain comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37; and b) a light chain variable domain comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and b) a light chain variable domain comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and b) a light chain variable domain comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 2, an HC-CDR2 comprising the amino acid sequence of SEQ ID NO: 26, and an HC-CDR3 comprising the amino acid sequence of SEQ ID NO: 43; and b) a light chain variable domain comprising an LC-CDR1 comprising the amino acid sequence of SEQ ID NO: 57, an LC-CDR2 comprising the amino acid sequence of SEQ ID NO: 66, and an LC-CDR3 comprising the amino acid sequence of SEQ ID NO: 81. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 3, an HC-CDR2 comprising the amino acid sequence of SEQ ID NO: 27, and an HC-CDR3 comprising the amino acid sequence of SEQ ID NO: 44; and b) a light chain variable domain comprising an LC-CDR1 comprising the amino acid sequence of SEQ ID NO: 58, an LC-CDR2 comprising the amino acid sequence of SEQ ID NO: 67, and an LC-CDR3 comprising the amino acid sequence of SEQ ID NO: 82. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 4, an HC-CDR2 comprising the amino acid sequence of SEQ ID NO: 28, and an HC-CDR3 comprising the amino acid sequence of SEQ ID NO: 45; and b) a light chain variable domain comprising an LC-CDR1 comprising the amino acid sequence of SEQ ID NO: 59, an LC-CDR2 comprising the amino acid sequence of SEQ ID NO: 68, and an LC-CDR3 comprising the amino acid sequence of SEQ ID NO: 83. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 5, an HC-CDR2 comprising the amino acid sequence of SEQ ID NO: 29, and an HC-CDR3 comprising the amino acid sequence of SEQ ID NO: 46; and b) a light chain variable domain comprising an LC-CDR1 comprising the amino acid sequence of SEQ ID NO: 60, an LC-CDR2 comprising the amino acid sequence of SEQ ID NO: 69, and an LC-CDR3 comprising the amino acid sequence of SEQ ID NO: 84. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 6, an HC-CDR2 comprising the amino acid sequence of SEQ ID NO: 30, and an HC-CDR3 comprising the amino acid sequence of SEQ ID NO: 47; and b) a light chain variable domain comprising an LC-CDR1 comprising the amino acid sequence of SEQ ID NO: 61, an LC-CDR2 comprising the amino acid sequence of SEQ ID NO: 70, and an LC-CDR3 comprising the amino acid sequence of SEQ ID NO: 85. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 7, an HC-CDR2 comprising the amino acid sequence of SEQ ID NO: 31, and an HC-CDR3 comprising the amino acid sequence of SEQ ID NO: 48; and b) a light chain variable domain comprising an LC-CDR1 comprising the amino acid sequence of SEQ ID NO: 62, an LC-CDR2 comprising the amino acid sequence of SEQ ID NO: 71, and an LC-CDR3 comprising the amino acid sequence of SEQ ID NO: 86. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 8, an HC-CDR2 comprising the amino acid sequence of SEQ ID NO: 32, and an HC-CDR3 comprising the amino acid sequence of SEQ ID NO: 49; and b) a light chain variable domain comprising an LC-CDR1 comprising the amino acid sequence of SEQ ID NO: 63, an LC-CDR2 comprising the amino acid sequence of SEQ ID NO: 72, and an LC-CDR3 comprising the amino acid sequence of SEQ ID NO: 87. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a) a heavy chain variable domain comprising an HC-CDR1 comprising the amino acid sequence of SEQ ID NO: 9, an HC-CDR2 comprising the amino acid sequence of SEQ ID NO: 33, and an HC-CDR3 comprising the amino acid sequence of SEQ ID NO: 50; and b) a light chain variable domain comprising an LC-CDR1 comprising the amino acid sequence of SEQ ID NO: 64, an LC-CDR2 comprising the amino acid sequence of SEQ ID NO: 73, and an LC-CDR3 comprising the amino acid sequence of SEQ ID NO: 88. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and b) a light chain variable domain comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34; and b) a light chain variable domain comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37; and b) a light chain variable domain comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and b) a light chain variable domain comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and b) a light chain variable domain comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and b) a light chain variable domain comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and b) a light chain variable domain comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 2, an HC-CDR2 comprising the amino acid sequence of SEQ ID NO: 26, and an HC-CDR3 comprising the amino acid sequence of SEQ ID NO: 43; and b) a light chain variable domain comprising an LC-CDR1 comprising the amino acid sequence of SEQ ID NO: 57, an LC-CDR2 comprising the amino acid sequence of SEQ ID NO: 66, and an LC-CDR3 comprising the amino acid sequence of SEQ ID NO: 81. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 3, an HC-CDR2 comprising the amino acid sequence of SEQ ID NO: 27, and an HC-CDR3 comprising the amino acid sequence of SEQ ID NO: 44; and b) a light chain variable domain comprising an LC-CDR1 comprising the amino acid sequence of SEQ ID NO: 58, an LC-CDR2 comprising the amino acid sequence of SEQ ID NO: 67, and an LC-CDR3 comprising the amino acid sequence of SEQ ID NO: 82. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 4, an HC-CDR2 comprising the amino acid sequence of SEQ ID NO: 28, and an HC-CDR3 comprising the amino acid sequence of SEQ ID NO: 45; and b) a light chain variable domain comprising an LC-CDR1 comprising the amino acid sequence of SEQ ID NO: 59, an LC-CDR2 comprising the amino acid sequence of SEQ ID NO: 68, and an LC-CDR3 comprising the amino acid sequence of SEQ ID NO: 83. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 5, an HC-CDR2 comprising the amino acid sequence of SEQ ID NO: 29, and an HC-CDR3 comprising the amino acid sequence of SEQ ID NO: 46; and b) a light chain variable domain comprising an LC-CDR1 comprising the amino acid sequence of SEQ ID NO: 60, an LC-CDR2 comprising the amino acid sequence of SEQ ID NO: 69, and an LC-CDR3 comprising the amino acid sequence of SEQ ID NO: 84. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 6, an HC-CDR2 comprising the amino acid sequence of SEQ ID NO: 30, and an HC-CDR3 comprising the amino acid sequence of SEQ ID NO: 47; and b) a light chain variable domain comprising an LC-CDR1 comprising the amino acid sequence of SEQ ID NO: 61, an LC-CDR2 comprising the amino acid sequence of SEQ ID NO: 70, and an LC-CDR3 comprising the amino acid sequence of SEQ ID NO: 85. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 7, an HC-CDR2 comprising the amino acid sequence of SEQ ID NO: 31, and an HC-CDR3 comprising the amino acid sequence of SEQ ID NO: 48; and b) a light chain variable domain comprising an LC-CDR1 comprising the amino acid sequence of SEQ ID NO: 62, an LC-CDR2 comprising the amino acid sequence of SEQ ID NO: 71, and an LC-CDR3 comprising the amino acid sequence of SEQ ID NO: 86. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 8, an HC-CDR2 comprising the amino acid sequence of SEQ ID NO: 32, and an HC-CDR3 comprising the amino acid sequence of SEQ ID NO: 49; and b) a light chain variable domain comprising an LC-CDR1 comprising the amino acid sequence of SEQ ID NO: 63, an LC-CDR2 comprising the amino acid sequence of SEQ ID NO: 72, and an LC-CDR3 comprising the amino acid sequence of SEQ ID NO: 87. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a) a heavy chain variable domain comprising an HC-CDR1 comprising the amino acid sequence of SEQ ID NO: 9, an HC-CDR2 comprising the amino acid sequence of SEQ ID NO: 33, and an HC-CDR3 comprising the amino acid sequence of SEQ ID NO: 50; and b) a light chain variable domain comprising an LC-CDR1 comprising the amino acid sequence of SEQ ID NO: 64, an LC-CDR2 comprising the amino acid sequence of SEQ ID NO: 73, and an LC-CDR3 comprising the amino acid sequence of SEQ ID NO: 88. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of any one of SEQ ID NOs: 93-138, or a variant thereof having at least about 80% (such as at least about any of 80%, 85%, 90%, 95%, 96%, 97%, 98%, or 99%) sequence identity to the amino acid sequence of any one of SEQ ID NOs: 93-138, and a light chain variable domain comprising the amino acid sequence of any one of SEQ ID NOs: 139-156, or a variant thereof having at least about 80% (such as at least about any of 80%, 85%, 90%, 95%, 96%, 97%, 98%, or 99%) sequence identity to the amino acid sequence of any one of SEQ ID NOs: 139-156. In some embodiments, the IgG1 is human IgG1. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG2 constant domains, wherein the anti-FXIIa antibody comprises a heavy chain variable domain comprising the amino acid sequence of any one of SEQ ID NOs: 93-138, or a variant thereof having at least about 80% (such as at least about any of 80%, 85%, 90%, 95%, 96%, 97%, 98%, or 99%) sequence identity to the amino acid sequence of any one of SEQ ID NOs: 93-138, and a light chain variable domain comprising the amino acid sequence of any one of SEQ ID NOs: 139-156, or a variant thereof having at least about 80% (such as at least about any of 80%, 85%, 90%, 95%, 96%, 97%, 98%, or 99%) sequence identity to the amino acid sequence of any one of SEQ ID NOs: 139-156. In some embodiments, the IgG2 is human IgG2. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a full-length anti-FXIIa antibody comprising IgG3 constant domains, wherein the anti-FXIIa antibody comprises a heavy chain variable domain comprising the amino acid sequence of any one of SEQ ID NOs: 93-138, or a variant thereof having at least about 80% (such as at least about any of 80%, 85%, 90%, 95%, 96%, 97%, 98%, or 99%) sequence identity to the amino acid sequence of any one of SEQ ID NOs: 93-138, and a light chain variable domain comprising the amino acid sequence of any one of SEQ ID NOs: 139-156, or a variant thereof having at least about 80% (such as at least about any of 80%, 85%, 90%, 95%, 96%, 97%, 98%, or 99%) sequence identity to the amino acid sequence of any one of SEQ ID NOs: 139-156. In some embodiments, the IgG3 is human IgG3. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of any one of SEQ ID NOs: 93-138, or a variant thereof having at least about 80% (such as at least about any of 80%, 85%, 90%, 95%, 96%, 97%, 98%, or 99%) sequence identity to the amino acid sequence of any one of SEQ ID NOs: 93-138, and a light chain variable domain comprising the amino acid sequence of any one of SEQ ID NOs: 139-156, or a variant thereof having at least about 80% (such as at least about any of 80%, 85%, 90%, 95%, 96%, 97%, 98%, or 99%) sequence identity to the amino acid sequence of any one of SEQ ID NOs: 139-156. In some embodiments, the IgG4 is human IgG4. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of any one of SEQ ID NOs: 93-138, and a light chain variable domain comprising the amino acid sequence of any one of SEQ ID NOs: 139-156. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of any one of SEQ ID NOs: 93-138, and a light chain variable domain comprising the amino acid sequence of any one of SEQ ID NOs: 139-156. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 93, or a variant thereof having at least about 80% sequence identity to the amino acid sequence of SEQ ID NO: 93; and a light chain variable domain comprising the amino acid sequence of SEQ ID NO: 139, or a variant thereof having at least about 80% sequence identity to the amino acid sequence of SEQ ID NO: 139. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 94, or a variant thereof having at least about 80% sequence identity to the amino acid sequence of SEQ ID NO: 94; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 95, or a variant thereof having at least about 80% sequence identity to the amino acid sequence of SEQ ID NO: 95; and a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 96, or a variant thereof having at least about 80% sequence identity to the amino acid sequence of SEQ ID NO: 96; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 97, or a variant thereof having at least about 80% sequence identity to the amino acid sequence of SEQ ID NO: 97; and a light chain variable domain comprising the amino acid sequence of SEQ ID NO: 143, or a variant thereof having at least about 80% sequence identity to the amino acid sequence of SEQ ID NO: 143. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 98, or a variant thereof having at least about 80% sequence identity to the amino acid sequence of SEQ ID NO: 98; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 96, or a variant thereof having at least about 80% sequence identity to the amino acid sequence of SEQ ID NO: 96; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 99, or a variant thereof having at least about 80% sequence identity to the amino acid sequence of SEQ ID NO: 99; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 94, or a variant thereof having at least about 80% sequence identity to the amino acid sequence of SEQ ID NO: 94; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 100, or a variant thereof having at least about 80% sequence identity to the amino acid sequence of SEQ ID NO: 100; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 101, or a variant thereof having at least about 80% sequence identity to the amino acid sequence of SEQ ID NO: 101; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 102, or a variant thereof having at least about 80% sequence identity to the amino acid sequence of SEQ ID NO: 102; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 103, or a variant thereof having at least about 80% sequence identity to the amino acid sequence of SEQ ID NO: 103; and a light chain variable domain comprising the amino acid sequence of SEQ ID NO: 144, or a variant thereof having at least about 80% sequence identity to the amino acid sequence of SEQ ID NO: 144. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 104, or a variant thereof having at least about 80% sequence identity to the amino acid sequence of SEQ ID NO: 104; and a light chain variable domain comprising the amino acid sequence of SEQ ID NO: 145, or a variant thereof having at least about 80% sequence identity to the amino acid sequence of SEQ ID NO: 145. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 105, or a variant thereof having at least about 80% sequence identity to the amino acid sequence of SEQ ID NO: 105; and a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 106, or a variant thereof having at least about 80% sequence identity to the amino acid sequence of SEQ ID NO: 106; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 107, or a variant thereof having at least about 80% sequence identity to the amino acid sequence of SEQ ID NO: 107; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 108, or a variant thereof having at least about 80% sequence identity to the amino acid sequence of SEQ ID NO: 108; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 109, or a variant thereof having at least about 80% sequence identity to the amino acid sequence of SEQ ID NO: 109; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 110, or a variant thereof having at least about 80% sequence identity to the amino acid sequence of SEQ ID NO: 110; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 111, or a variant thereof having at least about 80% sequence identity to the amino acid sequence of SEQ ID NO: 111; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 112, or a variant thereof having at least about 80% sequence identity to the amino acid sequence of SEQ ID NO: 112; and a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 113, or a variant thereof having at least about 80% sequence identity to the amino acid sequence of SEQ ID NO: 113; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 114, or a variant thereof having at least about 80% sequence identity to the amino acid sequence of SEQ ID NO: 114; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 108, or a variant thereof having at least about 80% sequence identity to the amino acid sequence of SEQ ID NO: 108; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 93, or a variant thereof having at least about 80% sequence identity to the amino acid sequence of SEQ ID NO: 93; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 115, or a variant thereof having at least about 80% sequence identity to the amino acid sequence of SEQ ID NO: 115; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 116, or a variant thereof having at least about 80% sequence identity to the amino acid sequence of SEQ ID NO: 116; and a light chain variable domain comprising the amino acid sequence of SEQ ID NO: 146, or a variant thereof having at least about 80% sequence identity to the amino acid sequence of SEQ ID NO: 146. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 117, or a variant thereof having at least about 80% sequence identity to the amino acid sequence of SEQ ID NO: 117; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 118, or a variant thereof having at least about 80% sequence identity to the amino acid sequence of SEQ ID NO: 118; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 104, or a variant thereof having at least about 80% sequence identity to the amino acid sequence of SEQ ID NO: 104; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 119, or a variant thereof having at least about 80% sequence identity to the amino acid sequence of SEQ ID NO: 119; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 120, or a variant thereof having at least about 80% sequence identity to the amino acid sequence of SEQ ID NO: 120; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 111, or a variant thereof having at least about 80% sequence identity to the amino acid sequence of SEQ ID NO: 111; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 121, or a variant thereof having at least about 80% sequence identity to the amino acid sequence of SEQ ID NO: 121; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 122, or a variant thereof having at least about 80% sequence identity to the amino acid sequence of SEQ ID NO: 122; and a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 123, or a variant thereof having at least about 80% sequence identity to the amino acid sequence of SEQ ID NO: 123; and a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 124, or a variant thereof having at least about 80% sequence identity to the amino acid sequence of SEQ ID NO: 124; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 125, or a variant thereof having at least about 80% sequence identity to the amino acid sequence of SEQ ID NO: 125; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 126, or a variant thereof having at least about 80% sequence identity to the amino acid sequence of SEQ ID NO: 126; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 112, or a variant thereof having at least about 80% sequence identity to the amino acid sequence of SEQ ID NO: 112; and a light chain variable domain comprising the amino acid sequence of SEQ ID NO: 147, or a variant thereof having at least about 80% sequence identity to the amino acid sequence of SEQ ID NO: 147. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 108, or a variant thereof having at least about 80% sequence identity to the amino acid sequence of SEQ ID NO: 108; and a light chain variable domain comprising the amino acid sequence of SEQ ID NO: 148, or a variant thereof having at least about 80% sequence identity to the amino acid sequence of SEQ ID NO: 148. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 127, or a variant thereof having at least about 80% sequence identity to the amino acid sequence of SEQ ID NO: 127; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 128, or a variant thereof having at least about 80% sequence identity to the amino acid sequence of SEQ ID NO: 128; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 129, or a variant thereof having at least about 80% sequence identity to the amino acid sequence of SEQ ID NO: 129; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 130, or a variant thereof having at least about 80% sequence identity to the amino acid sequence of SEQ ID NO: 130; and a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 131, or a variant thereof having at least about 80% sequence identity to the amino acid sequence of SEQ ID NO: 131; and a light chain variable domain comprising the amino acid sequence of SEQ ID NO: 149, or a variant thereof having at least about 80% sequence identity to the amino acid sequence of SEQ ID NO: 149. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 132, or a variant thereof having at least about 80% sequence identity to the amino acid sequence of SEQ ID NO: 132; and a light chain variable domain comprising the amino acid sequence of SEQ ID NO: 150, or a variant thereof having at least about 80% sequence identity to the amino acid sequence of SEQ ID NO: 150. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 133, or a variant thereof having at least about 80% sequence identity to the amino acid sequence of SEQ ID NO: 133; and a light chain variable domain comprising the amino acid sequence of SEQ ID NO: 151, or a variant thereof having at least about 80% sequence identity to the amino acid sequence of SEQ ID NO: 151. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 134, or a variant thereof having at least about 80% sequence identity to the amino acid sequence of SEQ ID NO: 134; and a light chain variable domain comprising the amino acid sequence of SEQ ID NO: 152, or a variant thereof having at least about 80% sequence identity to the amino acid sequence of SEQ ID NO: 152. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 135, or a variant thereof having at least about 80% sequence identity to the amino acid sequence of SEQ ID NO: 135; and a light chain variable domain comprising the amino acid sequence of SEQ ID NO: 153, or a variant thereof having at least about 80% sequence identity to the amino acid sequence of SEQ ID NO: 153. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 136, or a variant thereof having at least about 80% sequence identity to the amino acid sequence of SEQ ID NO: 136; and a light chain variable domain comprising the amino acid sequence of SEQ ID NO: 154, or a variant thereof having at least about 80% sequence identity to the amino acid sequence of SEQ ID NO: 154. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 137, or a variant thereof having at least about 80% sequence identity to the amino acid sequence of SEQ ID NO: 137; and a light chain variable domain comprising the amino acid sequence of SEQ ID NO: 155, or a variant thereof having at least about 80% sequence identity to the amino acid sequence of SEQ ID NO: 155. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG1 constant domains, wherein the anti-FXIIa antibody comprises a heavy chain variable domain comprising the amino acid sequence of SEQ ID NO: 138, or a variant thereof having at least about 80% sequence identity to the amino acid sequence of SEQ ID NO: 138; and a light chain variable domain comprising the amino acid sequence of SEQ ID NO: 156, or a variant thereof having at least about 80% sequence identity to the amino acid sequence of SEQ ID NO: 156. In some embodiments, the heavy chain constant region comprises or consists of the amino acid sequence of SEQ ID NO: 157. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 157 and the light chain constant region comprises or consists of the amino acid sequence of SEQ ID NO: 159. In some embodiments, the heavy chain constant region comprises or consists of the amino acid sequence of SEQ ID NO: 157 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 93, or a variant thereof having at least about 80% sequence identity to the amino acid sequence of SEQ ID NO: 93; a light chain variable domain comprising the amino acid sequence of SEQ ID NO: 139, or a variant thereof having at least about 80% sequence identity to the amino acid sequence of SEQ ID NO: 139. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 94, or a variant thereof having at least about 80% sequence identity to the amino acid sequence of SEQ ID NO: 94; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 95, or a variant thereof having at least about 80% sequence identity to the amino acid sequence of SEQ ID NO: 95; a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 96, or a variant thereof having at least about 80% sequence identity to the amino acid sequence of SEQ ID NO: 96; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 97, or a variant thereof having at least about 80% sequence identity to the amino acid sequence of SEQ ID NO: 97; a light chain variable domain comprising the amino acid sequence of SEQ ID NO: 143, or a variant thereof having at least about 80% sequence identity to the amino acid sequence of SEQ ID NO: 143. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 98, or a variant thereof having at least about 80% sequence identity to the amino acid sequence of SEQ ID NO: 98; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 96, or a variant thereof having at least about 80% sequence identity to the amino acid sequence of SEQ ID NO: 96; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 99, or a variant thereof having at least about 80% sequence identity to the amino acid sequence of SEQ ID NO: 99; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 94, or a variant thereof having at least about 80% sequence identity to the amino acid sequence of SEQ ID NO: 94; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 100, or a variant thereof having at least about 80% sequence identity to the amino acid sequence of SEQ ID NO: 100; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 101, or a variant thereof having at least about 80% sequence identity to the amino acid sequence of SEQ ID NO: 101; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 102, or a variant thereof having at least about 80% sequence identity to the amino acid sequence of SEQ ID NO: 102; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 103, or a variant thereof having at least about 80% sequence identity to the amino acid sequence of SEQ ID NO: 103; a light chain variable domain comprising the amino acid sequence of SEQ ID NO: 144, or a variant thereof having at least about 80% sequence identity to the amino acid sequence of SEQ ID NO: 144. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 104, or a variant thereof having at least about 80% sequence identity to the amino acid sequence of SEQ ID NO: 104; a light chain variable domain comprising the amino acid sequence of SEQ ID NO: 145, or a variant thereof having at least about 80% sequence identity to the amino acid sequence of SEQ ID NO: 145. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 105, or a variant thereof having at least about 80% sequence identity to the amino acid sequence of SEQ ID NO: 105; a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 106, or a variant thereof having at least about 80% sequence identity to the amino acid sequence of SEQ ID NO: 106; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 107, or a variant thereof having at least about 80% sequence identity to the amino acid sequence of SEQ ID NO: 107; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 108, or a variant thereof having at least about 80% sequence identity to the amino acid sequence of SEQ ID NO: 108; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 109, or a variant thereof having at least about 80% sequence identity to the amino acid sequence of SEQ ID NO: 109; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 110, or a variant thereof having at least about 80% sequence identity to the amino acid sequence of SEQ ID NO: 110; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 111, or a variant thereof having at least about 80% sequence identity to the amino acid sequence of SEQ ID NO: 111; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 112, or a variant thereof having at least about 80% sequence identity to the amino acid sequence of SEQ ID NO: 112; a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 113, or a variant thereof having at least about 80% sequence identity to the amino acid sequence of SEQ ID NO: 113; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 114, or a variant thereof having at least about 80% sequence identity to the amino acid sequence of SEQ ID NO: 114; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 108, or a variant thereof having at least about 80% sequence identity to the amino acid sequence of SEQ ID NO: 108; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 93, or a variant thereof having at least about 80% sequence identity to the amino acid sequence of SEQ ID NO: 93; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 115, or a variant thereof having at least about 80% sequence identity to the amino acid sequence of SEQ ID NO: 115; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 116, or a variant thereof having at least about 80% sequence identity to the amino acid sequence of SEQ ID NO: 116; a light chain variable domain comprising the amino acid sequence of SEQ ID NO: 146, or a variant thereof having at least about 80% sequence identity to the amino acid sequence of SEQ ID NO: 146. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 117, or a variant thereof having at least about 80% sequence identity to the amino acid sequence of SEQ ID NO: 117; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 118, or a variant thereof having at least about 80% sequence identity to the amino acid sequence of SEQ ID NO: 118; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 104, or a variant thereof having at least about 80% sequence identity to the amino acid sequence of SEQ ID NO: 104; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 119, or a variant thereof having at least about 80% sequence identity to the amino acid sequence of SEQ ID NO: 119; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 120, or a variant thereof having at least about 80% sequence identity to the amino acid sequence of SEQ ID NO: 120; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 111, or a variant thereof having at least about 80% sequence identity to the amino acid sequence of SEQ ID NO: 111; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 121, or a variant thereof having at least about 80% sequence identity to the amino acid sequence of SEQ ID NO: 121; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 122, or a variant thereof having at least about 80% sequence identity to the amino acid sequence of SEQ ID NO: 122; a light chain variable domain comprising the amino acid sequence of SEQ ID NO: 141, or a variant thereof having at least about 80% sequence identity to the amino acid sequence of SEQ ID NO: 141. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 123, or a variant thereof having at least about 80% sequence identity to the amino acid sequence of SEQ ID NO: 123; a light chain variable domain comprising the amino acid sequence of SEQ ID NO: 142, or a variant thereof having at least about 80% sequence identity to the amino acid sequence of SEQ ID NO: 142. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 124, or a variant thereof having at least about 80% sequence identity to the amino acid sequence of SEQ ID NO: 124; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 125, or a variant thereof having at least about 80% sequence identity to the amino acid sequence of SEQ ID NO: 125; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 126, or a variant thereof having at least about 80% sequence identity to the amino acid sequence of SEQ ID NO: 126; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 112, or a variant thereof having at least about 80% sequence identity to the amino acid sequence of SEQ ID NO: 112; a light chain variable domain comprising the amino acid sequence of SEQ ID NO: 147, or a variant thereof having at least about 80% sequence identity to the amino acid sequence of SEQ ID NO: 147. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 108, or a variant thereof having at least about 80% sequence identity to the amino acid sequence of SEQ ID NO: 108; a light chain variable domain comprising the amino acid sequence of SEQ ID NO: 148, or a variant thereof having at least about 80% sequence identity to the amino acid sequence of SEQ ID NO: 148. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 127, or a variant thereof having at least about 80% sequence identity to the amino acid sequence of SEQ ID NO: 127; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 128, or a variant thereof having at least about 80% sequence identity to the amino acid sequence of SEQ ID NO: 128; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 129, or a variant thereof having at least about 80% sequence identity to the amino acid sequence of SEQ ID NO: 129; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 130, or a variant thereof having at least about 80% sequence identity to the amino acid sequence of SEQ ID NO: 130; a light chain variable domain comprising the amino acid sequence of SEQ ID NO: 140, or a variant thereof having at least about 80% sequence identity to the amino acid sequence of SEQ ID NO: 140. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 131, or a variant thereof having at least about 80% sequence identity to the amino acid sequence of SEQ ID NO: 131; a light chain variable domain comprising the amino acid sequence of SEQ ID NO: 149, or a variant thereof having at least about 80% sequence identity to the amino acid sequence of SEQ ID NO: 149. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 132, or a variant thereof having at least about 80% sequence identity to the amino acid sequence of SEQ ID NO: 132; a light chain variable domain comprising the amino acid sequence of SEQ ID NO: 150, or a variant thereof having at least about 80% sequence identity to the amino acid sequence of SEQ ID NO: 150. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 133, or a variant thereof having at least about 80% sequence identity to the amino acid sequence of SEQ ID NO: 133; a light chain variable domain comprising the amino acid sequence of SEQ ID NO: 151, or a variant thereof having at least about 80% sequence identity to the amino acid sequence of SEQ ID NO: 151. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 134, or a variant thereof having at least about 80% sequence identity to the amino acid sequence of SEQ ID NO: 134; a light chain variable domain comprising the amino acid sequence of SEQ ID NO: 152, or a variant thereof having at least about 80% sequence identity to the amino acid sequence of SEQ ID NO: 152. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 135, or a variant thereof having at least about 80% sequence identity to the amino acid sequence of SEQ ID NO: 135; a light chain variable domain comprising the amino acid sequence of SEQ ID NO: 153, or a variant thereof having at least about 80% sequence identity to the amino acid sequence of SEQ ID NO: 153. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 136, or a variant thereof having at least about 80% sequence identity to the amino acid sequence of SEQ ID NO: 136; a light chain variable domain comprising the amino acid sequence of SEQ ID NO: 154, or a variant thereof having at least about 80% sequence identity to the amino acid sequence of SEQ ID NO: 154. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 137, or a variant thereof having at least about 80% sequence identity to the amino acid sequence of SEQ ID NO: 137; a light chain variable domain comprising the amino acid sequence of SEQ ID NO: 155, or a variant thereof having at least about 80% sequence identity to the amino acid sequence of SEQ ID NO: 155. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

In some embodiments, there is provided a full-length anti-FXIIa antibody comprising IgG4 constant domains, wherein the anti-FXIIa antibody comprises a heavy chain variable domain comprising the amino acid sequence of SEQ ID NO: 138, or a variant thereof having at least about 80% sequence identity to the amino acid sequence of SEQ ID NO: 138; a light chain variable domain comprising the amino acid sequence of SEQ ID NO: 156, or a variant thereof having at least about 80% sequence identity to the amino acid sequence of SEQ ID NO: 156. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160. In some embodiments, the heavy chain constant region comprises or consists of the amino acid sequence of SEQ ID NO: 158 and the light chain constant region comprises or consists of the amino acid sequence of SEQ ID NO: 159. In some embodiments, the heavy chain constant region comprises or consists of the amino acid sequence of SEQ ID NO: 158 and the light chain constant region comprises or consists of the amino acid sequence of SEQ ID NO: 160.

### Binding affinity

Binding affinity can be indicated by Kd, Koff, Kon, or Ka. The term "Koff", as used herein, is intended to refer to the off-rate constant for dissociation of an antibody from the antibody /antigen complex, as determined from a kinetic selection set up. The term "Kon", as used herein, is intended to refer to the on-rate constant for association of an antibody to the antigen to form the antibody/antigen complex. The term dissociation constant "Kd", as used herein, refers to the dissociation constant of a particular antibody-antigen interaction, and describes the concentration of antigen required to occupy one half of all of the antibody-binding domains present in a solution of antibody molecules at equilibrium, and is equal to Koff/Kon. The measurement of Kd presupposes that all binding agents are in solution. In the case where
the antibody is tethered to a cell wall, *e.g.,* in a yeast expression system, the corresponding equilibrium rate constant is expressed as EC50, which gives a good approximation of Kd. The affinity constant, Ka, is the inverse of the dissociation constant, Kd.

The dissociation constant (Kd) is used as an indicator showing affinity of antibody moieties to antigens. For example, easy analysis is possible by the Scatchard method using antibodies marked with a variety of marker agents, as well as by using Biacore (made by Amersham Biosciences), analysis of biomolecular interactions by surface plasmon resonance, according to the user's manual and attached kit. The Kd value that can be derived using these methods is expressed in units of M. An antibody that specifically binds to a target may have a Kd of, for example, ≤ 10⁻⁷ M, ≤ 10⁻⁸ M, ≤ 10⁻⁹ M, ≤ 10⁻¹⁰ M, ≤ 10⁻¹¹ M, ≤ 10⁻¹² M, or ≤ 10⁻¹³ M.

Binding specificity of the antibody can be determined experimentally by methods known in the art. Such methods comprise, but are not limited to, Western blots, ELISA-, RIA-, ECL-, IRMA-, EIA-, BIAcore-tests and peptide scans.

In some embodiments, the anti-FXIIa antibody specifically binds to a target FXIIa with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the anti-FXIIa antibody and FXIIa, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻³ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the anti-FXIIa antibody and a FXIIa is about 10⁻⁷ M to about 10⁻¹³ M.

In some embodiments, the Kd of the binding between the anti-FXIIa antibody and a non-target is more than the Kd of the binding between the anti-FXIIa antibody and the target, and is herein referred to in some embodiments as the binding affinity, of the anti-FXIIa antibody to the target (e.g., FXIIa) is higher than that to a non-target. In some embodiments, the non-target is an antigen that is not FXIIa. In some embodiments, the Kd of the binding between the anti-FXIIa antibody (against FXIIa) and a non-FXIIa target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the anti-FXIIa antibody and a target FXIIa.

In some embodiments, the anti-FXIIa antibody binds to a non-target with a Kd of about 10⁻¹ M to about 10⁻⁶ M (such as about 10⁻¹ M to about 10⁻⁶ M, about 10⁻¹ M to about 10⁻⁵ M, or about 10⁻² M to about 10⁻⁴ M). In some embodiments, the non-target is an antigen that is not FXIIa. Thus in some embodiments, the Kd of the binding between the anti-FXIIa antibody and a non-FXIIa target is about 10⁻¹ M to about 10⁻⁶ M, about 1×10⁻¹ M to about 5×10⁻⁶ M, about 10⁻¹ M to about 10⁻⁵ M, about 1×10⁻¹ M to about 5×10⁻⁵ M, about 10⁻¹ M to about 10⁻⁴ M, about 1×10⁻¹ M to about 5×10⁻⁴ M, about 10⁻¹ M to about 10⁻³ M, about 1×10⁻¹ M to about 5×10⁻³ M, about 10⁻¹ M to about 10⁻² M, about 10⁻² M to about 10⁻⁶ M, about 1 x 10⁻² M to about 5×10⁻⁶ M, about 10⁻² M to about 10⁻⁵ M, about 1×10⁻² M to about 5×10⁻⁵ M, about 10⁻² M to about 10⁻⁴ M, about 1×10⁻² M to about 5×10⁻⁴ M, about 10⁻² M to about 10⁻³ M, about 10⁻³ M to about 10⁻⁶ M, about 1×10⁻³ M to about 5×10⁻⁶ M, about 10⁻³ M to about 10⁻⁵ M, about 1×10⁻³ M to about 5×10⁻⁵ M, about 10⁻³ M to about 10⁻⁴ M, about 10⁻⁴ M to about 10⁻⁶ M, about 1×10⁻⁴ M to about 5×10⁻⁶ M, about 10⁻⁴ M to about 10⁻⁵ M, or about 10⁻⁵ M to about 10⁻⁶ M_{.}

In some embodiments, when referring to that the anti-FXIIa antibody specifically recognizes a target FXIIa at a high binding affinity, and binds to a non-target at a low binding affinity, the anti-FXIIa antibody will bind to the target FXIIa with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M), and will bind to the non-target with a Kd of about 10⁻¹ M to about 10⁻⁶ M (such as about 10⁻¹ M to about 10⁻⁶ M, about 10⁻¹ M to about 10⁻⁵ M, or about 10⁻³ M to about 10⁻⁴ M).

In some embodiments, when referring to that the anti-FXIIa antibody specifically recognizes FXIIa, the binding affinity of the anti-FXIIa antibody is compared to that of a control anti-FXIIa antibody (such as CSL312). In some embodiments, the Kd of the binding between the control anti-FXIIa antibody and FXIIa can be at least about 2 times, such as about 2 times, about 3 times, about 4 times, about 5 times, about 6 times, about 7 times, about 8 times, about 9 times, about 10 times, about 10-100 times, about 100-1000 times, about 10³-10⁴ times of the Kd of the binding between the anti-FXIIa antibody described herein and FXIIa.

### Nucleic Acids

Nucleic acid molecules encoding the anti-FXIIa antibodies are also contemplated. In some embodiments, there is provided a nucleic acid (or a set of nucleic acids) encoding a full-length anti-FXIIa antibody, including any of the full-length anti-FXIIa antibodies described herein. In some embodiments, the nucleic acid (or a set of nucleic acids) encoding the anti-FXIIa antibody described herein may further comprises a nucleic acid sequence encoding a peptide tag (such as protein purification tag, e.g., His-tag, HA tag).

Also contemplated here are isolated host cells comprising an anti-FXIIa antibody, an isolated nucleic acid encoding the polypeptide components of the anti-FXIIa antibody, or a vector comprising a nucleic acid encoding the polypeptide components of the anti-FXIIa antibody described herein.

The present application also includes variants to these nucleic acid sequences. For example, the variants include nucleotide sequences that hybridize to the nucleic acid sequences encoding the anti-FXIIa antibodies of the present application under at least moderately stringent hybridization conditions.

The present application also provides vectors in which a nucleic acid of the present application is inserted.

In brief summary, the expression of an anti-FXIIa antibody (e.g., full-length anti-FXIIa antibody) by a natural or synthetic nucleic acid encoding the anti-FXIIa antibody can be achieved by inserting the nucleic acid into an appropriate expression vector, such that the nucleic acid is operably linked to 5' and 3' regulatory elements, including for example a promoter (e.g., a lymphocyte-specific promoter) and a 3' untranslated region (UTR). The vectors can be suitable for replication and integration in eukaryotic host cells. Typical cloning and expression vectors contain transcription and translation terminators, initiation sequences, and promoters useful for regulation of the expression of the desired nucleic acid sequences.

The nucleic acids of the present application may also be used for nucleic acid immunization and gene therapy, using standard gene delivery protocols. Methods for gene delivery are known in the art. See, *e.g.,* U.S. Pat. Nos. 5,399,346, 5,580,859, 5,589,466, incorporated by reference herein in their entireties. In some embodiments, the application provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Green and Sambrook (2013, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers (*see, e.g.,* WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In some embodiments, lentivirus vectors are used. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Additional promoter elements, *e.g.*, enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence to which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In some embodiments, the expression of the anti-FXIIa antibody is inducible. In some embodiments, a nucleic acid sequence encoding the anti-FXIIa antibody is operably linked to an inducible promoter, including any inducible promoter described herein.

### Inducible promoters

The use of an inducible promoter provides a molecular switch capable of turning on expression of the polynucleotide sequence to which it is operatively linked when such expression is desired, or turning off the expression when expression is not desired. Exemplary inducible promoter systems for use in eukaryotic cells include, but are not limited to, hormone-regulated elements (*e.g., see* Mader, S. and White, J. H. (1993) Proc. Natl. Acad. Sci. USA 90:5603-5607), synthetic ligand-regulated elements (*see, e.g.,* Spencer, D. M. et al. (1993) Science 262: 1019-1024) and ionizing radiation-regulated elements (*e.g.,* see Manome, Y. et al. (1993) Biochemistry 32: 10607-10613; Datta, R. et al. (1992) Proc. Natl. Acad. Sci. USA 89: 1014- 10153). Further exemplary inducible promoter systems for use in in vitro or in vivo mammalian systems are reviewed in Gingrich et al. (1998) Annual Rev. Neurosci 21:377-405. In some embodiments, the inducible promoter system for use to express the anti-FXIIa antibody is the Tet system. In some embodiments, the inducible promoter system for use to express the anti-FXIIa antibody is the lac repressor system from *E. coli.*

An exemplary inducible promoter system for use in the present application is the Tet system. Such systems are based on the Tet system described by Gossen *et al.* (1993). In an exemplary embodiment, a polynucleotide of interest is under the control of a promoter that comprises one or more Tet operator (TetO) sites. In the inactive state, Tet repressor (TetR) will bind to the TetO sites and repress transcription from the promoter. In the active state, *e.g.*, in the presence of an inducing agent such as tetracycline (Tc), anhydrotetracycline, doxycycline (Dox), or an active analog thereof, the inducing agent causes release of TetR from TetO, thereby allowing transcription to take place. Doxycycline is a member of the tetracycline family of antibiotics having the chemical name of 1-dimethylamino-2,4a,5,7,12-pentahydroxy-11-methyl-4,6-dioxo-1,4a,11,11a,12,12a-hexahydrotetracene-3-carboxamide.

In one embodiment, a TetR is codon-optimized for expression in mammalian cells, *e.g.*, murine or human cells. Most amino acids are encoded by more than one codon due to the degeneracy of the genetic code, allowing for substantial variations in the nucleotide sequence of a given nucleic acid without any alteration in the amino acid sequence encoded by the nucleic acid. However, many organisms display differences in codon usage, also known as "codon bias" (*i.e.,* bias for use of a particular codon(s) for a given amino acid). Codon bias often correlates with the presence of a predominant species of tRNA for a particular codon, which in turn increases efficiency of mRNA translation. Accordingly, a coding sequence derived from a particular organism (*e.g.,* a prokaryote) may be tailored for improved expression in a different organism *(e.g.,* a eukaryote) through codon optimization.

Other specific variations of the Tet system include the following "Tet-Off" and "Tet-On" systems. In the Tet-Off system, transcription is inactive in the presence of Tc or Dox. In that system, a tetracycline-controlled transactivator protein (tTA), which is composed of TetR fused to the strong transactivating domain of VP16 from Herpes simplex virus, regulates expression of a target nucleic acid that is under transcriptional control of a tetracycline-responsive promoter element (TRE). The TRE is made up of TetO sequence concatamers fused to a promoter (commonly the minimal promoter sequence derived from the human cytomegalovirus (hCMV) immediate-early promoter). In the absence of Tc or Dox, tTA binds to the TRE and activates transcription of the target gene. In the presence of Tc or Dox, tTA cannot bind to the TRE, and expression from the target gene remains inactive.

Conversely, in the Tet-On system, transcription is active in the presence of Tc or Dox. The Tet-On system is based on a reverse tetracycline-controlled transactivator, rtTA. Like tTA, rtTA is a fusion protein comprised of the TetR repressor and the VP16 transactivation domain. However, a four amino acid change in the TetR DNA binding moiety alters rtTA's binding characteristics such that it can only recognize the tetO sequences in the TRE of the target transgene in the presence of Dox. Thus, in the Tet-On system, transcription of the TRE-regulated target gene is stimulated by rtTA only in the presence of Dox.

Another inducible promoter system is the lac repressor system from *E. coli* (*See* Brown et al., Cell 49:603-612 (1987)). The lac repressor system functions by regulating transcription of a polynucleotide of interest operably linked to a promoter comprising the lac operator (lacO). The lac repressor (lacR) binds to LacO, thus preventing transcription of the polynucleotide of interest. Expression of the polynucleotide of interest is induced by a suitable inducing agent, *e.g.*, isopropyl-β-D-thiogalactopyranoside (IPTG).

In order to assess the expression of a polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, *e.g.*, enzymatic activity. Expression of the reporter gene is assayed at a suitable time after the DNA has been introduced into the recipient cells. Suitable reporter genes may include genes encoding luciferase, β-galactosidase, chloramphenicol acetyl transferase, secreted alkaline phosphatase, or the green fluorescent protein gene (*e.g.,* Ui-Tel et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some embodiments, there is provided nucleic acid encoding a full-length anti-FXIIa antibody according to any of the full-length anti-FXIIa antibodies described herein. In some embodiments, the nucleic acid comprises one or more nucleic acid sequences encoding the heavy and light chains of the full-length anti-FXIIa antibody. In some embodiments, each of the one or more nucleic acid sequences are contained in separate vectors. In some embodiments, at least some of the nucleic acid sequences are contained in the same vector. In some embodiments, all of the nucleic acid sequences are contained in the same vector. Vectors may be selected, for example, from the group consisting of mammalian expression vectors and viral vectors (such as those derived from retroviruses, adenoviruses, adeno-associated viruses, herpes viruses, and lentiviruses).

Methods of introducing and expressing genes into a cell are known in the art. In the context of an expression vector, the vector can be readily introduced into a host cell, *e.g.*, mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Green and Sambrook (2013, Molecular Cloning: A Laboratory Manual, Cold Spring Harbor Laboratory, New York). In some embodiments, the introduction of a polynucleotide into a host cell is carried out by calcium phosphate transfection.

Biological methods for introducing a polynucleotide of interest into a host cell include the use of DNA and RNA vectors. Viral vectors, and especially retroviral vectors, have become the most widely used method of inserting genes into mammalian, *e.g.*, human cells. Other viral vectors can be derived from lentivirus, poxviruses, herpes simplex virus 1, adenoviruses and adeno-associated viruses, and the like. *See,* for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (*e.g.,* an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Regardless of the method used to introduce exogenous nucleic acids into a host cell or otherwise expose a cell to the inhibitor of the present application, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) or by assays described herein to identify agents falling within the scope of the application.

### Preparation of anti-FXIIa antibodies

In some embodiments, the anti-FXIIa antibody is a monoclonal antibody or derived from a monoclonal antibody. In some embodiments, the anti-FXIIa antibody comprises V_{H} and V_{L} domains, or variants thereof, from the monoclonal antibody. In some embodiments, the anti-FXIIa antibody further comprises C_{H}1 and C_{L} domains, or variants thereof, from the monoclonal antibody. Monoclonal antibodies can be prepared, *e.g.*, using known methods in the art, including hybridoma methods, phage display methods, or using recombinant DNA methods. Additionally, exemplary phage display methods are described herein and in the Examples below.

In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized *in vitro.* The immunizing agent can include a polypeptide or a fusion protein of the protein of interest. Generally, peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

In some embodiments, the immortalized cell lines fuse efficiently, support stable high-level expression of antibody by the selected antibody-producing cells, and are sensitive to a medium such as HAT medium. In some embodiments, the immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies.

The culture medium in which the hybridoma cells are cultured can then be assayed for the presence of monoclonal antibodies directed against the polypeptide. The binding specificity of monoclonal antibodies produced by the hybridoma cells can be determined by immunoprecipitation or by an *in vitro* binding assay, such as radioimmunoassay (RIA) or enzyme-linked immunosorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones can be sub-cloned by limiting dilution procedures and grown by standard methods. Goding, *supra.* Suitable culture media for this purpose include, for example, Dulbecco's Modified Eagle's Medium and RPMI-1640 medium. Alternatively, the hybridoma cells can be grown *in vivo* as ascites in a mammal.

The monoclonal antibodies secreted by the sub-clones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

In some embodiments, according to any of the anti-FXIIa antibodies described herein, the anti-FXIIa antibody comprises sequences from a clone selected from an antibody library (such as a phage library presenting scFv or Fab fragments). The clone may be identified by screening combinatorial libraries for antibody fragments with the desired activity or activities. For example, a variety of methods are known in the art for generating phage display libraries and screening such libraries for antibodies possessing the desired binding characteristics. Such methods are reviewed, *e.g.,* in Hoogenboom et al., Methods in Molecular Biology 178:1-37 (O'Brien et al., ed., Human Press, Totowa, N.J., 2001) and further described, *e.g.,* in McCafferty et al., Nature 348:552-554; Clackson et al., Nature 352: 624-628 (1991); Marks et al., J. Mol. Biol. 222: 581-597 (1992); Marks and Bradbury, Methods in Molecular Biology 248:161-175 (Lo, ed., Human Press, Totowa, N.J., 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as scFv fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: U.S. Pat. No. 5,750,373, and US Patent Publication Nos. 2005/0079574, 2005/0119455, 2005/0266000, 2007/0117126, 2007/0160598, 2007/0237764, 2007/0292936, and 2009/0002360.

The anti-FXIIa antibodies can be prepared using phage display to screen libraries for anti-FXIIa antibody moieties specific to the target FXIIa. The library can be a human scFv phage display library having a diversity of at least 1 × 10⁹ (such as at least about any of 1 × 10⁹, 2.5 × 10⁹, 5 × 10⁹, 7.5 × 10⁹, 1 × 10¹⁰, 2.5 × 10¹⁰, 5 × 10¹⁰, 7.5 × 10¹⁰, or 1 × 10¹¹) unique human antibody fragments. In some embodiments, the library is a naive human library constructed from DNA extracted from human PMBCs and spleens from healthy donors, encompassing all human heavy and light chain subfamilies. In some embodiments, the library is a naive human library constructed from DNA extracted from PBMCs isolated from patients with various diseases, such as patients with autoimmune diseases, cancer patients, and patients with infectious diseases. In some embodiments, the library is a semi-synthetic human library, wherein heavy chain CDR3 is completely randomized, with all amino acids (with the exception of cysteine) equally likely to be present at any given position (*see, e.g.,* Hoet, R.M. et al., Nat. Biotechnol. 23(3):344-348, 2005). In some embodiments, the heavy chain CDR3 of the semi-synthetic human library has a length from about 5 to about 24 (such as about any of 5, 6, 7, 8, 9, 10, 11, 12, 13, 14, 15, 16, 17, 18, 19, 20, 21, 22, 23, or 24) amino acids. In some embodiments, the library is a fully-synthetic phage display library. In some embodiments, the library is a non-human phage display library.

Phage clones that bind to the target FXIIa with high affinity can be selected by iterative binding of phage to the target FXIIa, which is bound to a solid support (such as, for example, beads for solution panning or mammalian cells for cell panning), followed by removal of non-bound phage and by elution of specifically bound phage. The bound phage clones are then eluted and used to infect an appropriate host cell, such as *E*. *coli* XL1-Blue, for expression and purification. The panning can be performed for multiple (such as about any of 2, 3, 4, 5, 6 or more) rounds with solution panning, cell panning, or a combination of both, to enrich for phage clones binding specifically to the target FXIIa. Enriched phage clones can be tested for specific binding to the target FXIIa by any methods known in the art, including for example ELISA and FACS.

An alternative method for screening antibody libraries is to display the protein on the surface of yeast cells. Wittrup et al. (US Patent Nos. 6,699,658 and 6,696,25 1) have developed a method for a yeast cell display library. In this yeast display system, a component involves the yeast agglutinin protein (Aga1), which is anchored to the yeast cell wall. Another component involves a second subunit of the agglutinin protein Aga2, which can display on the surface yeast cells through disulfide bonds to Aga1 protein. The protein Aga1 is expressed from a yeast chromosome after the Aga1 gene integration. A library of single chain variable fragments (scFv) is fused genetically to Aga2 sequence in the yeast display plasmid, which, after transformation, is maintained in yeast episomally with a nutritional marker. Both of the Aga1 and Aga2 proteins were expressed under the control of the galactose-inducible promoter.

Human antibody V gene repertoire (V_{H} and V_{K} fragments) are obtained by PCR method using a pool of degenerate primers (Sblattero, D. & Bradbury, A. Immunotechnology 3, 271-278 1998). The PCR templates are from the commercially available RNAs or cDNAs, including PBMC, spleen, lymph nodes, bone marrow and tonsils. Separate V_{H} and V_{K} PCR libraries were combined, then assembled together in the scFv format by overlap extension PCR ( Sheets, M.D. et al., Proc. Natl. Acad. Sci. USA 95, 6157-6162 1998.). To construct the yeast scFv display library, the resultant scFv PCR products are cloned into the yeast display plasmid in the yeasts by homologous recombination. (Chao, G, et al., Nat Protoc. 2006;1(2):755-68. Miller KD, et al., Current Protocols in Cytometry 4.7.1-4.7.30, 2008).

The anti-FXIIa antibodies can be discovered using mammalian cell display systems in which antibody moieties are displayed on the cell surface and those specific to the target FXIIa are isolated by the antigen-guided screening method, as described in U.S. patent No. 7,732,195B2. A Chinese hamster ovary (CHO) cell library representing a large set of human IgG antibody genes can be established and used to discover the clones expressing high-affinity antibody genes. Another display system has been developed to enable simultaneous high-level cell surface display and secretion of the same protein through alternate splicing, where the displayed protein phenotype remains linked to genotype, allowing soluble secreted antibody to be simultaneously characterized in biophysical and cell-based functional assays. This approach overcomes many limitations of previous mammalian cell display, enabling direct selection and maturation of antibodies in the form of full-length, glycosylated IgGs (Peter M. Bowers, et al., Methods 2014,65:44-56). Transient expression systems are suitable for a single round of antigen selection before recovery of the antibody genes and therefore most useful for the selection of antibodies from smaller libraries. Stable episomal vectors offer an attractive alternative. Episomal vectors can be transfected at high efficiency and stably maintained at low copy number, permitting multiple rounds of panning and the resolution of more complex antibody libraries.

The IgG library is based on germline sequence V-gene segments joined to rearranged (D)J regions isolated from a panel of human donors. RNA collected from 2000 human blood samples was reverse-transcribed into cDNA, and the V_{H} and V_{K} fragments were amplified using V_{H}- and V_{g}-specific primers and purified by gel extraction. IgG libraries were generated by sub-cloning the V_{H} and V_{K} fragments into the display vectors containing IgG1 or K constant regions respectively and then electroporating into or transducing 293T cells. To generate the scFv antibody display library, scFvs were generated by linking V_{H} and V_{K}, and then sub-cloned into the display vector, which were then electroporated into or transduce 293T cells. As we known, the IgG library is based on germline sequence V-gene segments joined to rearranged (D)J regions isolated from a panel of donors, the donor can be a mouse, rat, rabbit, or monkey.

Monoclonal antibodies can also be made by recombinant DNA methods, such as those described in U.S. Patent No. 4,816,567. DNA encoding the monoclonal antibodies of the application can be readily isolated and sequenced using conventional procedures (*e.g.*, by using oligonucleotide probes that are capable of binding specifically to genes encoding the heavy and light chains of murine antibodies). Hybridoma cells as described above or FXIIa-specific phage clones of the application can serve as a source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy- and light-chain constant domains and/or framework regions in place of the homologous non-human sequences (U.S. Patent No. 4,816,567; Morrison *et al., supra*) or by covalently joining to the immunoglobulin coding sequence all or part of the coding sequence for a non-immunoglobulin polypeptide. Such a non-immunoglobulin polypeptide can be substituted for the constant domains of an antibody of the application, or can be substituted for the variable domains of one antigen-combining site of an antibody of the application to create a chimeric bivalent antibody.

The antibodies can be monovalent antibodies. Methods for preparing monovalent antibodies are known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using any method known in the art.

Antibody variable domains with the desired binding specificities (antibody-antigen combining sites) can be fused to immunoglobulin constant-domain sequences. The fusion preferably is with an immunoglobulin heavy-chain constant domain, comprising at least part of the hinge, CH2, and CH3 regions. In some embodiments, the first heavy-chain constant region (CH1) containing the site necessary for light-chain binding is present in at least one of the fusions. DNAs encoding the immunoglobulin heavy-chain fusions and, if desired, the immunoglobulin light chain, are inserted into separate expression vectors, and are co-transfected into a suitable host organism.

### Human and Humanized Antibodies

The anti-FXIIa antibodies (*e.g.*, full-length anti-FXIIa antibodies) can be humanized antibodies or human antibodies. Humanized forms of non-human (*e.g.*, murine) antibody moieties are chimeric immunoglobulins, immunoglobulin chains, or fragments thereof (such as Fv, Fab, Fab', F(ab')₂, scFv, or other antigen-binding subsequences of antibodies) that typically contain minimal sequence derived from non-human immunoglobulin. Humanized antibody moieties include human immunoglobulins, immunoglobulin chains, or fragments thereof (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibody moieties can also comprise residues that are found neither in the recipient antibody nor in the imported CDR or framework sequences. In general, the humanized antibody can comprise substantially at least one, and typically two, variable domains, in which all or substantially all of the CDR regions correspond to those of a non-human immunoglobulin, and all or substantially all of the FR regions are those of a human immunoglobulin consensus sequence.

Generally, a humanized antibody has one or more amino acid residues introduced into it from a source that is non-human. These non-human amino acid residues are often referred to as "import" residues, which are typically taken from an "import" variable domain. According to some embodiments, humanization can be essentially performed following the method of Winter and co-workers (Jones et al., Nature, 321: 522-525 (1986); Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibody moieties are antibody moieties (U.S. Patent No. 4,816,567), wherein substantially less than an intact human variable domain has been substituted by the corresponding sequence from a non-human species. In practice, humanized antibody moieties are typically human antibody moieties in which some CDR residues and possibly some FR residues are substituted by residues from analogous sites in rodent antibodies.

As an alternative to humanization, human antibody moieties can be generated. For example, it is now possible to produce transgenic animals (*e.g.*, mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., PNAS USA, 90:2551 (1993); Jakobovits et al., Nature, 362:255-258 (1993); Bruggemann et al., Year in Immunol., 7:33 (1993); U.S. Patent Nos. 5,545,806, 5,569,825, 5,591,669; 5,545,807; and WO 97/17852. Alternatively, human antibodies can be made by introducing human immunoglobulin loci into transgenic animals, *e.g.*, mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

Human antibodies may also be generated by *in vitro* activated B cells (see U.S. Patents 5,567,610 and 5,229,275) or by using various techniques known in the art, including phage display libraries. Hoogenboom and Winter, J. Mol. Biol., 227:381 (1991); Marks et al., J. Mol. Biol., 222:581 (1991). The techniques of Cole *et al.* and Boerner *et al.* are also available for the preparation of human monoclonal antibodies. Cole et al., Monoclonal Antibodies and Cancer Therapy, Alan R. Liss, p.77 (1985) and Boerner et al., J. Immunol., 147(1): 86-95 (1991).

### Anti-FXIIa antibody variants

In some embodiments, amino acid sequences of the anti-FXIIa antibody variants (e.g., full-length anti-FXIIa antibody) provided herein are contemplated. For example, it may be desirable to improve the binding affinity and/or other biological properties of the antibody. Amino acid sequences of an antibody variant may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g.*, antigen-binding.

In some embodiments, anti-FXIIa antibody variants having one or more amino acid substitutions are provided. Sites of interest for substitutional mutagenesis include the HVRs and FRs. Amino acid substitutions may be introduced into an antibody of interest and the products screened for a desired activity, *e.g*., improved bioactivity, retained/improved antigen binding, decreased immunogenicity, or improved ADCC or CDC.

Conservative substitutions are shown in Table 4 below.

**TABLE 4: CONSERVATIVE SUBSTITUTIONS**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties:
a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

An exemplary substitutional variant is an affinity matured antibody, which may be conveniently generated, *e.g.*, using phage display-based affinity maturation techniques. Briefly, one or more CDR residues are mutated and the variant antibody moieties displayed on phage and screened for a particular biological activity (*e.g.*, bioactivity based on biological activity of FXIIa enzyme active substrate S2302 in cleavage inhibition experiment or binding affinity). Alterations *(e.g.,* substitutions) may be made in HVRs, *e.g.,* to improve bioactivity based on biological activity of FXIIa enzyme active substrate S2302 in cleavage inhibition experiment or binding affinity. Such alterations may be made in HVR "hotspots", *e.g.*, residues encoded by codons that undergo mutation at high frequency during the somatic maturation process (see, *e.g.*, Chowdhury, Methods Mol. Biol. 207:179-196 (2008)), and/or specificity determining residues (SDRs), with the resulting variant V_{H} and V_{L} being tested for binding affinity. Affinity maturation by constructing and reselecting from secondary libraries has been described, *e.g.,* in Hoogenboom et al., in Methods in Molecular Biology 178:1-37 (O'Brien et al., ed., Human Press, Totowa, NJ, (2001)).

In some embodiments of affinity maturation, diversity is introduced into the variable genes chosen for maturation by any of a variety of methods (*e.g.*, error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g.*, 4-6 residues at a time) are randomized. HVR residues involved in antigen binding may be specifically identified, *e.g.*, using alanine scanning mutagenesis or modeling. CDR-H3 and CDR-L3 in particular are often targeted.

In some embodiments, substitutions, insertions, or deletions may occur within one or more HVRs so long as such alterations do not substantially reduce the ability of the antibody to bind antigen. For example, conservative alterations (*e.g.*, conservative substitutions as provided herein) that do not substantially reduce binding affinity may be made in HVRs. Such alterations may be outside of HVR "hotspots" or SDRs. In some embodiments of the variant V_{H} and V_{L} sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e.g*., charged residues such as Arg, Asp, His, Lys, and Glu) are identified and replaced by a neutral or negatively charged amino acid (*e.g.,* Ala or Glu) to determine whether the interaction of the antibody with antigen is affected. Further substitutions may be introduced at the amino acid locations to demonstrate functional sensitivity to the initial substitutions. Alternatively, or additionally, a crystal structure of an antigen-antibody complex can be determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.*, for ADEPT) or a polypeptide which increases the serum half-life of the antibody.

### Fc Region Variants

In some embodiments, one or more amino acid modifications may be introduced into the Fc region of an antibody (*e.g.*, a full-length anti-FXIIa antibody or anti-FXIIa Fc fusion protein) provided herein, thereby generating an Fc region variant. In some embodiments, the Fc region variant has enhanced ADCC effector function, often related to binding to Fc receptors (FcRs). In some embodiments, the Fc region variant has decreased ADCC effector function. There are many examples of changes or mutations to Fc sequences that can alter effector function. For example, WO 00/42072 and Shields et al., J Biol. Chem. 9(2): 6591-6604 (2001) describe antibody variants with improved or diminished binding to FcRs. The contents of those publications are specifically incorporated herein by reference.

Antibody-Dependent Cell-Mediated Cytotoxicity (ADCC) is a mechanism of action of therapeutic antibodies against tumor cells. ADCC is a cell-mediated immune defense whereby an effector cell of the immune system actively lyses a target cell (*e.g.,* a cancer cell), whose membrane-surface antigens have been bound by specific antibodies (*e.g.*, an anti-FXIIa antibody). The typical ADCC involves activation of NK cells by antibodies. An NK cell expresses CD16 which is an Fc receptor. This receptor recognizes, and binds to, the Fc portion of an antibody bound to the surface of a target cell. The most common Fc receptor on the surface of an NK cell is called CD16 or FcγRIII. Binding of the Fc receptor to the Fc region of an antibody results in NK cell activation, release of cytolytic granules and consequent target cell apoptosis. The contribution of ADCC to tumor cell killing can be measured with a specific test that uses NK-92 cells that have been transfected with a high-affinity FcR. Results are compared to wild-type NK-92 cells that do not express the FcR.

In some embodiments, the application contemplates an anti-FXIIa antibody variant (such as a full-length anti-FXIIa antibody variant) comprising an Fc region that possesses some but not all effector functions, which makes it a desirable candidate for applications in which the half-life of the anti-FXIIa antibody *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcyRII and FcγRIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Pat. No. 5,500,362 (see, *e.g.,* Hellstrom, I. et al., Proc. Nat'l Acad. Sci. USA 83:7059-7063 (1986)) and Hellstrom, I. et al., Proc. Nat'lAcad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI^{™} non-radioactive cytotoxicity assay for flow cytometry (CellTechnology, Inc. Mountain View, Calif.; and CYTOTOX 96^{™} non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al., Proc. Nat'lAcad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half-life determinations can also be performed using methods known in the art (see, *e.g.,* Petkova, S. B. et al., Int'l. Immunol. 18(12):1759-1769 (2006)).

Antibodies with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

Certain antibody variants with improved or diminished binding to FcRs are described. (See, *e.g.,* U.S. Pat. No. 6,737,056; WO 2004/056312, and Shields et al., J. Biol. Chem. 9(2): 6591-6604 (2001).)

In some embodiments, there is provided an anti-FXIIa antibody (such as a full-length anti-FXIIa antibody) variant comprising a variant Fc region comprising one or more amino acid substitutions which improve ADCC. In some embodiments, the variant Fc region comprises one or more amino acid substitutions which improve ADCC, wherein the substitutions are at positions 298, 333, and/or 334 of the variant Fc region (EU numbering of residues). In some embodiments, the anti-FXIIa antibody (e.g., full-length anti-FXIIa antibody) variant comprises the following amino acid substitution in its variant Fc region: S298A, E333A, and K334A.

In some embodiments, alterations are made in the Fc region that result in altered (*i.e.,* either improved or diminished) C1q binding and/or Complement Dependent Cytotoxicity (CDC), *e.g.,* as described in U.S. Pat. No. 6,194,551, WO 99/51642, and Idusogie et al., J. Immunol. 164: 4178-4184 (2000).

In some embodiments, there is provided an anti-FXIIa antibody (such as a full-length anti-FXIIa antibody) variant comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g*., substitution of Fc region residue 434 (U.S. Pat. No. 7,371,826).

*See also* Duncan & Winter, Nature 322:738-40 (1988); U.S. Pat. No. 5,648,260; U.S. Pat. No. 5,624,821; and WO 94/29351 concerning other examples of Fc region variants.

Anti-FXIIa antibodies (such as full-length anti-FXIIa antibodies) comprising any of the Fc variants described herein, or combinations thereof, are contemplated.

### Glycosylation Variants

In some embodiments, an anti-FXIIa antibody (such as a full-length anti-FXIIa antibody) provided herein is altered to increase or decrease the extent to which the anti-FXIIa antibody is glycosylated. Addition or deletion of glycosylation sites to an anti-FXIIa antibody may be conveniently accomplished by altering the amino acid sequence of the anti-FXIIa antibody or polypeptide portion thereof such that one or more glycosylation sites are created or removed.

Wherein the anti-FXIIa antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al., TIBTECH 15:26-32 (1997). The oligosaccharide may include various carbohydrates, *e.g.,* mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an anti-FXIIa antibody of the application may be made in order to create anti-FXIIa antibody variants with certain improved properties.

The N-glycans attached to the CH2 domain of Fc is heterogeneous. Antibodies or Fc fusion proteins generated in CHO cells are fucosylated by fucosyltransferase activity. See Shoji-Hosaka et al., J. Biochem. 2006, 140:777- 83. Normally, a small percentage of naturally occurring afucosylated IgGs may be detected in human serum. N-glycosylation of the Fc is important for binding to FcyR; and afucosylation of the N-glycan increases Fc's binding capacity to FcγRIIIa. Increased FcγRIIIa binding can enhance ADCC, which can be advantageous in certain antibody therapeutic applications in which cytotoxicity is desirable.

In some embodiments, an enhanced effector function can be detrimental when Fc-mediated cytotoxicity is undesirable. In some embodiments, the Fc fragment or CH2 domain is not glycosylated. In some embodiments, the N-glycosylation site in the CH2 domain is mutated to prevent from glycosylation.

In some embodiments, anti-FXIIa antibody (such as a full-length anti-FXIIa antibody) variants are provided comprising an Fc region wherein a carbohydrate structure attached to the Fc region has reduced fucose or lacks fucose, which may improve ADCC function. Specifically, anti-FXIIa antibodies are contemplated herein that have reduced fucose relative to the amount of fucose on the same anti-FXIIa antibody produced in a wild-type CHO cell. That is, they are characterized by having a lower amount of fucose than they would otherwise have if produced by native CHO cells *(e.g.,* a CHO cell that produce a native glycosylation pattern, such as, a CHO cell containing a native FUT8 gene). In some embodiments, the anti-FXIIa antibody is one wherein less than about 50%, 40%, 30%, 20%, 10%, or 5% of the N-linked glycans thereon comprise fucose. For example, the amount of fucose in such an anti-FXIIa antibody may be from 1% to 80%, from 1% to 65%, from 5% to 65% or from 20% to 40%. In some embodiments, the anti-FXIIa antibody is one wherein none of the N-linked glycans thereon comprise fucose, *i.e.,* wherein the anti-FXIIa antibody is completely without fucose, or has no fucose or is afucosylated. The amount of fucose is determined by calculating the average amount of fucose within the sugar chain at Asn297, relative to the sum of all glycostructures attached to Asn 297 (*e.g.*, complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ±3 amino acids upstream or downstream of position 297, *i.e.,* between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al., J. Mol. Biol. 336:1239-1249 (2004); Yamane-Ohnuki et al., Biotech. Bioeng. 87: 614 (2004). Examples of cell lines capable of producing defucosylated antibodies include Lec13 CHO cells deficient in protein fucosylation (Ripka et al., Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such asα-1,6-fucosyltransferase gene, FUT8, knockout CHO cells (see, *e.g.,* Yamane-Ohnuki et al., Biotech. Bioeng. 87: 614 (2004); Kanda, Y. et al., Biotechnol. Bioeng. 94(4):680-688 (2006); and WO2003/085107).

Anti-FXIIa antibody (such as a full-length anti-FXIIa antibody) variants are further provided with bisected oligosaccharides, e.g., in which a biantennary oligosaccharide attached to the Fc region of the anti-FXIIa antibody is bisected by GlcNAc. Such anti-FXIIa antibody (such as a full-length anti-FXIIa antibody) variants may have reduced fucosylation and/or improved ADCC function. Examples of such antibody variants are described, *e.g.,* in WO 2003/011878 (Jean-Mairet et al.); U.S. Pat. No. 6,602,684 (Umana et al.); US 2005/0123546 (Umana et al.), and Ferrara et al., Biotechnology and Bioengineering, 93(5): 851-861 (2006). Anti-FXIIa antibody (such as full-length anti-FXIIa antibody) variants with at least one galactose residue in the oligosaccharide attached to the Fc region are also provided. Such anti-FXIIa antibody variants may have improved CDC function. Such antibody variants are described, *e.g.,* in WO 1997/30087 (Patel et al.); WO 1998/58964 (Raju, S.); and WO 1999/22764 (Raju, S.).

In some embodiments, the anti-FXIIa antibody (such as a full-length anti-FXIIa antibody) variants comprising an Fc region are capable of binding to an FcγRIII. In some embodiments, the anti-FXIIa antibody (such as a full-length anti-FXIIa antibody) variants comprising an Fc region have ADCC activity in the presence of human effector cells (*e.g.,* T cell) or have increased ADCC activity in the presence of human effector cells compared to the otherwise same anti-FXIIa antibody (such as a full-length anti-FXIIa antibody) comprising a human wild-type IgG1Fc region.

### Cysteine Engineered Variants

In some embodiments, it may be desirable to create cysteine engineered anti-FXIIa antibodies (such as a full-length anti-FXIIa antibody) in which one or more amino acid residues are substituted with cysteine residues. In some embodiments, the substituted residues occur at accessible sites of the anti-FXIIa antibody. By substituting those residues with cysteine, reactive thiol groups are thereby positioned at accessible sites of the anti-FXIIa antibody and may be used to conjugate the anti-FXIIa antibody to other moieties, such as drug moieties or linker-drug moieties, to create an anti-FXIIa immunoconjugate, as described further herein. Cysteine engineered anti-FXIIa antibodies (*e.g.*, full-length anti-FXIIa antibodies) may be generated as described, *e.g.*, in U.S. Pat. No. 7,521,541.

### Derivatives

In some embodiments, an anti-FXIIa antibody (such as a full-length anti-FXIIa antibody) provided herein may be further modified to contain additional non-proteinaceous moieties that are known in the art and readily available. The moieties suitable for derivatization of the anti-FXIIa antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (*e.g.*, glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the anti-FXIIa antibody may vary, and if more than one polymer are attached, they can be the same or different molecules. In general, the number and/or type of polymers used for derivatization can be determined based on considerations including, but not limited to, the particular properties or functions of anti-FXIIa antibody to be improved, whether the anti-FXIIa antibody derivative will be used in a therapy under defined conditions, *etc.*

### Pharmaceutical Compositions

Also provided herein are compositions (such as pharmaceutical compositions, also referred to herein as formulations) comprising any of the anti-FXIIa antibodies (such as a full-length anti-FXIIa antibody), nucleic acids encoding the antibodies, vectors comprising the nucleic acids encoding the antibodies, or host cells comprising the nucleic acids or vectors described herein. In some embodiments, there is provided a pharmaceutical composition comprising any one of the anti-FXIIa antibodies described herein and a pharmaceutically acceptable carrier.

Suitable formulations of the anti-FXIIa antibodies are obtained by mixing an anti-FXIIa antibody having the desired degree of purity with optional pharmaceutically acceptable carriers, excipients or stabilizers *(*Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG). Exemplary formulations are described in WO98/56418, expressly incorporated herein by reference. Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be treated herein. Lipofectins or liposomes can be used to deliver the anti-FXIIa antibodies of this application into cells.

The formulation herein may also contain one or more active compounds in addition to the anti-FXIIa antibody (such as a full-length anti-FXIIa antibody) as necessary for the particular indication being treated, preferably those with complementary activities that do not adversely affect each other. For example, it may be desirable to further provide an anti-thrombotic agents, anti-platelet agents (such as anti-thrombotic agents, anti-platelet agents (e.g. cyclooxygenase inhibitors, adenosine diphosphate receptor inhibitors, phosphodiesterase inhibitors, glycoprotein IIB/IIIA inhibitors, adenosine reuptake inhibitors, thromboxane inhibitors), anticoagulants (e.g. vitamin K antagonists, heparin, bivalirudin, antithrombin II, apixaban), fibrinolytic agents (e.g. anistreplase, reteplase, streptokinase), anti-inflammatory agents (e.g., NSAID, corticosteroids), C1 esterase inhibitors; azathioprine, pirfenidone, cyclophosphamide, acetylcysteine) in addition to the anti-FXIIa antibody. Such molecules are suitably present in combination in amounts that are effective for the purpose intended. The effective amount of such other agents depends on the amount of anti-FXIIa antibody present in the formulation, the type of disease or disorder or treatment, and other factors discussed above. These are generally used in the same dosages and with administration routes as described herein or about from 1 to 99% of the heretofore employed dosages.

The anti-FXIIa antibodies (e.g., full-length anti-FXIIa antibodies) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Sustained-release preparations may be prepared.

Sustained-release preparations of the anti-FXIIa antibodies (e.g., full-length anti-FXIIa antibodies) can be prepared. Suitable examples of sustained-release preparations include semipermeable matrices of solid hydrophobic polymers containing the antibody (or fragment thereof), which matrices are in the form of shaped articles, e.g., films, or microcapsules. Examples of sustained-release matrices include polyesters, hydrogels (for example, poly(2-hydroxyethyl-methacrylate ), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D (-)-3-hydroxybutyric acid. While polymers such as ethylene-vinyl acetate and lactic acid-glycolic acid enable release of molecules for over 100 days, certain hydro gels release proteins for shorter time periods. When encapsulated antibody remain in the body for a long time, they can denature or aggregate as a result of exposure to moisture at 37°C, resulting in a loss of biological activity and possible changes in immunogenicity. Rational strategies can be devised for stabilization of anti-FXIIa antibodies depending on the mechanism involved. For example, if the aggregation mechanism is discovered to be intermolecular S-S bond formation through thio-disulfide interchange, stabilization can be achieved by modifying sulfhydryl residues, lyophilizing from acidic solutions, controlling moisture content, using appropriate additives, and developing specific polymer matrix compositions.

In some embodiments, the anti-FXIIa antibody (such as a full-length anti-FXIIa antibody) is formulated in a buffer comprising a citrate, NaCl, acetate, succinate, glycine, polysorbate 80 (Tween 80), or any combination of the foregoing.

The formulations to be used for in vivo administration must be sterile. This is readily accomplished by, *e.g.*, filtration through sterile filtration membranes.

### Methods of treatment using anti-FXIIa antibodies

The anti-FXIIa antibodies (*e.g.*, full-length anti-FXIIa antibodies) and/or compositions of the application can be administered to individuals (*e.g.*, mammals such as humans) to treat a disease and/or disorder associated with FXIIa signaling (*e.g.*, thrombosis, FXII/FXIIa induced kinin formation related disease, FXII/FXIIa induced complement activation related disease, contact activation system related disease, inflammation related diseases). These diseases include, but are not limited to, venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. The present application thus in some embodiments provides a method of treating a disease or condition associated with FXIIa signaling (*e.g.*, thrombosis, FXII/FXIIa induced kinin formation related disease, FXII/FXIIa induced complement activation related disease, contact activation system related disease, inflammation related diseases) in an individual comprising administering to the individual an effective amount of a composition (such as a pharmaceutical composition) comprising an anti-FXIIa antibody (*e.g.*, a full-length anti-FXIIa antibody), such as any one of the anti-FXIIa antibodies (*e.g.*, full-length anti-FXIIa antibodies) described herein. In some embodiments, the individual is human.

For example, in some embodiments, there is provided a method of treating an individual having a disease or condition associated with FXIIa signaling (e.g., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (e.g., full-length anti-FXIIa antibody) specifically binding to an epitope on human FXIIa , wherein the epitope comprises amino acid residues of human FXIIa. In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

For example, in some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising a heavy chain variable domain (V_{H}) comprising a heavy chain complementarity determining region (HC-CDR) 1 comprising SQTMS (SEQ ID NO: 1); an HC-CDR2 comprising SX₁X₂X₃ISX₄RX₅YYADSVKG (SEQ ID NO: 89), wherein X₁ is I or S, X₂ is H or S, X₃ is H, V, or Y, X₄ is G, K, or R, and X₅ is A, D, K, M, N, P, R, S, T, or Y; and an HC-CDR3 comprising X₁HX₂FDX₃ (SEQ ID NO: 90), wherein X₁ is A, G, H, Q, S, or W, X₂ is A, D, or R, and X₃ is L or V; and a light chain variable domain (V_{L}) comprising a light chain complementarity determining region (LC-CDR) 1 comprising RASQSX₁X₂X₃X₄LA (SEQ ID NO: 91), wherein X₁ is L or V, X₂ is F, W, or Y, X₃ is S or Y, and X₄ is A, H, or N; an LC-CDR2 comprising GASTRAT (SEQ ID NO: 65); and an LC-CDR3 comprising QQX₁X₂X₃WPLS (SEQ ID NO: 92), wherein X₁ is N or Y, X₂ is H, K, N, or Q, and X₃ is K, R, or W. In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of any one of SEQ ID NOs: 10-25, and an HC-CDR3 comprising the amino acid sequence of any one of SEQ ID NOs: 34-42, or a variant thereof comprising up to 5 amino acid substitutions in the HC-CDRs; and a V_{L} comprising an LC-CDR1 comprising the amino acid sequence of any one of SEQ ID NOs: 51-56, an LC-CDR2 comprising the amino acid sequence of SEQ ID NO: 65, and an LC-CDR3 comprising the amino acid sequence of any one of SEQ ID NOs: 74-80, or a variant thereof comprising up to 5 amino acid substitutions in the LC-CDRs.

In some embodiments, there is provided a method of treating an individual having a disease or condition associated with FXIIa signaling (e.g., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (e.g., full-length anti-FXIIa antibody) comprising a V_{H} comprising the amino acid sequence of any one of SEQ ID NOs: 93-130 or a variant thereof having at least about 80% sequence identity to the amino acid sequence of any one of SEQ ID NOs: 93-130, and a V_{L} comprising the amino acid sequence of any one of SEQ ID NOs: 139-148, or a variant thereof having at least about 80% sequence identity to the amino acid sequence of any one of SEQ ID NOs: 139-148.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74, or a variant thereof comprising up to 5 amino acid substitutions in the LC-CDRs.

In some embodiments, the anti-FXIIa antibody provided herein comprises a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 139, or a variant thereof having at least about 80% sequence identity to the amino acid sequence of SEQ ID NO: 139. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g*., full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76, or a variant thereof comprising up to 5 amino acid substitutions in the LC-CDRs.

In some embodiments, the anti-FXIIa antibody provided herein comprises a V_{H} comprising the amino acid sequence of SEQ ID NO: 95, or a variant thereof having at least about 80% sequence identity to the amino acid sequence of SEQ ID NO: 95; and a V_{L} comprising the amino acid sequence of SEQ ID NO: 141, or a variant thereof having at least about 80% sequence identity to the amino acid sequence of SEQ ID NO: 141. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g*., full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 96, or a variant thereof having at least about 80% sequence identity to the amino acid sequence of SEQ ID NO: 96; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 97, or a variant thereof having at least about 80% sequence identity to the amino acid sequence of SEQ ID NO: 97; and a V_{L} comprising the amino acid sequence of SEQ ID NO: 143, or a variant thereof having at least about 80% sequence identity to the amino acid sequence of SEQ ID NO: 143. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 98, or a variant thereof having at least about 80% sequence identity to the amino acid sequence of SEQ ID NO: 98; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.,* thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 96, or a variant thereof having at least about 80% sequence identity to the amino acid sequence of SEQ ID NO: 96; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 99, or a variant thereof having at least about 80% sequence identity to the amino acid sequence of SEQ ID NO: 99; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g*., full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 100, or a variant thereof having at least about 80% sequence identity to the amino acid sequence of SEQ ID NO: 100; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 101, or a variant thereof having at least about 80% sequence identity to the amino acid sequence of SEQ ID NO: 101; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 102, or a variant thereof having at least about 80% sequence identity to the amino acid sequence of SEQ ID NO: 102; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37, or a variant thereof comprising up to 5 amino acid substitutions in the HC-CDRs; and a V_{L} comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77, or a variant thereof comprising up to 5 amino acid substitutions in the LC-CDRs.

In some embodiments, the anti-FXIIa antibody provided herein comprises a V_{H} comprising the amino acid sequence of SEQ ID NO: 103, or a variant thereof having at least about 80% sequence identity to the amino acid sequence of SEQ ID NO: 103; and a V_{L} comprising the amino acid sequence of SEQ ID NO: 144, or a variant thereof having at least about 80% sequence identity to the amino acid sequence of SEQ ID NO: 144. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 104, or a variant thereof having at least about 80% sequence identity to the amino acid sequence of SEQ ID NO: 104; and a V_{L} comprising the amino acid sequence of SEQ ID NO: 145, or a variant thereof having at least about 80% sequence identity to the amino acid sequence of SEQ ID NO: 145. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76, or a variant thereof comprising up to 5 amino acid substitutions in the LC-CDRs.

In some embodiments, the anti-FXIIa antibody provided herein comprises a V_{H} comprising the amino acid sequence of SEQ ID NO: 105, or a variant thereof having at least about 80% sequence identity to the amino acid sequence of SEQ ID NO: 105; and a V_{L} comprising the amino acid sequence of SEQ ID NO: 141, or a variant thereof having at least about 80% sequence identity to the amino acid sequence of SEQ ID NO: 141. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g*., full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 106, or a variant thereof having at least about 80% sequence identity to the amino acid sequence of SEQ ID NO: 106; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g*., full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 107, or a variant thereof having at least about 80% sequence identity to the amino acid sequence of SEQ ID NO: 107; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 108, or a variant thereof having at least about 80% sequence identity to the amino acid sequence of SEQ ID NO: 108; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 109, or a variant thereof having at least about 80% sequence identity to the amino acid sequence of SEQ ID NO: 109; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 110, or a variant thereof having at least about 80% sequence identity to the amino acid sequence of SEQ ID NO: 110; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 111, or a variant thereof having at least about 80% sequence identity to the amino acid sequence of SEQ ID NO: 111; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 112, or a variant thereof having at least about 80% sequence identity to the amino acid sequence of SEQ ID NO: 112; and a V_{L} comprising the amino acid sequence of SEQ ID NO: 141, or a variant thereof having at least about 80% sequence identity to the amino acid sequence of SEQ ID NO: 141. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 113, or a variant thereof having at least about 80% sequence identity to the amino acid sequence of SEQ ID NO: 113; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 114, or a variant thereof having at least about 80% sequence identity to the amino acid sequence of SEQ ID NO: 114; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 108, or a variant thereof having at least about 80% sequence identity to the amino acid sequence of SEQ ID NO: 108; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 115, or a variant thereof having at least about 80% sequence identity to the amino acid sequence of SEQ ID NO: 115; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78, or a variant thereof comprising up to 5 amino acid substitutions in the LC-CDRs.

In some embodiments, the anti-FXIIa antibody provided herein comprises a V_{H} comprising the amino acid sequence of SEQ ID NO: 116, or a variant thereof having at least about 80% sequence identity to the amino acid sequence of SEQ ID NO: 116; and a V_{L} comprising the amino acid sequence of SEQ ID NO: 146, or a variant thereof having at least about 80% sequence identity to the amino acid sequence of SEQ ID NO: 146. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 117, or a variant thereof having at least about 80% sequence identity to the amino acid sequence of SEQ ID NO: 117; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 118, or a variant thereof having at least about 80% sequence identity to the amino acid sequence of SEQ ID NO: 118; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 104, or a variant thereof having at least about 80% sequence identity to the amino acid sequence of SEQ ID NO: 104; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 119, or a variant thereof having at least about 80% sequence identity to the amino acid sequence of SEQ ID NO: 119; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 120, or a variant thereof having at least about 80% sequence identity to the amino acid sequence of SEQ ID NO: 120; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 111, or a variant thereof having at least about 80% sequence identity to the amino acid sequence of SEQ ID NO: 111; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 121, or a variant thereof having at least about 80% sequence identity to the amino acid sequence of SEQ ID NO: 121; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76, or a variant thereof comprising up to 5 amino acid substitutions in the LC-CDRs.

In some embodiments, the anti-FXIIa antibody provided herein comprises a V_{H} comprising the amino acid sequence of SEQ ID NO: 122, or a variant thereof having at least about 80% sequence identity to the amino acid sequence of SEQ ID NO: 122; and a V_{L} comprising the amino acid sequence of SEQ ID NO: 141, or a variant thereof having at least about 80% sequence identity to the amino acid sequence of SEQ ID NO: 141. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 123, or a variant thereof having at least about 80% sequence identity to the amino acid sequence of SEQ ID NO: 123; and a V_{L} comprising the amino acid sequence of SEQ ID NO: 142, or a variant thereof having at least about 80% sequence identity to the amino acid sequence of SEQ ID NO: 142. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 124, or a variant thereof having at least about 80% sequence identity to the amino acid sequence of SEQ ID NO: 124; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 125, or a variant thereof having at least about 80% sequence identity to the amino acid sequence of SEQ ID NO: 125; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 126, or a variant thereof having at least about 80% sequence identity to the amino acid sequence of SEQ ID NO: 126; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g*., full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79, or a variant thereof comprising up to 5 amino acid substitutions in the LC-CDRs.

In some embodiments, the anti-FXIIa antibody provided herein comprises a V_{H} comprising the amino acid sequence of SEQ ID NO: 112, or a variant thereof having at least about 80% sequence identity to the amino acid sequence of SEQ ID NO: 112; and a V_{L} comprising the amino acid sequence of SEQ ID NO: 147, or a variant thereof having at least about 80% sequence identity to the amino acid sequence of SEQ ID NO: 147. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39, or a variant thereof comprising up to 5 amino acid substitutions in the HC-CDRs; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80, or a variant thereof comprising up to 5 amino acid substitutions in the LC-CDRs.

In some embodiments, the anti-FXIIa antibody provided herein comprises a V_{H} comprising the amino acid sequence of SEQ ID NO: 108, or a variant thereof having at least about 80% sequence identity to the amino acid sequence of SEQ ID NO: 108; and a V_{L} comprising the amino acid sequence of SEQ ID NO: 148, or a variant thereof having at least about 80% sequence identity to the amino acid sequence of SEQ ID NO: 148. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 127, or a variant thereof having at least about 80% sequence identity to the amino acid sequence of SEQ ID NO: 127; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 128, or a variant thereof having at least about 80% sequence identity to the amino acid sequence of SEQ ID NO: 128; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the anti-FXIIa antibody provided herein comprises a V_{H} comprising the amino acid sequence of SEQ ID NO: 129, or a variant thereof having at least about 80% sequence identity to the amino acid sequence of SEQ ID NO: 129; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, there is provided a method of treating an individual having a disease or condition associated with FXIIa signaling (*e.g.*, thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e.g.*, full-length anti-FXIIa antibody) comprising: a V_{H} comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75, or a variant thereof comprising up to 5 amino acid substitutions in the LC-CDRs.

In some embodiments, the anti-FXIIa antibody provided herein comprises a V_{H} comprising the amino acid sequence of SEQ ID NO: 130, or a variant thereof having at least about 80% sequence identity to the amino acid sequence of SEQ ID NO: 130; and a V_{L} comprising the amino acid sequence of SEQ ID NO: 140, or a variant thereof having at least about 80% sequence identity to the amino acid sequence of SEQ ID NO: 140. In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 2, an HC-CDR2 comprising the amino acid sequence of SEQ ID NO: 26, and an HC-CDR3 comprising the amino acid sequence of SEQ ID NO: 43, or a variant thereof comprising up to 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 66, and an LC-CDR3 comprising the amino acid sequence of SEQ ID NO: 81, or a variant thereof comprising up to 5 amino acid substitutions in the LC-CDRs.In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 131 or a variant thereof having at least about 80% sequence identity to the amino acid sequence of SEQ ID NO: 131, and a V_{L} comprising the amino acid sequence of SEQ ID NO: 149, or a variant thereof having at least about 80% sequence identity to the amino acid sequence of SEQ ID NO: 149.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 3, an HC-CDR2 comprising the amino acid sequence of SEQ ID NO: 27, and an HC-CDR3 comprising the amino acid sequence of SEQ ID NO: 44, or a variant thereof comprising up to 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 67, and an LC-CDR3 comprising the amino acid sequence of SEQ ID NO: 82, or a variant thereof comprising up to 5 amino acid substitutions in the LC-CDRs.In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 132 or a variant thereof having at least about 80% sequence identity to the amino acid sequence of SEQ ID NO: 132, and a V_{L} comprising the amino acid sequence of SEQ ID NO: 150, or a variant thereof having at least about 80% sequence identity to the amino acid sequence of SEQ ID NO: 150.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 4, an HC-CDR2 comprising the amino acid sequence of SEQ ID NO: 28, and an HC-CDR3 comprising the amino acid sequence of SEQ ID NO: 45, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 68, and an LC-CDR3 comprising the amino acid sequence of SEQ ID NO: 83, or a variant thereof comprising up to 5 amino acid substitutions in the LC-CDRs. In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 133 or a variant thereof having at least about 80% sequence identity to the amino acid sequence of SEQ ID NO: 133, and a V_{L} comprising the amino acid sequence of SEQ ID NO: 151, or a variant thereof having at least about 80% sequence identity to the amino acid sequence of SEQ ID NO: 151.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 5, an HC-CDR2 comprising the amino acid sequence of SEQ ID NO: 29, and an HC-CDR3 comprising the amino acid sequence of SEQ ID NO: 46, or a variant thereof comprising up to 5 amino acid substitutions in the HC-CDRs; and a V_{L} comprising an LC-CDR1 comprising the amino acid sequence of SEQ ID NO: 60, an LC-CDR2 comprising the amino acid sequence of SEQ ID NO: 69, and an LC-CDR3 comprising the amino acid sequence of SEQ ID NO: 84, or a variant thereof comprising up to 5 amino acid substitutions in the LC-CDRs.In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 134 or a variant thereof having at least about 80% sequence identity to the amino acid sequence of SEQ ID NO: 134, and a V_{L} comprising the amino acid sequence of SEQ ID NO: 152, or a variant thereof having at least about 80% sequence identity to the amino acid sequence of SEQ ID NO: 152.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 6, an HC-CDR2 comprising the amino acid sequence of SEQ ID NO: 30, and an HC-CDR3 comprising the amino acid sequence of SEQ ID NO: 47, or a variant thereof comprising up to 5 amino acid substitutions in the HC-CDRs; and a V_{L} comprising an LC-CDR1 comprising the amino acid sequence of SEQ ID NO: 61, an LC-CDR2 comprising the amino acid sequence of SEQ ID NO: 70, and an LC-CDR3 comprising the amino acid sequence of SEQ ID NO: 85, or a variant thereof comprising up to 5 amino acid substitutions in the LC-CDRs.In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 135 or a variant thereof having at least about 80% sequence identity to the amino acid sequence of SEQ ID NO: 135, and a V_{L} comprising the amino acid sequence of SEQ ID NO: 153, or a variant thereof having at least about 80% sequence identity to the amino acid sequence of SEQ ID NO: 153.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 7, an HC-CDR2 comprising the amino acid sequence of SEQ ID NO: 31, and an HC-CDR3 comprising the amino acid sequence of SEQ ID NO: 48, or a variant thereof comprising up to 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 71, and an LC-CDR3 comprising the amino acid sequence of SEQ ID NO: 86, or a variant thereof comprising up to 5 amino acid substitutions in the LC-CDRs. In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 136 or a variant thereof having at least about 80% sequence identity to the amino acid sequence of SEQ ID NO: 136, and a V_{L} comprising the amino acid sequence of SEQ ID NO: 154, or a variant thereof having at least about 80% sequence identity to the amino acid sequence of SEQ ID NO: 154.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 8, an HC-CDR2 comprising the amino acid sequence of SEQ ID NO: 32, and an HC-CDR3 comprising the amino acid sequence of SEQ ID NO: 49, or a variant thereof comprising up to 5 amino acid substitutions in the HC-CDRs; and a V_{L} comprising an LC-CDR1 comprising the amino acid sequence of SEQ ID NO: 63, an LC-CDR2 comprising the amino acid sequence of SEQ ID NO: 72, and an LC-CDR3 comprising the amino acid sequence of SEQ ID NO: 87, or a variant thereof comprising up to 5 amino acid substitutions in the LC-CDRs. In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 137 or a variant thereof having at least about 80% sequence identity to the amino acid sequence of SEQ ID NO: 137, and a V_{L} comprising the amino acid sequence of SEQ ID NO: 155, or a variant thereof having at least about 80% sequence identity to the amino acid sequence of SEQ ID NO: 155.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

For example, in some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising an HC-CDR1 comprising the amino acid sequence of SEQ ID NO: 9, an HC-CDR2 comprising the amino acid sequence of SEQ ID NO: 33, and an HC-CDR3 comprising the amino acid sequence of SEQ ID NO: 50, or a variant thereof comprising up to 5 amino acid substitutions in the HC-CDRs; and a V_{L} comprising an LC-CDR1 comprising the amino acid sequence of SEQ ID NO: 64, an LC-CDR2 comprising the amino acid sequence of SEQ ID NO: 73, and an LC-CDR3 comprising the amino acid sequence of SEQ ID NO: 88, or a variant thereof comprising up to 5 amino acid substitutions in the LC-CDRs.In some embodiments, the anti-FXIIa antibody is a full-length antibody. In some embodiments, the full-length anti-FXIIa antibody is an IgG1 or IgG4 antibody. In some embodiments, the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis. In some embodiments, the individual is human.

In some embodiments, there is provided a method of treating an individual having a disease or condition associated with FXIIa signaling (*e*.*g*., thrombosis, FXII/FXIIa induced kinin formation related disease, FXII/FXIIa induced complement activation related disease, contact activation system related disease, inflammation related diseases) comprising administering to the individual an effective amount of a pharmaceutical composition comprising an anti-FXIIa antibody (*e*.*g*., full-length anti-FXIIa antibody) comprising a V_{H} comprising the amino acid sequence of SEQ ID NO: 138 or a variant thereof having at least about 80% sequence identity to the amino acid sequence of SEQ ID NO: 138, and a V_{L} comprising the amino acid sequence of SEQ ID NO: 156, or a variant thereof having at least about 80% sequence identity to the amino acid sequence of SEQ ID NO: 156.

In some embodiments, the anti-FXIIa antibody provided herein is a full-length anti-FXIIa antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 157. In some embodiments, the heavy chain constant region comprises or consists of the amino acid sequence of SEQ ID NO: 158. In some embodiments, the light chain constant region comprises or consists of the amino acid sequence of SEQ ID NO: 159. In some embodiments, the light chain constant region comprises or consists of the amino acid sequence of SEQ ID NO: 160.

In some embodiments, the individual is a mammal (*e*.*g*., human, non-human primate, rat, mouse, cow, horse, pig, sheep, goat, dog, cat, *etc*.). In some embodiments, the individual is a human. In some embodiments, the individual is a clinical patient, a clinical trial volunteer, an experimental animal, *etc.* In some embodiments, the individual is younger than about 60 years old (including for example younger than about any of 50, 40, 30, 25, 20, 15, or 10 years old). In some embodiments, the individual is older than about 60 years old (including for example older than about any of 70, 80, 90, or 100 years old). In some embodiments, the individual is diagnosed with or genetically prone to one or more of the diseases or disorders described herein (such as thrombosis, FXII/FXIIa induced kinin formation related disease, FXII/FXIIa induced complement activation related disease, contact activation system related disease, inflammation related diseases). In some embodiments, the individual has one or more risk factors associated with one or more diseases or disorders described herein.

The present application in some embodiments provides a method of delivering an anti-FXIIa antibody (such as any one of the anti-FXIIa antibodies described herein, *e.g.*, an isolated anti-FXIIa antibody) to a cell producing FXIIa in an individual, the method comprising administering to the individual a composition comprising the anti-FXIIa antibody.

Many diagnostic methods for thrombosis, FXII/FXIIa induced kinin formation related disease, FXII/FXIIa induced complement activation related disease, contact activation system related disease, inflammation related diseases or any other disease associated with FXIIa signaling and the clinical delineation of those diseases are known in the art. Such methods include, but are not limited to, *e.g.*, immunohistochemistry, PCR, and fluorescent in situ hybridization (FISH).

In some embodiments, the anti-FXIIa antibodies (*e*.*g*., full-length anti-FXIIa antibodies) and/or compositions of the application are administered in combination with a second, third, or fourth agent (including, *e*.*g*., anti-thrombotic agents, anti-platelet agents (*e*.*g*. cyclooxygenase inhibitors, adenosine diphosphate receptor inhibitors, phosphodiesterase inhibitors, glycoprotein IIB/IIIA inhibitors, adenosine reuptake inhibitors, thromboxane inhibitors), anticoagulants (e.g. vitamin K antagonists, heparin, bivalirudin, antithrombin II, apixaban), fibrinolytic agents (e.g. anistreplase, reteplase, streptokinase), anti-inflammatory agents (e.g., NSAID, corticosteroids), C1 esterase inhibitors; azathioprine, pirfenidone, cyclophosphamide, acetylcysteine) to treat diseases or disorders associated with FXIIa signaling.

Anti-thrombotic therapy is evaluated using parameters such as plasma kallikrein activity, activated partial thromboplastin time (aPTT), thrombus size, fibrinogen count, and platelet count. Methods for determining treatment efficacy can be employed, including, for example, detecting response through immunofluorescence staining, radiographic imaging, anatomical experiments, or behavioral experiments.

In some embodiments, the therapeutic effect is evaluated by the percentage decrease in fibrinogen (%), calculated using equation 100- (T/C × 100), where T is the amount of fibrin in the treated thrombus and C is the amount of fibrin in the untreated thrombus. In some embodiments, the percentage (%) is about 10%, 20%, 30%, 40%, 50%, 60%, 70%, 80%, 90%, 91%, 92%, 93%, 94%, 95% or more than 95%.

In some embodiments, the therapeutic effect is evaluated by thrombus size, calculated by the rate of thrombus size change compared to before antibody administration. In some embodiments, the thrombus size is reduced by at least about 1%, 2%, 5%, 10%, 15%, 20%, 25%, 30%, or 35%.

In some embodiments, the therapeutic effect is evaluated by platelet deposition levels. In some embodiments, platelet deposition is reduced by at least about 10%, 20%, 30%, 40%, 50%, 60%, 70%, 80%, 90%, 91%, 92%, 93%, 94%, 95% or more.

### Dosing and method of administering the anti-FXIIa antibodies

The dose of the anti-FXIIa antibody (such as isolated anti-FXIIa antibody) compositions administered to an individual (such as a human) may vary with the particular composition, the mode of administration, and the type of disease being treated. In some embodiments, the amount of the composition (such as composition comprising isolated anti-FXIIa antibody) is effective to result in an objective response (such as a partial response or a complete response) in the treatment of thrombosis, FXII/FXIIa induced kinin formation related disease, FXII/FXIIa induced complement activation related disease, contact activation system related disease, inflammation related diseases. In some embodiments, the amount of the anti-FXIIa antibody composition is sufficient to result in a complete response in the individual. In some embodiments, the amount of the anti-FXIIa antibody composition is sufficient to result in a partial response in the individual. In some embodiments, the amount of the anti-FXIIa antibody composition administered (for example when administered alone) is sufficient to produce an overall response rate of more than about any of 20%, 25%, 30%, 35%, 40%, 45%, 50%, 55%, 60%, 64%, 65%, 70%, 75%, 80%, 85%, or 90% among a population of individuals treated with the anti-FXIIa antibody composition. Responses of an individual to the treatment of the methods described herein can be determined, for example, based on thrombotic volume.

In some embodiments, the amount of the composition (such as composition comprising isolated anti-FXIIa antibody) is sufficient to control symptoms and reduce the risk of exacerbations of the individual. In some embodiments, the amount of the composition is sufficient to control symptoms and reduce the risk of exacerbations of the individual. In some embodiments, the amount of the composition (for example when administered along) is sufficient to produce clinical benefit of more than about any of 50%, 60%, 70%, or 77% among a population of individuals treated with the anti-FXIIa antibody composition.

In some embodiments, the amount of the composition (such as composition comprising isolated anti-FXIIa antibody), alone or in combination with a second, third, and/or fourth agent, is an amount sufficient to control symptoms and reduce the risk of exacerbations in the same subject prior to treatment or compared to the corresponding activity in other subjects not receiving the treatment. Standard methods can be used to measure the magnitude of this effect, such as *in vitro* assays with purified enzyme, cell-based assays, animal models, or human testing.

In some embodiments, the amount of the anti-FXIIa antibody (such as a full-length anti-FXIIa antibody) in the composition is below the level that induces a toxicological effect (*i.e*., an effect above a clinically acceptable level of toxicity) or is at a level where a potential side effect can be controlled or tolerated when the composition is administered to the individual.

In some embodiments, the amount of the composition is close to a maximum tolerated dose (MTD) of the composition following the same dosing regimen. In some embodiments, the amount of the composition is more than about any of 80%, 90%, 95%, or 98% of the MTD.

In some embodiments, the amount of an anti-FXIIa antibody (such as a full-length anti-FXIIa antibody) in the composition is included in a range of about 0.001 µg to about 1000 µg.

In some embodiments of any of the above aspects, the effective amount of anti-FXIIa antibody (such as a full-length anti-FXIIa antibody) in the composition is in the range of about 0.1 µg/kg to about 100 mg/kg of total body weight.

The anti-FXIIa antibody compositions can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal or transdermal. In some embodiments, sustained continuous release formulation of the composition may be used. In some embodiments, the composition is administered inhaled. In some embodiments, the composition is administered intravenously. In some embodiments, the composition is administered intraportally. In some embodiments, the composition is administered intraarterially. In some embodiments, the composition is administered intraperitoneally. In some embodiments, the composition is administered intrahepatically. In some embodiments, the composition is administered by hepatic arterial infusion. In some embodiments, the administration is to an injection site distal to a first disease site.

### Articles of Manufacture and Kits

In some embodiments of the application, there is provided an article of manufacture containing materials useful for the treatment of disease or condition associated with FXIIa signaling, (*e*.*g*., thrombosis, FXII/FXIIa induced kinin formation related disease, FXII/FXIIa induced complement activation related disease, contact activation system related disease, inflammation related diseases) or for delivering an anti-FXIIa antibody (such as a full-length anti-FXIIa antibody) to a cell producing FXIIa of the individual. The article of manufacture can comprise a container and a label or package insert on or associated with the container. Suitable containers include, for example, bottles, vials, syringes, *etc.* The containers may be formed from a variety of materials such as glass or plastic. Generally, the container holds a composition which is effective for treating a disease or disorder described herein, and may have a sterile access port (for example, the container may be an intravenous solution bag or a vial having a stopper pierceable by a hypodermic injection needle). At least one active agent in the composition is an anti-FXIIa antibody of the application. The label or package insert indicates that the composition is used for treating the particular condition. The label or package insert will further comprise instructions for administering the anti-FXIIa antibody composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating a disease or condition associated with FXIIa signaling (such as thrombosis, FXII/FXIIa induced kinin formation related disease, FXII/FXIIa induced complement activation related disease, contact activation system related disease, inflammation related diseases). In some embodiments, the package insert indicates that the composition is used for treating disease or condition selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, multiple sclerosis.

Additionally, the article of manufacture may further comprise a second container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

Kits are also provided that are useful for various purposes, *e.g.*, for treatment of disease or condition associated with FXIIa signaling (*e*.*g*., thrombosis, FXII/FXIIa induced kinin formation related disease, FXII/FXIIa induced complement activation related disease, contact activation system related disease, inflammation related diseases), or for delivering an anti-FXIIa antibody (such as a full-length anti-FXIIa antibody) to a cell producing FXIIa of the individual, optionally in combination with the articles of manufacture. Kits of the application include one or more containers comprising anti-FXIIa antibody composition (or unit dosage form and/or article of manufacture), and in some embodiments, further comprise another agent (such as the agents described herein) and/or instructions for use in accordance with any of the methods described herein. The kit may further comprise a description of selection of individuals suitable for treatment. Instructions supplied in the kits of the application are typically written instructions on a label or package insert (*e.g.*, a paper sheet included in the kit), but machine-readable instructions (*e.g*., instructions carried on a magnetic or optical storage disk) are also acceptable.

For example, in some embodiments, the kit comprises a composition comprising an anti-FXIIa antibody (such as a full-length anti-FXIIa antibody). In some embodiments, the kit comprises a) a composition comprising any one of the anti-FXIIa antibodies described herein, and b) an effective amount of at least one other agent, wherein the other agent enhances the effects (*e*.*g*., treatment effect, detecting effect) of the anti-FXIIa antibody. In some embodiments, the kit comprises a) a composition comprising any one of the anti-FXIIa antibodies described herein, and b) instructions for administering the anti-FXIIa antibody composition to an individual for treatment of a disease or condition associated with FXIIa signaling (*e*.*g*., thrombosis, FXII/FXIIa induced kinin formation related disease, FXII/FXIIa induced complement activation related disease, contact activation system related disease, inflammation related diseases). In some embodiments, the kit comprises a) a composition comprising any one of the anti-FXIIa antibodies described herein, b) an effective amount of at least one other agent, wherein the other agent enhances the effect (*e*.*g*., treatment effect, detecting effect) of the anti-FXIIa antibody, and c) instructions for administering the anti-FXIIa antibody composition and the other agent(s) to an individual for treatment of a disease or condition associated with FXIIa signaling (*e*.*g*., thrombosis, FXII/FXIIa induced kinin formation related disease, FXII/FXIIa induced complement activation related disease, contact activation system related disease, inflammation related diseases. The anti-FXIIa antibody and the other agent(s) can be present in separate containers or in a single container. For example, the kit may comprise one distinct composition or two or more compositions wherein one composition comprises an anti-FXIIa antibody and another composition comprises another agent.

In some embodiments, the kit comprises a nucleic acid (or a set of nucleic acids) encoding an anti-FXIIa antibody (such as a full-length anti-FXIIa antibody). In some embodiments, the kit comprises a) a nucleic acid (or a set of nucleic acids) encoding an anti-FXIIa antibody, and b) a host cell for expressing the nucleic acid (or a set of nucleic acids). In some embodiments, the kit comprises a) a nucleic acid (or a set of nucleic acids) encoding an anti-FXIIa antibody, and b) instructions for i) expressing the anti-FXIIa antibody in a host cell, ii) preparing a composition comprising the anti-FXIIa antibody, and iii) administering the composition comprising the anti-FXIIa antibody to an individual for the treatment of a disease or condition associated with FXIIa signaling (*e*.*g*., thrombosis, FXII/FXIIa induced kinin formation related disease, FXII/FXIIa induced complement activation related disease, contact activation system related disease, inflammation related diseases. In some embodiments, the kit comprises a) a nucleic acid (or a set of nucleic acids) encoding an anti-FXIIa antibody, b) a host cell for expressing the nucleic acid (or a set of nucleic acids), and c) instructions for i) expressing the anti-FXIIa antibody in the host cell, ii) preparing a composition comprising the anti-FXIIa antibody, and iii) administering the composition comprising the anti-FXIIa antibody to an individual for the treatment of a disease or condition associated with FXIIa signaling (*e*.*g*., thrombosis, FXII/FXIIa induced kinin formation related disease, FXII/FXIIa induced complement activation related disease, contact activation system related disease, inflammation related diseases).

The kits of the application are in suitable packaging. Suitable packaging includes, but is not limited to, vials, bottles, jars, flexible packaging (*e*.*g*., sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the anti-FXIIa antibody compositions generally include information as to dosage, dosing schedule, and route of administration for the intended treatment. The containers may be unit doses, bulk packages (*e*.*g*., multi-dose packages) or sub-unit doses. For example, kits may be provided that contain sufficient dosages of an anti-FXIIa antibody (such as a full-length anti-FXIIa antibody) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the anti-FXIIa antibody and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this application. The application will now be described in greater detail by reference to the following non-limiting examples. The following examples further illustrate the application but, of course, should not be construed as in any way limiting its scope.

### EXAMPLES

### Example 1: Generation of FXII Recombinant Antigens and Screening of Anti-FXIIa Antibodies

### Generation of FXII recombinant antigens

The nucleic acid sequences encoding human FXII (huFXII, Uniprot P00748), including the full-length FXII (amino acids of 20-615 position), fragment β FXII (amino acids of 354-615 position), rhesus monkey FXII (rheFXII, Uniprot F7FY72), rabbit FXII (rabFXII, Uniprot G1SIB7), and mouse FXII (musFXII, NCBI NP_067464.2), were fused with his- and/or Avi- tag genes, and constructed into mammalian cell expression vectors, respectively through subcloning, to obtain the huBFXII-avi-his, huFXII-his, rheFXII-his, rabFXII-his, and musFXII-his recombinant expression vector plasmids. Wherein, the "his or His" represents the His-tag, and the "Avi" represents the Avidin-tag.

The FXII recombinant fusion proteins mentioned above were expressed and purified. Firstly, the expression vector plasmids were transfected into HEK293F cells, and the cells were cultured at 37 °C, 5% CO₂, and 120rpm for 5 days, followed by collection of cell culture medium. Then, according to the manufacturer's instruction, the recombinant protein with His-tag was purified using a nickel column (Ni). The specific operation is as follows: use Ni-NTA (5ml) from Changzhou Smart-Lifesciences Biotechnology Co., Ltd. for immobilized metal affinity chromatography (IMAC). Firstly, the nickel column was equilibrated with 10-fold column volume of equilibration buffer (PBS, pH 7.4), then washed with 10-fold column volume of solution (PBS + 20mM imidazole, pH 7.4), subsequently, 5-fold column volume of elution buffer (PBS + 20mM imidazole, pH 7.4) was used for elution and the interest protein was collected, and then the solution was concentrated and replaced with PBS using a suitable molecular weight cut-off ultrafiltration tube.

For the huFXII-his, rheFXII-his, rabFXII-his, and museFXII-his proteins, the proteins described above were self activated into the active FXIIa form during the purification process. For the huβFXII-avi-his protein, a separate activation step was required after purification. In short, the huβFXII-avi-his protein was diluted to 1 µM by the buffer containing Hepes (7.5 mM, pH 7.6), NaCl (50 mM), and BSA (1 mg/ml). Then, 2 µM of dextran sulfate (molar ratio of huβFXII-avi-his to dextran sulfate is 50:1) was added and reacted at 37 ° C for 1 hour. Then, the protein was purified again by nickel column (Ni) following the above steps, and the activated form of huβFXIIa-avi-his was obtained.

### Generation of biotinylated FXII antigens

The huβFXIIa-avi-his protein was biotinylated using an in vivo biotin labeling method according to the operation instructions. In brief, the vector expressing the above proteins and the vector expressing biotin ligase BirA were co-transfected into 293F cells, and 0.2 µg/ml of D-biotin was added to the culture medium. The cells were then cultured at 37 °C, 120rpm, for 5 days. After collecting the cell culture supernatant, the His tagged protein was purified using the nickel column (Ni). The biotinylated protein was named Bavih-huβFXIIa. The biotinylation efficiency was detected by magnetic bead adsorption method, and the biotinylation efficiency was found to be at least 60%.

### Screening of anti-FXIIa antibodies

*Construction of scFv antibody yeast display library*: RNA was extracted from 2000 human blood samples, and cDNA was obtained by reverse transcription. The V_{H} and V_{K} specific primers were used to amplify V_{H} and V_{K} fragments. After gel recovery and purification, the V_{H} and V_{K} were ligated to construct scFv, which was cloned into yeast display plasmid PYD1. Subsequently, the plasmid was electrotransferred to yeast to obtain scFv antibody yeast display library.

### Screening of anti-FXIIa antibodies

ScFvs recognizing huFXIIa were isolated from the above yeast display library. In brief, yeast cells expressing huFXIIa scFv were first enriched by MACS magnetic bead sorting. Yeast cells of 1000 OD were centrifuged at 2500g for 5 min. The obtained cell pellets were resuspended with 1L SGCAA medium according to the initial concentration of OD600=1, and induced to express at 20 ° C and 250rpm for 40-48 h. After centrifugation and washing with PBSM solution, the cell pellet was resuspended with 5-10-fold volume of PBSM solution containing 1 µm Bavih-huβFXIIa and incubated at 4 ° C for 1 h. After centrifugation and washing with PBSM solution, the unbound antigen was washed away by PBSM solution. The magnetic beads were added, mixed well, incubated on ice for 30min, and mixed gently every 2min. Centrifuged at 2500g for 5 minutes, the supernatant was removed, and the pellet was resuspended with 5-10-fold volume of PBSM solution. 7ml of cell suspension was added to the column each time until all cell suspensions was flowed through the column. The cells bound to the column was collected.

Then the yeast cells enriched above were identified through three rounds of FACS by using huFXII-his to screen positive clones with strong binding activity. Briefly, the MACS sorted positive cells were induced to propagate. The induced 50 OD yeast cells were centrifuged at 2500g for 1min and washed with PBSF buffer. 500nm Bavih-huβFXIIa antigen was added, incubated on ice for 1h, centrifuged at 2500g for 1 min, and the positive cells were washed with PBSF buffer. Then the detection antibodies streptomycin avidin-fluorochrome conjugate SA-PE (BD, 554061) and V5-647 (GeneScript, A01805-100) were added, incubated on ice for 30min, the cells were collected by centrifugation and washed. Finally, the positive cells were detected and sorted by flow cytometry. The sorted positive cells were subjected to the second and third rounds of FACS according to the above steps. 200nm Bavih-huβFXIIa antigen was used in the second round of FACS, and 100nm Bavih-huβFXIIa antigen was used in the third round of FACS.

The positive yeast cells obtained from the above three rounds of FACS were subjected to monoclonal identification, and the genomic DNA (gDNA) of the positive clone was extracted. The V_{H} and V_{K} fragments were amplified by PCR with V_{H} and V_{K} specific primers, and then the candidate lead antibodies (FX-Y17, and FX-Y39) were obtained by sequencing. The variable domain sequences of the heavy and light chains are shown in Table 2 and Table 3.

### Example 2: Generation and Characterization of Full-length Anti-FXIIa Antibodies

### Generation of full-length anti-FXIIa antibodies

The V_{L} and V_{H} of the candidate lead anti-FXIIa antibodies obtained by sequencing were introduced into eukaryotic expression vectors pTTa1-L (containing kappa constant domain) and pTTa1-H1-LALA (containing IgG1 heavy chain constant domain, the L at positions 234 and 235 mutated to A, EU numbering system), respectively, to construct human IgG1 antibody. Plasmids expressing the light and heavy chains were extracted and used to co-transfect 293F cells. Then the cells were cultured at 37°C, 8% CO₂ and 120rpm for 5 days.

The culture media was purified using Protein A affinity chromatography. Briefly, Protein A column was first equilibrated with 6-fold column volume of 50mM PBS buffer (pH7.4) at a flow rate of 150cm/h. The supernatant of the culture media (pH was adjusted to 7.4) was passed through the column at 150cm/h. Upon further equilibration, 0.1M glycine (containing 0.15M NaCl, pH3.2) was used to wash the column and the eluent was collected, the pH value was adjusted to neutral. After ultrafiltration concentration to the PBS buffer, IgG concentration was measured to obtain the full-length anti-FXIIa antibodies for subsequent biochemical and biological analysis.

### Detection of inhibitory effect of anti-FXIIa antibodies on FXIIa activity using FXIIa chemical substrate S2302

S2302 is a specific enzyme active substrate of FXIIa. The binding of anti-FXIIa antibodies with FXIIa can inhibit the cleavage effect of FXIIa on S2302. The inhibitory effect of anti-FXIIa antibodies on FXIIa enzyme activity was reflected by detecting the OD 405 value of S2302 after being cleaved by FXIIa.

In short, the anti-FXIIa antibodies or positive control antibody CSL312, which is diluted in 3-fold gradient (starting concentration of 1mg/ml) was mixed with the equal volume of purified 0.03mg/ml huFXIIa-his, and placed at room temperature for 10 minutes to form an antigen-antibody complex. Next, 33.3 µl of 1.5mM S2302 substrate (purchased from CHROMOGENIX company) and 66.6 µl of the antigen-antibody complex were added to a transparent flat bottom 96 well plate, placed at 37 °C for 15 minutes, the OD405 was read and the binding curve was generated using PRISM software to analyze the inhibitory effect of anti-FXIIa antibody to be tested on the cleavage of S2302 substrate by FXIIa, and the IC50 value was calculated.

As shown in Table 5, the lead antibodies FX-Y17 and FX-Y39 can effectively inhibit the cleavage of S2302 substrate by FXIIa, and the inhibitory activity is comparable to that of the positive control antibody CS312.

**Table 5 The inhibitory effect of anti-FXIIa antibodies on the cleavage of chemical substrate S2302 by FXIIa**

| **Antibody Name** | **IC₅₀ (µg/ml)** |
|---|---|
| FX-Y17 | 0.0251 |
| FX-Y39 | 0.0398 |
| CSL312 | 0.0232 |

### Detection of binding activity of anti-FXIIa antibodies by ELISA method

The binding activity of the candidate lead anti-FXIIa antibodies were detected by ELISA assay. Briefly, the ELISA plates were coated with the purified huFXII-his antigen with the concentration of 1 µg/ml, incubated overnight at 4 °C, and washed 6 times with PBST. Subsequently, 50 µl of 4% skim milk and 50 µl of gradient diluted candidate lead anti-FXIIa antibody or positive control antibody CSL312 were added. After 1 hour of reaction at 37 °C, the plates were washed 6 times with PBST. Next, HRP labeled sheep anti-human secondary antibody was added. After 1 hour of reaction at 37 °C, the plate was washed 6 times with PBST. 100 µl TMB was added and reacted at room temperature for 10 minutes. 50 µl of 2M H₂SO₄ was added to terminate the reaction. The OD450 was read using an enzyme-linked immunosorbent assay (ELISA) reader, and a binding curve was generated using PRISM software to analyze the binding activity of each candidate lead anti-FXIIa antibody to human FXIIa antigen and the EC50 value was calculated. The binding activity between the candidate lead antibody and human FXIIa is weaker than that of the positive control antibody CSL312, therefore subsequent affinity maturation is performed (data not shown).

### Example 3: Affinity Maturation of Anti-FXIIa Antibodies

*Preparation of affinity matured anti FXIIa antibodies:* The lead antibody FX-Y17 was selected for further affinity maturation. In short, using CDR walking technology (CDR regions numbered according to Kabat definition), one mutation was randomly selected in the CDR1, CDR2, CDR3 (L1, L2&L3) regions of the light chain and the CDR1, CDR2, CDR3 (H1, H2&H3) regions of the heavy chain, with a theoretical library capacity of 4E8. The yeast display system was used to display and screen mutants. Sequencing was performed on scFv antibodies with enhanced biological activity. The variable domain sequences of the heavy and light chains of affinity mature antibodies are shown in Tables 2 and 3.

*Preparation of full-length anti FXIIa antibody:* The VL and VH sequences of the sequenced anti-FXIIa antibody were constructed into eukaryotic expression vectors pTTa1-L (containing the constant region of the kappa light chain) and pTTa1-H1-LALA (containing the constant region of the IgG1 heavy chain, with L mutations at positions 234 and 235 designated as A, EU number), respectively, for the construction of human IgG1 antibodies; In addition, VL and VH sequences were separately constructed into pTTa1-L (containing the constant region of the kappa light chain) and pTTa1-H1-LALA-YTE (containing the constant region of the IgG1 heavy chain, with L mutations at positions 234 and 235 to A, and amino acid at positions 252, 254, and 256 mutated to Y, T, and E, EU numbering) for the construction of long-acting human IgG1 antibodies. The extracted plasmids expressing the corresponding light and heavy chains were co transfected into 293F cells and cultured at 37 ° C, 5% CO2, and 120rpm for 5 days.

The culture medium was purified using a protein A affinity chromatography column, following the method described in Example 2. After obtaining full-length anti-FXIIa antibodies with mature affinity, further biochemical and biological analysis was conducted.

### Example 4: Characterization of Full-length Anti-FXIIa Antibodies with maturation of affinity

### Detection of the inhibitory effect of anti-FXIIa antibodies on FXIIa activity using FXIIa chemical substrate S2302

Using the method described in Example 2, the inhibitory effect of affinity mature anti FXIIa antibodies on FXIIa cleavage of S2302 substrate was detected.

As shown in Table 6, the parent antibody FX-Y17 and 45 affinity mature anti-FXIIa antibodies can effectively inhibit the cleavage of S2302 substrate by FXIIa, and the inhibitory activity is equivalent to that of the positive control antibody CS312.

**Table 6 The inhibitory effect of anti-FXIIa antibodies on the cleavage of chemical substrate S2302 by FXIIa**

| **Antibody Name** | **IC₅₀ (µg/ml)** | **Antibody Name** | **IC₅₀ (µg/ml)** |
|---|---|---|---|
| CSL312 | 0.02581 | FX-Y17-Y23 | 0.01289 |
| FX-Y17 | 0.02130 | FX-Y17-Y24 | 0.01659 |
| FX-Y17-Y1 | 0.02366 | FX-Y17-Y26 | 0.01628 |
| FX-Y17-Y2 | 0.01779 | FX-Y17-Y28 | 0.02670 |
| FX-Y17-Y3 | 0.01543 | FX-Y17-Y30 | 0.01272 |
| FX-Y17-Y4 | 0.01161 | FX-Y17-Y31 | 0.01556 |
| FX-Y17-Y5 | 0.02537 | FX-Y17-Y33 | 0.02200 |
| FX-Y17-Y6 | 0.01569 | FX-Y17-Y34 | 0.02821 |
| FX-Y17-Y7 | 0.01998 | FX-Y17-Y35 | 0.01390 |
| FX-Y17-Y8 | 0.01958 | FX-Y17-Y36 | 0.01552 |
| FX-Y17-Y9 | 0.02438 | FX-Y17-Y37 | 0.01432 |
| FX-Y17-Y10 | 0.02797 | FX-Y17-Y38 | 0.01600 |
| FX-Y17-Y11 | 0.01650 | FX-Y17-Y39 | 0.04221 |
| FX-Y17-Y12 | 0.02961 | FX-Y17-Y40 | 0.01547 |
| FX-Y17-Y13 | 0.02586 | FX-Y17-Y41 | 0.01904 |
| FX-Y17-Y14 | 0.01378 | FX-Y17-Y42 | 0.01768 |
| FX-Y17-Y15 | 0.02016 | FX-Y17-Y43 | 0.01458 |
| FX-Y17-Y16 | 0.02101 | FX-Y17-Y45 | 0.02214 |
| FX-Y17-Y17 | 0.01986 | FX-Y17-Y46 | 0.03207 |
| FX-Y17-Y18 | 0.02416 | FX-Y17-Y47 | 0.01786 |
| FX-Y17-Y19 | 0.02137 | FX-Y17-Y48 | 0.01412 |
| FX-Y17-Y20 | 0.01866 | FX-Y17-Y50 | 0.02253 |
| FX-Y17-Y21 | 0.01322 | FX-Y17-Y51 | 0.01552 |
| FX-Y17-Y22 | 0.02022 | | |

### ELISA method for detecting the binding activity of anti-FXIIa antibodies

Using the method described in Example 2, detect the binding activity of affinity mature anti-FXIIa antibodies to FXIIa.

As shown in Table 7, the exemplary anti-FXIIa antibody can effectively bind to FXIIa, and its binding activity is superior to or equivalent to that of the positive control antibody CSL312.

**Table 7 The binding affinity between anti-FXIIa antibodies and human FXIIa**

| **Antibody Name** | **EC₅₀ (µg/ml)** | **Antibody Name** | **EC₅₀ (µg/ml)** |
|---|---|---|---|
| CSL312 | 0.4213 | FX-Y17-Y16 | 0.1250 |
| FX-Y17-Y1 | 0.1508 | FX-Y17-Y17 | 0.1694 |
| FX-Y17-Y2 | 0.1635 | FX-Y17-Y18 | 0.1718 |
| FX-Y17-Y3 | 0.2156 | FX-Y17-Y19 | 0.1495 |
| FX-Y17-Y4 | 0.2048 | FX-Y17-Y20 | 0.1270 |
| FX-Y17-Y5 | 0.1665 | FX-Y17-Y21 | 0.1008 |
| FX-Y17-Y7 | 0.1359 | FX-Y17-Y22 | 0.1873 |
| FX-Y17-Y8 | 0.2369 | FX-Y17-Y23 | 0.0942 |
| FX-Y17-Y9 | 0.2280 | FX-Y17-Y30 | 0.0830 |
| FX-Y17-Y10 | 0.1611 | FX-Y17-Y31 | 0.1171 |
| FX-Y17-Y11 | 0.2389 | FX-Y17-Y37 | 0.0629 |
| FX-Y17-Y12 | 0.3642 | FX-Y17-Y40 | 0.0781 |
| FX-Y17-Y13 | 0.1279 | FX-Y17-Y45 | 0.3581 |
| FX-Y17-Y14 | 0.1395 | FX-Y17-Y46 | 0.2568 |
| FX-Y17-Y15 | 0.1311 | | |

### Detection of the inhibitory effect of anti-FXIIa antibodies on FXIIa induced coagulation by APTT

Activated partial thromboplastin time (APTT) is the most commonly used sensitive screening test in clinical practice to reflect the coagulation activity of the endogenous coagulation system. Adding a sufficient amount of activating contact factor activators, such as ellagic acid, to anticoagulant plasma, along with an appropriate amount of calcium ions, can meet all the conditions for endogenous anticoagulation. The time required from the addition of calcium ions to plasma coagulation is called activated partial thromboplastin time. The APTT test can detect the inhibitory effect of anti-FXIIa antibodies on FXIIa induced coagulation.

According to the operating instructions of the activated partial thromboplastin time detection kit (coagulation method) (purchased from SYSMEX), 50 µl of human serum (purchased from SYSMEX) and 50 µl of serially 2-fold diluted anti-FXIIa antibody (take FX-Y17-Y4, FX-Y17-Y8, FX-Y17-Y11, FX-Y17-Y23, FX-Y17-Y30, FX-Y17-Y34, FX-Y17-Y45 as examples), or positive control CSL312, and 50 µl APTT reagent, was added to a transparent flat bottomed 96 well plate in sequence. Mix well and incubate at 37 °C for 5 minutes. Afterwards, 50 µl of 25mM CaCl2 (purchased from SYSMEX) was added, and the OD660 was immediately read using an enzyme-linked immunosorbent assay (ELISA) reader every 5 seconds for a total of 60 times. The time when the OD660 significantly increased was recorded as the time of APTT occurrence.

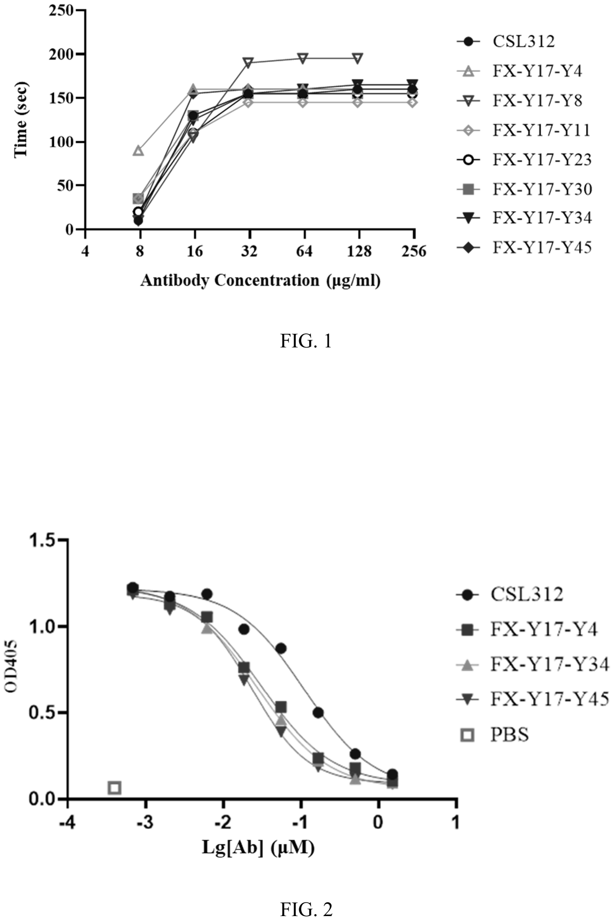

As shown in Figure 1, the exemplary anti-FXIIa antibody FX-Y17-Y4, FX-Y17-Y8, FX-Y17-Y11, FX-Y17-Y23, FX-Y17-Y30, FX-Y17-Y34, and FX-Y17-Y45 can all effectively prolong the APTT time.

### Detection of the inhibitory effect of anti-FXIIa antibodies on the cleavage of PK by FXIIa using pKa chemical substrate CS-3102

FXIIa can cleave prekallikrein (PK) and produce active plasma kallikrein (pKa), and CS-3102 is a PKa specific enzyme active substrate. The activity of FXIIa can be indirectly reflect by detecting the OD405 value of CS-3102 after it is cleaved by PKa. Anti-FXIIa antibodies can inhibit FXIIa cleavage of prekallikrein, thereby reducing the OD405 value of CS-3102 after cleavage by PKa.

In short, 33.3 µl of CS-3102 (purchased from BIOPHEN) with a concentration of 1mM, 33.3 µl of PK (purchased from Enzyme Research Lab) with a concentration of 200nM, 33.3 µl of anti-FXIIa antibody (taking FX-Y17-Y4, FX-Y17-Y34, FX-Y17-Y45 as examples) diluted in a 3-fold gradient (starting concentration of 1.5 µM) or positive control antibody CSL312 and 33.3 µl of FXIIa (purchased from Enzyme Research Lab) with a concentration of 25nM was added to a transparent flat bottomed 96 well plate sequentially. PBS was added to the negative control group. After incubation at 37 °C for 15 minutes, the OD405 was read and a binding curve was generated using PRISM software to analyze the inhibitory effect of each tested anti-FXIIa antibody on FXIIa cleavage of prekallikrein, and the IC50 value was calculated.

As shown in Figure 2 and Table 8, the exemplary anti-FXIIa antibodies FX-Y17-Y4, FX-Y17-Y34, and FX-Y17-Y45 can effectively inhibit the cleavage of prekallikrein by FXIIa, and their inhibitory activity is significantly higher than that of the positive control antibody CSL312.

**Table 8 The inhibitory effect of anti-FXIIa antibodies on the cleavage of PK proenzyme by FXIIa**

| **Antibody Name** | **IC₅₀** (**µM**) |
|---|---|
| FX-Y17-Y4 | 0.03060 |
| FX-Y17-Y34 | 0.02709 |
| FX-Y17-Y45 | 0.02346 |
| CSL312 | 0.10980 |

### Example 5: Pharmacokinetics (PK) Study of Anti-FXIIa Antibodies in Rats

Twelve SD rats were randomly divided into three groups with four rats in each group, with half male and half female. Rats in each group were intravenously injected with anti-FXIIa antibody to be tested, with CSL312 antibody as positive control, and the dose was 10mpk. The whole blood was collected from the animal's eye socket before administration (0h), and at 5 min, 1 h, 5 h, 24 h (D1), 72 h (D3), 168 h (D7), 240 h (D10), and 336 h (D14) after administration, the serum was isolated, and the drug concentration in the serum of each sample was determined by ELISA. The drug time curve was plotted using Graphpad Prism software.

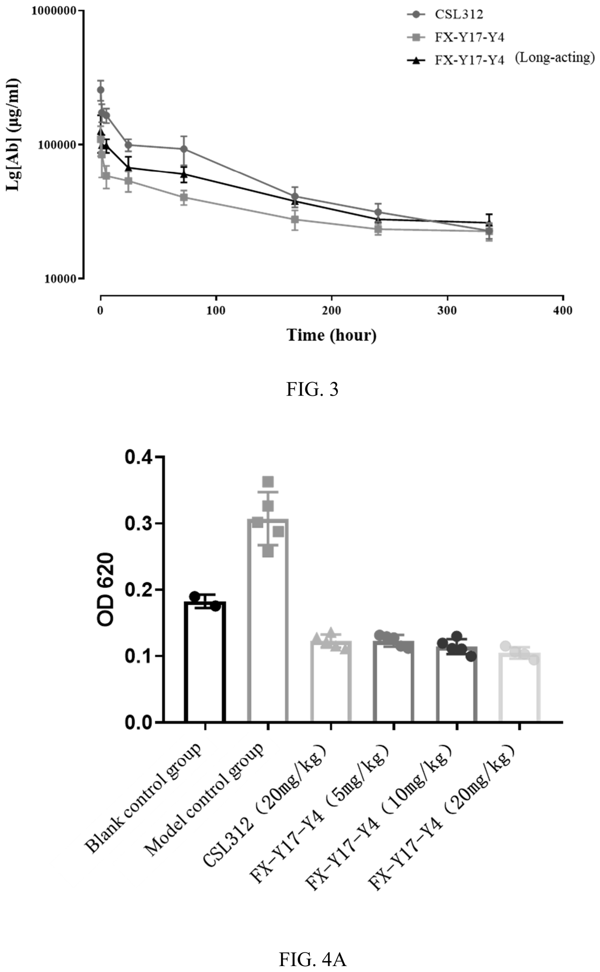

As shown in Figure 3, the blood concentration in rats after administration of the exemplary anti-FXIIa antibodies, FX-Y17-Y4, and FX-Y17-Y4 (long-acting) showed a similar elimination trend to the positive control antibody CSL312. Other exemplary anti-FXIIa antibodies, FX-Y17-Y23, FX-Y17-Y34, and FX-Y17-Y45, also showed the same trend in blood concentration changes (data not shown).

### Example 6: Efficacy Study of Anti-FXIIa Antibodies in Vivo

Captopril is an angiotensin converting enzyme inhibitor (ACE inhibitor), which is used in the treatment of hypertension in clinic. Captopril was used to induce increased vascular permeability in wild mice by interfering with the degradation of bradykinin to expand peripheral blood vessels. Anti-FXIIa antibody can inhibit the activity of bradykinin, thereby reducing the vascular permeability. According to the experiment, the vascular permeability changes was indicated by the leakage of Evans blue dye from the blood into the small intestine, and the efficacy of the anti-FXIIa antibodies applied in the application in mice is verified by measuring the role of anti-FXIIa antibodies in inhibiting the vascular permeability of mice.

In brief, firstly, Balb/c mice in each group were injected with anti-FXIIa antibodies or normal saline (0h) by intraperitoneal injection. The experimental group mice were injected with the exemplary anti-FXIIa antibodies to be tested in the application, and three doses of 5mg/kg, 10mg/kg and 20mg/kg were set respectively. Mice in the positive control group were injected with CSL312 antibody at a dose of 20mg/kg. Mice in model control group and blank control group were injected with equal volume of saline. Two hours later, mice in the experimental group, positive control group and model control group were injected with 2.5mg/kg captopril (C4042-5G, Sigma) via the tail vein, while mice in the blank control group were injected with equal volume of saline. Two hours and 15min later, mice in each group were injected with 30mg/kg Evans blue dye (E2129-50G, Sigma) via the tail vein. 2 hours and 30 minutes later, the mice in each group were anesthetized, and 100 µl of blood was collected from the abdominal main vein. After blood collection, it was immediately mixed with 300 µl of ice ethanol, centrifuged at 14000rmp/min for 10min, and the absorbance was measured at the wavelength of 620nm to detect the content of dye in the blood of mice. In addition, 5cm of mice intestine was taken, soaked in 0.75 ml GA (blood/cell/tissue genomic DNA extraction kit, TIANGEN), sheared and ground, and 20 µl proteinase K was added. The mixture was digested in 56 °C thermostatic water bath overnight. Then 0.75ml GB (blood/cell/tissue genomic DNA extraction kit, TIANGEN) was added, mixed thoroughly upside down and incubated in 70 °C water bath for 10min. After centrifugation at 10000g /min for 10 min, 200 µl supernatant was taken and the absorbance was measured at the wavelength of 620nm to detect the content of dye in the intestine of mice.

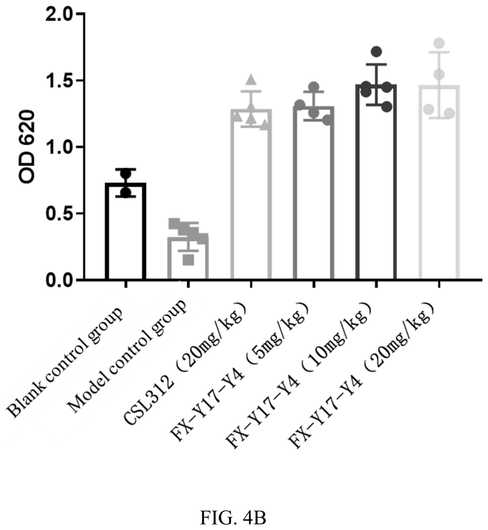

As shown in Figure 4A-4B, compared with the model control group, the exemplary anti-FXIIa antibodies FX-Y17-Y4 significantly reduced the dye leakage in the intestinal tissue of mice (Figure 4A) and effectively increased the dye residue in the blood (Figure 4B). Other exemplary antibodies FX-Y17-Y23, FX-Y17-Y34, and FX-Y17-Y45 can also significantly reduce dye leakage in intestinal tissues and increase dye residues in blood (specific data not shown). The above results show that the anti-FXIIa antibodies of the application can effectively reduce the vascular permeability in mice and have excellent in vivo activity.

## Claims

1. An isolated anti-FXIIa antibody, wherein the anti-FXIIa antibody comprises a heavy chain variable domain (V_{H}) comprising:
a heavy chain complementarity determining region (HC-CDR) 1 comprising SQTMS (SEQ ID NO: 1);
an HC-CDR2 comprising SX₁X₂X₃ISX₄RX₅YYADSVKG (SEQ ID NO: 89), wherein X₁ is I or S, X₂ is H or S, X₃ is H, V or Y, X₄ is G, K or R, and X₅ is A, D, K, M, N, P, R, S, T or Y; and
an HC-CDR3 comprising X₁HX₂FDX₃ (SEQ ID NO: 90), wherein X₁ is A, G, H, Q, S or W, X₂ is A, D or R, and X₃ is L or V;
and a light chain variable domain (V_{L}) comprising:
a light chain complementarity determining region (LC-CDR) 1 comprising RASQSX₁X₂X₃X₄LA (SEQ ID NO: 91), wherein X₁ is L or V, X₂ is F, W or Y, X₃ is S or Y, and X₄ is A, H or N;
an LC-CDR2 comprising GASTRAT (SEQ ID NO: 65); and
an LC-CDR3 comprising QQX₁X₂X₃WPLS (SEQ ID NO: 92), wherein X₁ is N or Y, X₂ is H, K, N or Q, and X₃ is K, R or W.

2. The isolated anti-FXIIa antibody of claim 1, comprising:
(i) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 74;
(ii) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(iii) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76;
(iv) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(v) a V_{H} comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 34; and a V_{L} comprising an LC-CDR1 comprising the amino acid sequence of SEQ ID NO: 54, an LC-CDR2 comprising the amino acid sequence of SEQ ID NO: 65, and an LC-CDR3 comprising the amino acid sequence of SEQ ID NO: 75;
(vi) a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(vii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(viii) a V_{H} comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(ix) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(x) a V_{H} comprising an HC-CDR1 comprising the amino acid sequence of SEQ ID NO: 1, an HC-CDR2 comprising the amino acid sequence of SEQ ID NO: 17, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xi) a V_{H} comprising an HC-CDR1 comprising the amino acid sequence of SEQ ID NO: 1, an HC-CDR2 comprising the amino acid sequence of SEQ ID NO: 16, and an HC-CDR3 comprising the amino acid sequence of SEQ ID NO: 35; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 36; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(xiii) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 37; and a V_{L} comprising an LC-CDR1 comprising the amino acid sequence of SEQ ID NO: 55, an LC-CDR2 comprising the amino acid sequence of SEQ ID NO: 65, and an LC-CDR3 comprising the amino acid sequence of SEQ ID NO: 77;
(xiv) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 34; and a V_{L} comprising an LC-CDR1 comprising the amino acid sequence of SEQ ID NO: 51, an LC-CDR2 comprising the amino acid sequence of SEQ ID NO: 65, and an LC-CDR3 comprising the amino acid sequence of SEQ ID NO: 75;
(xv) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 38; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76;
(xvi) a V_{H} comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xvii) a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xviii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(xix) a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 40; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(xx) a V_{H} comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxi) a V_{H} comprising an HC-CDR1 comprising the amino acid sequence of SEQ ID NO: 1, an HC-CDR2 comprising the amino acid sequence of SEQ ID NO: 19, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(xxii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxiii) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxiv) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 41; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxv) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 34; and a V_{L} comprising an LC-CDR1 comprising the amino acid sequence of SEQ ID NO: 56, an LC-CDR2 comprising the amino acid sequence of SEQ ID NO: 65, and an LC-CDR3 comprising the amino acid sequence of SEQ ID NO: 78;
(xxvi) a V_{H} comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(xxvii) a V_{H} comprising an HC-CDR1 comprising the amino acid sequence of SEQ ID NO: 1, an HC-CDR2 comprising the amino acid sequence of SEQ ID NO: 22, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxviii) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 42; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 76;
(xxix) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 35; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 75;
(xxx) a V_{H} comprising an HC-CDR1 comprising the amino acid sequence of SEQ ID NO: 1, an HC-CDR2 comprising the amino acid sequence of SEQ ID NO: 23, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxxi) a V_{H} comprising an HC-CDR1 comprising the amino acid sequence of SEQ ID NO: 1, an HC-CDR2 comprising the amino acid sequence of SEQ ID NO: 24, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxxii) a V_{H} comprising an HC-CDR1 comprising the amino acid sequence of SEQ ID NO: 1, an HC-CDR2 comprising the amino acid sequence of SEQ ID NO: 25, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75;
(xxxiii) a V_{H} comprising an HC-CDR1 comprising the amino acid sequence of SEQ ID NO: 1, an HC-CDR2 comprising the amino acid sequence of SEQ ID NO: 12, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 79;
(xxxiv) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 39; and a V_{L} comprising an LC-CDR1 comprising the amino acid sequence of SEQ ID NO: 53, an LC-CDR2 comprising the amino acid sequence of SEQ ID NO: 65, and an LC-CDR3 comprising the amino acid sequence of SEQ ID NO: 80; or
(xxxv) a V_{H} comprising an HC-CDR1 comprising the amino acid sequence of SEQ ID NO: 1, an HC-CDR2 comprising the amino acid sequence of SEQ ID NO: 21, and an HC-CDR3 comprising the amino acid sequence of SEQ ID NO: 34; and a V_{L} comprising an LC-CDR1 comprising the amino acid sequence of SEQ ID NO: 52, an LC-CDR2 comprising the amino acid sequence of SEQ ID NO: 65, and an LC-CDR3 comprising the amino acid sequence of SEQ ID NO: 75.

3. The isolated anti-FXIIa antibody of any one of claims 1-2, comprising:
(i) a V_{H} comprising the amino acid sequence of SEQ ID NO: 93, or a variant thereof having at least 80% sequence identity to the amino acid sequence of SEQ ID NO: 93; and a V_{L} comprising the amino acid sequence of SEQ ID NO: 139, or a variant thereof having at least 80% sequence identity to the amino acid sequence of SEQ ID NO: 139;
(ii) a V_{H} comprising the amino acid sequence of SEQ ID NO: 94, or a variant thereof having at least 80% sequence identity to the amino acid sequence of SEQ ID NO: 94; and a V_{L} comprising the amino acid sequence of SEQ ID NO: 140, or a variant thereof having at least 80% sequence identity to the amino acid sequence of SEQ ID NO: 140;
(iii) a V_{H} comprising the amino acid sequence of SEQ ID NO: 95, or a variant thereof having at least 80% sequence identity to the amino acid sequence of SEQ ID NO: 95; and a V_{L} comprising the amino acid sequence of SEQ ID NO: 141, or a variant thereof having at least 80% sequence identity to the amino acid sequence of SEQ ID NO: 141;
(iv) a V_{H} comprising the amino acid sequence of SEQ ID NO: 96, or a variant thereof having at least 80% sequence identity to the amino acid sequence of SEQ ID NO: 96; and a V_{L} comprising the amino acid sequence of SEQ ID NO: 142, or a variant thereof having at least 80% sequence identity to the amino acid sequence of SEQ ID NO: 142;
(v) a V_{H} comprising the amino acid sequence of SEQ ID NO: 97, or a variant thereof having at least 80% sequence identity to the amino acid sequence of SEQ ID NO: 97; and a V_{L} comprising the amino acid sequence of SEQ ID NO: 143, or a variant thereof having at least 80% sequence identity to the amino acid sequence of SEQ ID NO: 143;
(vi) a V_{H} comprising the amino acid sequence of SEQ ID NO: 98, or a variant thereof having at least 80% sequence identity to the amino acid sequence of SEQ ID NO: 98; and a V_{L} comprising the amino acid sequence of SEQ ID NO: 140, or a variant thereof having at least 80% sequence identity to the amino acid sequence of SEQ ID NO: 140;
(vii) a V_{H} comprising the amino acid sequence of SEQ ID NO: 96, or a variant thereof having at least 80% sequence identity to the amino acid sequence of SEQ ID NO: 96; and a V_{L} comprising the amino acid sequence of SEQ ID NO: 140, or a variant thereof having at least 80% sequence identity to the amino acid sequence of SEQ ID NO: 140;
(viii) a V_{H} comprising the amino acid sequence of SEQ ID NO: 99, or a variant thereof having at least 80% sequence identity to the amino acid sequence of SEQ ID NO: 99; and a V_{L} comprising the amino acid sequence of SEQ ID NO: 140, or a variant thereof having at least 80% sequence identity to the amino acid sequence of SEQ ID NO: 140;
(ix) a V_{H} comprising the amino acid sequence of SEQ ID NO: 94, or a variant thereof having at least 80% sequence identity to the amino acid sequence of SEQ ID NO: 94; and a V_{L} comprising the amino acid sequence of SEQ ID NO: 142, or a variant thereof having at least 80% sequence identity to the amino acid sequence of SEQ ID NO: 142;
(x) a V_{H} comprising the amino acid sequence of SEQ ID NO: 100, or a variant thereof having at least 80% sequence identity to the amino acid sequence of SEQ ID NO: 100; and a V_{L} comprising the amino acid sequence of SEQ ID NO: 140, or a variant thereof having at least 80% sequence identity to the amino acid sequence of SEQ ID NO: 140;
(xi) a V_{H} comprising the amino acid sequence of SEQ ID NO: 101, or a variant thereof having at least 80% sequence identity to the amino acid sequence of SEQ ID NO: 101; and a V_{L} comprising the amino acid sequence of SEQ ID NO: 140, or a variant thereof having at least 80% sequence identity to the amino acid sequence of SEQ ID NO: 140;
(xii) a V_{H} comprising the amino acid sequence of SEQ ID NO: 102, or a variant thereof having at least 80% sequence identity to the amino acid sequence of SEQ ID NO: 102; and a V_{L} comprising the amino acid sequence of SEQ ID NO: 142, or a variant thereof having at least 80% sequence identity to the amino acid sequence of SEQ ID NO: 142;
(xiii) a V_{H} comprising the amino acid sequence of SEQ ID NO: 103, or a variant thereof having at least 80% sequence identity to the amino acid sequence of SEQ ID NO: 103; and a V_{L} comprising the amino acid sequence of SEQ ID NO: 144, or a variant thereof having at least 80% sequence identity to the amino acid sequence of SEQ ID NO: 144;
(xiv) a V_{H} comprising the amino acid sequence of SEQ ID NO: 104, or a variant thereof having at least 80% sequence identity to the amino acid sequence of SEQ ID NO: 104; and a V_{L} comprising the amino acid sequence of SEQ ID NO: 145, or a variant thereof having at least 80% sequence identity to the amino acid sequence of SEQ ID NO: 145;
(xv) a V_{H} comprising the amino acid sequence of SEQ ID NO: 105, or a variant thereof having at least 80% sequence identity to the amino acid sequence of SEQ ID NO: 105; and a V_{L} comprising the amino acid sequence of SEQ ID NO: 141, or a variant thereof having at least 80% sequence identity to the amino acid sequence of SEQ ID NO: 141;
(xvi) a V_{H} comprising the amino acid sequence of SEQ ID NO: 106, or a variant thereof having at least 80% sequence identity to the amino acid sequence of SEQ ID NO: 106; and a V_{L} comprising the amino acid sequence of SEQ ID NO: 140, or a variant thereof having at least 80% sequence identity to the amino acid sequence of SEQ ID NO: 140;
(xvii) a V_{H} comprising the amino acid sequence of SEQ ID NO: 107, or a variant thereof having at least 80% sequence identity to the amino acid sequence of SEQ ID NO: 107; and a V_{L} comprising the amino acid sequence of SEQ ID NO: 140, or a variant thereof having at least 80% sequence identity to the amino acid sequence of SEQ ID NO: 140;
(xviii) a V_{H} comprising the amino acid sequence of SEQ ID NO: 108, or a variant thereof having at least 80% sequence identity to the amino acid sequence of SEQ ID NO: 108; and a V_{L} comprising the amino acid sequence of SEQ ID NO: 142, or a variant thereof having at least 80% sequence identity to the amino acid sequence of SEQ ID NO: 142;
(xix) a V_{H} comprising the amino acid sequence of SEQ ID NO: 109, or a variant thereof having at least 80% sequence identity to the amino acid sequence of SEQ ID NO: 109; and a V_{L} comprising the amino acid sequence of SEQ ID NO: 142, or a variant thereof having at least 80% sequence identity to the amino acid sequence of SEQ ID NO: 142;
(xx) a V_{H} comprising the amino acid sequence of SEQ ID NO: 110, or a variant thereof having at least 80% sequence identity to the amino acid sequence of SEQ ID NO: 110; and a V_{L} comprising the amino acid sequence of SEQ ID NO: 140, or a variant thereof having at least 80% sequence identity to the amino acid sequence of SEQ ID NO: 140;
(xxi) a V_{H} comprising the amino acid sequence of SEQ ID NO: 111, or a variant thereof having at least 80% sequence identity to the amino acid sequence of SEQ ID NO: 111; and a V_{L} comprising the amino acid sequence of SEQ ID NO: 140, or a variant thereof having at least 80% sequence identity to the amino acid sequence of SEQ ID NO: 140;
(xxii) a V_{H} comprising the amino acid sequence of SEQ ID NO: 112, or a variant thereof having at least 80% sequence identity to the amino acid sequence of SEQ ID NO: 112; and a V_{L} comprising the amino acid sequence of SEQ ID NO: 141, or a variant thereof having at least 80% sequence identity to the amino acid sequence of SEQ ID NO: 141;
(xxiii) a V_{H} comprising the amino acid sequence of SEQ ID NO: 113, or a variant thereof having at least 80% sequence identity to the amino acid sequence of SEQ ID NO: 113; and a V_{L} comprising the amino acid sequence of SEQ ID NO: 142, or a variant thereof having at least 80% sequence identity to the amino acid sequence of SEQ ID NO: 142;
(xxiv) a V_{H} comprising the amino acid sequence of SEQ ID NO: 114, or a variant thereof having at least 80% sequence identity to the amino acid sequence of SEQ ID NO: 114; and a V_{L} comprising the amino acid sequence of SEQ ID NO: 142, or a variant thereof having at least 80% sequence identity to the amino acid sequence of SEQ ID NO: 142;
(xxv) a V_{H} comprising the amino acid sequence of SEQ ID NO: 108, or a variant thereof having at least 80% sequence identity to the amino acid sequence of SEQ ID NO: 108; and a V_{L} comprising the amino acid sequence of SEQ ID NO: 140, or a variant thereof having at least 80% sequence identity to the amino acid sequence of SEQ ID NO: 140;
(xxvi) a V_{H} comprising the amino acid sequence of SEQ ID NO: 93, or a variant thereof having at least 80% sequence identity to the amino acid sequence of SEQ ID NO: 93; and a V_{L} comprising the amino acid sequence of SEQ ID NO: 140, or a variant thereof having at least 80% sequence identity to the amino acid sequence of SEQ ID NO: 140;
(xxvii) a V_{H} comprising the amino acid sequence of SEQ ID NO: 115, or a variant thereof having at least 80% sequence identity to the amino acid sequence of SEQ ID NO: 115; and a V_{L} comprising the amino acid sequence of SEQ ID NO: 140, or a variant thereof having at least 80% sequence identity to the amino acid sequence of SEQ ID NO: 140;
(xxviii) a V_{H} comprising the amino acid sequence of SEQ ID NO: 116, or a variant thereof having at least 80% sequence identity to the amino acid sequence of SEQ ID NO: 116; and a V_{L} comprising the amino acid sequence of SEQ ID NO: 146, or a variant thereof having at least 80% sequence identity to the amino acid sequence of SEQ ID NO: 146;
(xxix) a V_{H} comprising the amino acid sequence of SEQ ID NO: 117, or a variant thereof having at least 80% sequence identity to the amino acid sequence of SEQ ID NO: 117; and a V_{L} comprising the amino acid sequence of SEQ ID NO: 142, or a variant thereof having at least 80% sequence identity to the amino acid sequence of SEQ ID NO: 142;
(xxx) a V_{H} comprising the amino acid sequence of SEQ ID NO: 118, or a variant thereof having at least 80% sequence identity to the amino acid sequence of SEQ ID NO: 118; and a V_{L} comprising the amino acid sequence of SEQ ID NO: 140, or a variant thereof having at least 80% sequence identity to the amino acid sequence of SEQ ID NO: 140;
(xxxi) a V_{H} comprising the amino acid sequence of SEQ ID NO: 104, or a variant thereof having at least 80% sequence identity to the amino acid sequence of SEQ ID NO: 104; and a V_{L} comprising the amino acid sequence of SEQ ID NO: 142, or a variant thereof having at least 80% sequence identity to the amino acid sequence of SEQ ID NO: 142;
(xxxii) a V_{H} comprising the amino acid sequence of SEQ ID NO: 119, or a variant thereof having at least 80% sequence identity to the amino acid sequence of SEQ ID NO: 119; and a V_{L} comprising the amino acid sequence of SEQ ID NO: 140, or a variant thereof having at least 80% sequence identity to the amino acid sequence of SEQ ID NO: 140;
(xxxiii) a V_{H} comprising the amino acid sequence of SEQ ID NO: 120, or a variant thereof having at least 80% sequence identity to the amino acid sequence of SEQ ID NO: 120; and a V_{L} comprising the amino acid sequence of SEQ ID NO: 140, or a variant thereof having at least 80% sequence identity to the amino acid sequence of SEQ ID NO: 140;
(xxxiv) a V_{H} comprising the amino acid sequence of SEQ ID NO: 111, or a variant thereof having at least 80% sequence identity to the amino acid sequence of SEQ ID NO: 111; and a V_{L} comprising the amino acid sequence of SEQ ID NO: 142, or a variant thereof having at least 80% sequence identity to the amino acid sequence of SEQ ID NO: 142;
(xxxv) a V_{H} comprising the amino acid sequence of SEQ ID NO: 121, or a variant thereof having at least 80% sequence identity to the amino acid sequence of SEQ ID NO: 121; and a V_{L} comprising the amino acid sequence of SEQ ID NO: 140, or a variant thereof having at least 80% sequence identity to the amino acid sequence of SEQ ID NO: 140;
(xxxvi) a V_{H} comprising the amino acid sequence of SEQ ID NO: 122, or a variant thereof having at least 80% sequence identity to the amino acid sequence of SEQ ID NO: 122; and a V_{L} comprising the amino acid sequence of SEQ ID NO: 141, or a variant thereof having at least 80% sequence identity to the amino acid sequence of SEQ ID NO: 141;
(xxxvii) a V_{H} comprising the amino acid sequence of SEQ ID NO: 123, or a variant thereof having at least 80% sequence identity to the amino acid sequence of SEQ ID NO: 123; and a V_{L} comprising the amino acid sequence of SEQ ID NO: 142, or a variant thereof having at least 80% sequence identity to the amino acid sequence of SEQ ID NO: 142;
(xxxviii) a V_{H} comprising the amino acid sequence of SEQ ID NO: 124, or a variant thereof having at least 80% sequence identity to the amino acid sequence of SEQ ID NO: 124; and a V_{L} comprising the amino acid sequence of SEQ ID NO: 140, or a variant thereof having at least 80% sequence identity to the amino acid sequence of SEQ ID NO: 140;
(xxxix) a V_{H} comprising the amino acid sequence of SEQ ID NO: 125, or a variant thereof having at least 80% sequence identity to the amino acid sequence of SEQ ID NO: 125; and a V_{L} comprising the amino acid sequence of SEQ ID NO: 140, or a variant thereof having at least 80% sequence identity to the amino acid sequence of SEQ ID NO: 140;
(xl) a V_{H} comprising the amino acid sequence of SEQ ID NO: 126, or a variant thereof having at least 80% sequence identity to the amino acid sequence of SEQ ID NO: 126; and a V_{L} comprising the amino acid sequence of SEQ ID NO: 140, or a variant thereof having at least 80% sequence identity to the amino acid sequence of SEQ ID NO: 140;
(xli) a V_{H} comprising the amino acid sequence of SEQ ID NO: 112, or a variant thereof having at least 80% sequence identity to the amino acid sequence of SEQ ID NO: 112; and a V_{L} comprising the amino acid sequence of SEQ ID NO: 147, or a variant thereof having at least 80% sequence identity to the amino acid sequence of SEQ ID NO: 147;
(xlii) a V_{H} comprising the amino acid sequence of SEQ ID NO: 108, or a variant thereof having at least 80% sequence identity to the amino acid sequence of SEQ ID NO: 108; and a V_{L} comprising the amino acid sequence of SEQ ID NO: 148, or a variant thereof having at least 80% sequence identity to the amino acid sequence of SEQ ID NO: 148;
(xliii) a V_{H} comprising the amino acid sequence of SEQ ID NO: 127, or a variant thereof having at least 80% sequence identity to the amino acid sequence of SEQ ID NO: 127; and a V_{L} comprising the amino acid sequence of SEQ ID NO: 140, or a variant thereof having at least 80% sequence identity to the amino acid sequence of SEQ ID NO: 140;
(xliv) a V_{H} comprising the amino acid sequence of SEQ ID NO: 128, or a variant thereof having at least 80% sequence identity to the amino acid sequence of SEQ ID NO: 128; and a V_{L} comprising the amino acid sequence of SEQ ID NO: 140, or a variant thereof having at least 80% sequence identity to the amino acid sequence of SEQ ID NO: 140;
(xlv) a V_{H} comprising the amino acid sequence of SEQ ID NO: 129, or a variant thereof having at least 80% sequence identity to the amino acid sequence of SEQ ID NO: 129; and a V_{L} comprising the amino acid sequence of SEQ ID NO: 140, or a variant thereof having at least 80% sequence identity to the amino acid sequence of SEQ ID NO: 140; or
(xlvi) a V_{H} comprising the amino acid sequence of SEQ ID NO: 130, or a variant thereof having at least 80% sequence identity to the amino acid sequence of SEQ ID NO: 130; and a V_{L} comprising the amino acid sequence of SEQ ID NO: 140, or a variant thereof having at least 80% sequence identity to the amino acid sequence of SEQ ID NO: 140.

4. The isolated anti-FXIIa antibody of any one of claims 1-3, wherein the anti- FXIIa antibody binds to the human FXIIa with a Kd from 0.1 pM to 1 nM.

5. The isolated anti-FXIIa antibody of any one of claims 1-4, wherein the anti-FXIIa antibody comprises an Fc fragment.

6. The isolated anti-FXIIa antibody of claim 5, wherein the anti-FXIIa antibody is a full-length IgA, IgD, IgE, IgG or IgM antibody.

7. The isolated anti-FXIIa antibody of claim 6, wherein the anti-FXIIa antibody is a full-length IgG1, IgG2, IgG3 or IgG4 antibody.

8. The isolated anti-FXIIa antibody of any one of claims 1-7, wherein the anti-FXIIa antibody is chimeric, human, or humanized.

9. The isolated anti-FXIIa antibody according to any one of claims 1-4, wherein the anti-FXIIa antibody is an antigen binding fragment selected from the group consisting of a Fab, a Fab', a F(ab)'₂, a Fab'-SH, a single-chain Fv (scFv), an Fv fragment, a dAb, a Fd, a nanobody, a diabody, and a linear antibody.

10. An isolated nucleic acid molecule that encodes the anti-FXIIa antibody according to any one of claims 1-9.

11. A vector comprising the nucleic acid molecule of claim 10.

12. An isolated host cell comprising the anti-FXIIa antibody of any one of claims 1-9, the nucleic acid molecule of claim 10, or the vector of claim 11.

13. A method of producing an anti-FXIIa antibody, comprising:
a) culturing the host cell of claim 12 under conditions effective to express the anti-FXIIa antibody; and
b) obtaining the expressed anti-FXIIa antibody from the host cell.

14. A pharmaceutical composition comprising the anti-FXIIa antibody according to any one of claims 1-9, the nucleic acid molecule of claim 10, the vector of claim 11, the isolated host cell of claim 12, or the antibody produced according to the method of claim 13, and a pharmaceutically acceptable carrier.

15. Use of the antibody according to any one of claims 1-9, the nucleic acid molecule of claim 10, the vector of claim 11, the isolated host cell of claim 12, the antibody produced according to the method of claim 13 or the pharmaceutical composition of claim 14 in the manufacture of a medicament for treating a disease or condition in an individual in need thereof, wherein the disease or condition is a disease and/or condition caused by the dysregulation of the FXIIa signaling pathway, such as thrombosis, FXII/FXIIa induced kinin formation related disease, FXII/FXIIa induced complement activation related disease, contact activation system related disease, and inflammation related diseases.

16. The use of claim 15, wherein the disease or condition is selected from the group consisting of venous, arterial or capillary thrombosis, intracardiac thrombosis, thrombosis during and/or after the contact of human or animal subject's blood with artificial surface, thromboembolism, atherosclerosis, interstitial lung disease, inflammation, neuroinflammatory disease, neurotrauma disease, traumatic brain injury, complement activation, fibrinolysis, angiogenesis, hereditary angioedema (HAE), pulmonary bacterial infection, trypanosoma infection, hypotension shock, pancreatitis Chagas disease, joint gout, arthritis, disseminated intravascular coagulation and sepsis, macular edema, diabetic retinopathy, hypertensive retinopathy, age-related macular degeneration, retinal vein occlusion, organ ischemia, distal ischemia-reperfusion injury (IRI), chronic kidney disease, renal fibrosis, and multiple sclerosis.
